# EUROPEAN PATENT SPECIFICATION

(11) **EP 3 692 068 B1**
(45) Date of publication and mention of the grant of the patent: **07.12.2022**
(21) Application number: 18783458.5
(22) Date of filing: 05.10.2018
(51) Int. Cl.: C07K 16/40, C07K 16/28, A61K 39/00, A61P 35/00, C12N 9/14

(54) **RESTORATION OF T CELL ACTIVITY VIA THE CD39/CD73 AXIS**
HERSTELLUNG DER T-ZELLAKTIVITÄT ÜBER DIE CD39 / CD73 - ACHSE
RESTAURATION DE L'ACTIVITÉ T CELLULAIRE VIA L'AXE CD39 / CD73

(30) Priority: 06.10.2017 US 201762568812 P; 18.06.2018 US 201862686143 P
(43) Date of publication of application: 12.08.2020
(73) Proprietor: Innate Pharma, 13009 Marseille (FR)
(72) Inventor: CHANTEUX, Stéphanie, 13009 Marseille (FR); GOURDIN, Nicolas, 13008 Marseille (FR); PATUREL, Carine, 69280 Marcy l'Etoile (FR); PERROT, Ivan, 13260 Cassis (FR); ROSSI, Benjamin, 13008 Marseille (FR)
(74) Representative: Cabinet Becker et Associés
(86) International application number: PCT/EP2018/077217
(87) International publication number: WO 2019/068907

(56) References cited:
- WO-A1-2009/095478
- WO-A1-2016/055609
- WO-A1-2018/167267
- PAUL A BEAVIS ET AL: "CD73: a potent suppressor of antitumor immune responses", TRENDS IN IMMUNOLOGY, vol. 33, no. 5, May 2012 (2012-05), pages 231-237, XP028479101, ISSN: 1471-4906, DOI: 10.1016/J.IT.2012.02.009 [retrieved on 2012-03-02]
- ALLARD B. ET AL.: "The ectonucleotidases CD39 and CD73: Novel checkpoint inhibitor targets", IMMUNOL. REV., vol. 276, March 2017 (2017-03), pages 121-144, XP002786951,
- YOUNG ARABELLA ET AL: "Co-inhibition of CD73 and A2AR Adenosine Signaling Improves Anti-tumor Immune Responses", CANCER CELL, CELL PRESS, US, vol. 30, no. 3, 12 September 2016 (2016-09-12), pages 391-403, XP029725575, ISSN: 1535-6108, DOI: 10.1016/J.CCELL.2016.06.025
- SEBASTIAN FM HÄUSLER ET AL: "Anti-CD39 and anti-CD73 antibodies A1 and 7G2 improve targeted therapy in ovarian cancer by blocking adenosine-dependent immune evasion", AM J TRANSL RES, vol. 6, no. 2, 15 January 2014 (2014-01-15), pages 129-139, XP055240896,
- Perrot I. et al.: "Preclinical development of humanized CD39 (IPH52) and CD73 (IPH53) blocking antibodiestargeting the ATP/Adenosine immune checkpoint pathway for cancer immunotherapy", AACR meeting, 14-18.04.2018, ID:2718 , 28 April 2018 (2018-04-28), XP002786975, Retrieved from the Internet: URL:https://www.innate-pharma.com/sites/de fault/files/poster_cd39_cd73_bat.pdf [retrieved on 2018-11-30]

## Description

### FIELD OF THE INVENTION

This invention relates to the use of CD39 neutralizing agents for the treatment of cancer.

### BACKGROUND OF THE INVENTION

NTPDase 1 (ectonucleoside triphosphate diphosphohydrolase1), also known as CD39/ENTPD1 or vascular CD39, functions together with another enzyme, CD73 (ecto-5'-nucleotidase), to hydrolyze extracellular adenosine triphosphate (ATP) and adenosine diphosphate (ADP) to generate adenosine, which binds to adenosine receptors and inhibits T-cell and natural killer (NK)-cell responses, thereby suppressing the immune system. The generation of adenosine via the CD73/CD39 pathway is recognized as a major mechanism of regulatory T cell (Treg) immunosuppressive function. CD39 has two transmembrane domains near the N- and C-terminal ends, short cytoplasmic N- and C-terminal segments, and a large extracellular domain containing the active site. However, while CD39 is typically anchored to the membrane by the two transmembrane domains at the two ends of the molecule, it has recently also been reported that a soluble catalytically active form of CD39 can be found in circulation in human and mice (Yegutkin et al., (2012) FASEB J. 26(9): 3875-3883).

CD73 (ecto-5'-nucleotidase) is a 70-kDa glycosylphosphatidylinositol (GPI)-anchored protein normally expressed on endothelial cells and subsets of hematopoietic cells. CD73 expression has been reported in a range of tumor cells, including, among others, leukemia, bladder cancer, glioma, glioblastoma, ovarian cancer, melanoma, prostate cancer, thyroid cancer, esophageal cancer and breast cancer. CD73 expression has also been associated with a prometastatic phenotype in melanoma and breast cancer.

Antibodies that specifically bind CD73 may provide an alternative to small molecules with greater selectivity for CD73. However the antibodies tested have generally only shown partial inhibition of the enzymatic activity of CD73. It has been shown that therapy with an antibody that binds murine CD73 can inhibit breast tumor growth and metastasis in mice (Stagg, et al. (2010) Proc. Natl. Acad. Sci. USA 104:1547-1552). Antibodies however generally do not cross react with human and mouse CD73, complicating the study of the antibodies and the biological functions of CD73. It has been shown that genetic deletion of A2A receptors can induce T cell-dependent tumor rejection (Ohta, et al., (2006) Proc Natl Acad Sci USA 103:13132-13137). Knock-down using siRNA or overexpression of CD73 on tumor cells can modulate tumor growth and metastasis (Beavis et al (2013 Proc. Natl. Acad. Sci. USA 110:14711-716; Stagg et al. (2010), supra; Jin et al. (2010) Cancer Res. 70: 2245-55). CD73-/- mice are protected from transplanted and spontaneous tumors (Stagg et al. (2010) Cancer Res. 71: 2892-2900). In humans, high CD73 expression can be a negative prognostic for cancer (Loi et al (2013 Proc. Natl. Acad. Sci. USA 110: 11091-11096). Antibodies that bind CD73 have been reported, for example clone AD2 (mouse IgG1 isotype), has been reported to functionally block CD73 by causing receptor clustering and internalization but have minimal effect on enzymatic activity. Sachsenmeier et al. ((2012) J. Biomed. Screening 17:993-998) and (Rust et al. (2013) Mol. Cancer 12:11) also reported an antibody that induces intracellular internalization. More recently, antibodies that block both soluble and cell surface CD73 have been disclosed WO2016/055609 and WO2016/131950, as well as antibodies that inhibit cell surface CD73 by inducing intracellular internalization of CD73 in cells (e.g. tumor cells) WO2017/064043; WO2016/075099 and WO2016/081748.

CD73, together with CD39, regulates adenosine triphosphate (ATP) metabolism. CD39 (NTPDase-1) converts ATP into AMP, with only trace amounts of ADP being released, while CD73 catalyzes the conversion of AMP to adenosine. The number of CD39⁺ Tregs is increased in some human cancers, and the importance of CD39⁺ Tregs in promoting tumor growth and metastasis has been demonstrated using several in vivo models. However, CD39 is also expressed by tumor cells and CD39⁺ tumor cells can mediate immunosuppression via the adenosine pathway. CD39 in cancer cells displays ATPase activity and, together with CD73, generates adenosine. CD73⁺CD39⁺ cancer cells inhibited the proliferation of CD4 and CD8 T cells and the generation of cytotoxic effector CD8 T cells (CTL) in a CD39- and adenosine-dependent manner. Antibodies that bind and inhibit CD39 are disclosed in WO2009/095478. Hayes et al. (2015) Am. J. Transl. Res. 7(6):1181-1188 makes use of an anti-CD39 that is stated to also be blocking but the antibody also binds FcyR and has effector function.

Hausler et al. (2014) Am J Transl Res. 6(2):129-139 report that antibodies against CD39 and CD73 that mediate ADCC (through an ability to bind Fcγ receptors each induce NK cell mediated lysis of target cells, and that each of the antibodies also cause a partial decrease the production of adenosine. However, when the anti-CD39 and CD73 antibodies were tested together for decrease the production of adenosine, there was no additive effect of the combination of antibodies.

CD39 expression on different cell types, including immune cells and tumor cells, combined with use of antibodies that either do not actually block CD39 or are not pure blockers, create a complex setting for evaluation of the underlying activity of antibodies. Blocking enzymatic active sites using protein agents such as antibodies has generally known to be difficult. There is therefore a need to understand whether and how antibodies can inhibit the ATPase activity of CD39, and how to design improved molecules.

Thus, despite the interest in targeting CD39 and CD73, the most effective way to reduce adenosine generation remains to be determined.

Beavis (2012); Trends Immunol. 33(5):231-237 teaches that targeting CD73 is a promising avenue for treating cancer. Allard (2017); Immunol. Rev. 276:121-144 discusses the biology of the ectonucleases and the importance of the CD39, respectively CD73, alongside A2aR and A2bR. It suggests that a combinatorial approach may be beneficial. Young (2016); Cancer Cell 30(3):391-403 actually implements successfully one of such combinatorial approaches, by blocking CD73 and A2AR (a downstream element).

### SUMMARY

The invention relates to the treatment of a certain cancers by administering: an antibody that binds and inhibits the ATPase activity of a soluble extracellular domain CD39, and an antibody that neutralizes the 5' ectonuclease activity of human CD73. The scope of the invention is defined by the appended claims.

The technical disclosure set out below may in some respects go beyond the scope of the invention as defined by the appended claims, to place the invention claimed in a broader technical context. Any information that does not fall within the scope of the claims is provided for information only.

The present invention arises, *inter alia*, from the discovery that unlike known neutralizing anti-CD39 antibodies which do not substantially reduce in immunosuppression when used in combination with CD73 blockade, antibodies that neutralize soluble CD39 protein provide a dramatic reduction in immunosuppression when used in combination with CD73 blockade.

By inhibiting the ATPase activity of soluble CD39, the anti-CD39 antibodies reduce the pool of substrate available for CD73, which in turn strongly enhances efficacy of agents that inhibit the enzymatic activity of CD73, possibly because agents that inhibit CD73 are limited in their ability to fully inhibit CD73 activity. In contrast, antibodies that inhibit membrane-bound CD39 but not soluble CD39 do not enhance the efficacy of agents that inhibit the enzymatic activity of CD73.

Furthermore, at increasing concentrations, the antibodies that neutralize soluble CD39 provide substantially complete inhibition of the catabolic activity of the CD39/CD73 axis. The effects of the antibodies that neutralize soluble CD39 are observed in the presence of significant levels of ATP, for example as may be observed in the presence of exogenously added ATP in assays systems (in vitro) or as may occur in tumors such as solid tumors or generally tumors with high catabolic activity of the CD39/CD73 axis (e.g. tumors characterized by CD73 polypeptide and/or CD73 polypeptide-expressing cells). The antibodies that neutralize soluble CD39 can therefore be used advantageously in tumors characterized by CD73 polypeptide and/or CD73 polypeptide-expressing cells, or in combination with agents that increase CD73 expression (e.g. chemotherapeutic agents, anthracyclines) or that lead to generation of ATP (e.g. chemotherapeutic agents).

Additionally, by neutralizing the ATPase activity of soluble and membrane-bound CD39, the antibodies permit the conservation of the available pool of ATP, which enhances anti-tumor immunity (ATP is immunostimulatory). The anti-CD39 antibodies can therefore be advantageously used in combination with therapeutic agents that induce the extracellular release of ATP from tumor cells, notably agents or treatments that induce immunogenic cancer cell death (e.g., chemotherapeutic agents, radiotherapy). The anti-CD39 antibodies will not only prevent the released ATP from increasing the pool of CD73 substrate (and ultimately adenosine), but will conserve the released ATP so as to promote its immunostimulatory function.

Accordingly, in one aspect the present invention provides improved methods of enhancing an anti-tumor immune response, via the use of antibodies that bind and neutralize soluble CD39, in combination with an agent that inhibits CD73.

In another aspect, the invention provides improved methods of enhancing an anti-tumor immune response, via the use of antibodies that bind and neutralize soluble CD39 with combined treatment with an agent that inhibits CD73 for treatment of cancers characterized by the presence of CD73 protein (e.g. tumors with soluble CD73 and/or CD73 expressing cells; CD73-positive tumors).

Without wishing to be bound by theory, it is believed that antibodies that neutralize membrane-bound CD39 at the cell surface function by inhibiting the domain motion of membrane-bound CD39 (memCD39), however without similarly affecting the activity of the soluble CD39 protein (sCD39). It has been reported that memCD39 occurs as homo-multimers (e.g. tetramers and/or other multimers, in addition to monomeric forms) while sCD39 is a monomer, and moreover that the transmembrane domains in memCD39 undergo dynamic motions that underlie a functional relationship with the active site (Schulte am Esch et al. 1999 Biochem. 38(8):2248-58). Antibodies that block only memCD39 may recognize CD39 outside of the enzyme active site and prevent multimerization without blocking the monomeric form of CD39. Blockade of multimerization may reduce enzyme activity, and it has been reported that CD39 multimerization substantially augments ATPase activity. In contrast, antibodies that also block sCD39 may interfere with CD39 substrate and inhibit monomeric form of the enzyme. Such antibodies may also prevent multimerization of memCD39, thus providing a second mechanism of inhibition of the enzymatic activity of CD39. In the presence of ATP (e.g. as in the tumor environment), partial inhibition of CD39 by prevention of multimerization without blockade of sCD39 may lead to sufficient residual AMP to prevent any detectable additive effect on an agent that inhibits CD73 because remaining AMP can lead to CD73-mediated adenosine production sufficient to mediate immunosuppression.

Consequently, antibodies that bind and inhibit the ATPase activity of soluble CD39 (e.g. monomeric sCD39) can be used advantageously to achieve greater neutralization of CD39 activity in an individual by neutralizing both membrane-bound and soluble CD39 protein (an extracellular domain protein in solution), thereby reducing immunosuppression, e.g., for the treatment of cancer and/or infectious disease.

In one aspect, provided herein is use of an anti-CD39 antibody (e.g. having the further characteristics described herein), for use in treating an individual having a cancer, where the treatment comprises administration to the individual of the anti-CD39 antibody in combination with a means for inhibiting the activity of CD73. In one aspect, the provided is an anti-CD39 antibody (e.g. having the further characteristics described herein), for use in treating an individual having a cancer, where the treatment comprises administration to the individual of the anti-CD39 antibody in combination with a pharmaceutical composition comprising (a) a an antibody agent for inhibiting the activity of CD73 and (b) a pharmaceutically acceptable carrier.

In another aspect, provided herein is the use of antibodies that bind CD39 and inhibit the enzymatic (ATPase activity) activity of soluble (extracellular domain) human CD39 protein, in combination with an agent that inhibits the activity of CD73. The antibodies that bind soluble CD39 additionally potently inhibit the enzymatic (ATPase activity) activity of the cell membrane bound CD39 enzyme (CD39 as expressed at the surface of cells). The neutralization of both monomeric (e.g., soluble and membrane bound CD39) and multimeric CD39 (e.g., membrane bound CD39) in combination with an agent that inhibits the activity of CD73 can be particularly advantageous to treat CD73-positive cancer. A CD73-positive cancer is a cancer known to be generally characterized by presence of soluble CD73 protein and/or CD73-expressing cells in the tumor or tumor environment. In one embodiment, the CD73-positive cancer is characterized by a tumor determined to comprise CD73-expressing cells. In one aspect, the CD73-positive cancer is characterized by tumor tissue comprising malignant cells that express CD73. In one embodiment, the CD73-positive cancer is characterized by tumor tissue or tumor-adjacent tissue characterized by infiltration of CD73-expressing immune cells (e.g. non-malignant immune cells). In one embodiment, the CD73-positive cancer is a leukemia, a glioma or glioblastoma, or a cancer of the bladder, breast, colon, esophagus, kidney, liver, lung, ovary, uterus, prostate, pancreas, stomach, cervix, thyroid, head and neck (head and neck squamous cell carcinoma, and skin (e.g. melanoma).

Neutralization of both monomeric (e.g., soluble and membrane bound CD39) and multimeric CD39 (e.g., membrane bound CD39) can be particularly advantageous to enhance the activity of CD73 inhibiting agents in treatment settings where such CD73 inhibiting agents are sub-optimally active, for example where CD73 inhibition is sought within the tumor tissue (e.g. in CD73-positive tumors), where the CD73 inhibiting agent is not able to be administered at a dose or regimen at which it saturates CD73 in tumor tissues and/or at which it provides the EC₅₀, EC₇₀ or EC₁₀₀ for CD73 inhibition in tumor tissues (e.g., in CD73-positive tumors; at dosages that provide tumor concentrations of less than (or no more than) 1, 5 or 10 µg/ml) for a sufficient period of time (e.g. 1, 2, 3, 4, 6 or 8 weeks, or more); where the CD73 inhibiting agent is not fully inhibiting of CD73 activity (e.g. anti-CD73 antibodies that do not inhibit soluble CD73; anti-CD73 antibodies that rely on induction of CD73 down-modulation/intracellular internalization to mediate inhibition of enzymatic activity); or more generally in the treatment of cancers characterized by significant levels of expression of CD73 by cells in the tumor or tumor-adjacent tissue (e.g., a glioma or glioblastoma, or a cancer of the bladder, breast, colon, esophagus, kidney, liver, lung, ovary, uterus, prostate, pancreas, stomach, cervix, thyroid, head and neck (head and neck squamous cell carcinoma, and skin (e.g. melanoma).

In one aspect, the compound that inhibits or neutralizes the ATPase activity of a CD39 protein is or comprises an antibody or antibody fragment that binds CD39 protein (e.g. a monospecific antibody, a bispecific or multispecific antibody).

In one aspect provided is a composition comprising an antibody that inhibits a human sCD39 polypeptide and an antibody that inhibits a human CD73 polypeptide for use in the treatment or prevention of a cancer, optionally a solid tumor, optionally a haematological malignancy, optionally a haematological malignancy characterized by malignant cells that express CD73, optionally a leukemia, optionally a cancer characterized by malignant cells, optionally a cancer characterized by malignant and/or non-malignant cells in the tumor or tumor adjacent tissue that express CD73 polypeptides.

The antibodies will be useful in inhibiting catabolism of AMP (and upstream ATP and ADP) to adenosine, e.g. decreasing the pool of available adenosine precursors at each step, and ultimately decreasing the concentration of adenosine in the tumor microenvironment. These antibodies will therefore be useful in reversing the immunosuppressive effect of CD39 and/or CD73 and/or adenosine on T cells, B cells and other cells that express adenosine receptors, for example in the treatment of cancer. In one aspect, the antibodies neutralize adenosine-mediated inhibition of proliferation, cytokine production, cytotoxicity and/or NFκB activity in T cells. In one aspect, the methods of the disclosure are useful for increasing or enhancing anti-tumor immunity, for reducing immunosuppression, for activating and/or potentiating the activity of a an immune effector cell, optionally a CD8+ tumor-infiltrating T cell or NK cell, in an individual, and/or for decreasing the amount and/or concentration of adenosine in the tumor environment.

In one aspect, the compound that inhibits a human CD73 polypeptide is an anti-CD73 antibody that inhibits the enzymatic activity of CD73. In another aspect, the anti-CD73 antibody inhibits the 5'-ectonucleotidase activity of CD73 by causing an increase and/or inducing intracellular internalization of, or more generally the down-modulation of, cell surface-expressed CD73 and/or depends thereupon at least partly for its ability to neutralize CD73. In one aspect, the anti-CD73 antibody that inhibits the enzymatic activity of CD73 is an antibody that is not capable of substantially neutralizing or inhibiting the enzymatic activity of a soluble CD73, optionally when the antibody is provided at higher (e.g. 10 fold) excess of antibody:enzyme. In one aspect of the disclosure, the anti-CD73 antibody that inhibits the enzymatic activity of CD73 is an antibody that is capable of neutralizing the enzymatic activity of a soluble CD73, optionally when the antibody is capable of neutralizing the enzymatic activity of a soluble CD73 at higher (e.g. 10 fold) excess of antibody:enzyme.

In one aspect of the disclosure, a cancer is a solid tumor. In one aspect of the disclosure, the cancer is a hematological malignancy. In one aspect of the disclosure, a cancer is characterized by cells in the tumor or tumor-adjacent tissue (e.g. malignant cells and/or non-malignant cells in the tumor environment) expressing detectable CD73 protein.

In one aspect of the disclosure, the individual can be specified to be a human.

In one aspect of the disclosure, the anti-CD39 antibodies are to be administered to an individual having a cancer in an amount and frequency sufficient to neutralize the activity of CD39 (sCD39 and/or memCD39) in the periphery and/or in the tumor microenvironment. In one aspect of the disclosure, the antibodies are to be administered in an amount and frequency sufficient to decrease the generation and/or concentration of AMP and/or adenosine in the tumor microenvironment. In one aspect of the disclosure, the antibodies are to be administered in an amount and frequency sufficient to neutralize the activity of CD39 expressed by tumor cells. In one aspect of the disclosure, the antibodies are to be administered in an amount and frequency sufficient to neutralize the activity of CD39 expressed by leukocytes or lymphocytes, e.g. CD4 T cells, CD8 T cells, TReg cells and/or B cells.

In one aspect of the disclosure, the agent that inhibits the activity of CD73 is to be administered to an individual having a cancer in an amount and frequency sufficient to neutralize the activity of CD73 (soluble CD73 protein and/or membrane bound CD73) in the periphery and/or in the tumor microenvironment. In one aspect of the disclosure, the antibodies are to be administered in an amount and frequency sufficient to neutralize the activity of CD73 expressed by tumor cells or non-tumor cells present in the tumor environment. In one aspect of the disclosure, the antibodies are to be administered in an amount and frequency sufficient to neutralize the activity of CD73 expressed by CD4 T cells, CD8 T cells and/or B cells.

In one aspect, the anti-CD39 antibody and the agent that inhibits the activity of CD73 are to be administered in an amount and frequency sufficient to decrease the generation and/or concentration of adenosine in the tumor microenvironment. In one aspect, the antibodies are to be administered in an amount and frequency sufficient to increase the generation and/or concentration of ATP in the tumor microenvironment.

In one aspect, the anti-CD39 antibody and the agent that inhibits the activity of CD73 is each to be administered for at least one administration cycle, the administration cycle comprising at least a first and second (and optionally a 3^{rd}, 4^{th}, 5^{th}, 6^{th}, 7^{th} and/or 8^{th} or further) administration of the anti-CD39 antibody and agent that inhibits the activity of CD73.

In one aspect, the cancer is an advanced and/or refractory solid tumor. In one aspect, the cancer is an advanced and/or refractory solid tumor. In one non-limiting embodiment, the cancer (e.g., the advanced refractory solid tumor) is selected from the group consisting of non-small cell lung cancer (NSCLC), kidney cancer, pancreatic or esophagus adenocarcinoma, breast cancer, renal cell carcinoma (RCC), melanoma, colorectal cancer, and ovarian cancer (and optionally a further cancer type described herein).

In certain optional aspects an anti-CD39 agent can be for use to treat a cancer in an individual having a cancer or tumor characterized by immunosuppression, optionally lack of or insufficient immune infiltrate in tumors, optionally lack of or insufficient anti-tumor immunity.

In certain optional aspects an anti-CD39 agent can be for use to treat a cancer in an individual having a poor disease prognosis, notably a poor prognosis for response to treatment with an agent that neutralizes CD73. An individual having a poor disease prognosis is, for example, at a higher risk of progression, based on one or more predictive factors. In one aspect, a predictive factor(s) comprises presence or absence of a mutation in one or more genes. In one aspect, the predictive factor(s) comprises level(s) of expression of one or more genes or proteins, or example inhibitory or activating receptors on immune effector cells. In one aspect, a predictive factor(s) comprises presence (e.g., numbers) of cells in circulation or in the tumor environment expressing CD73 and/or CD39, and/or expression levels of CD73 and/or CD39 on cells in circulation or in the tumor environment; in one aspect, the cells are tumor cells; in one embodiment the cells are leukocytes, e.g., B cells, regulatory T cells (Treg). Presence of elevated expression of CD73 and/or CD39, and/or elevated numbers of CD73- and/or CD39 expressing cells can indicate an individual has a poor prognosis for response to treatment with an antibody that neutralizes CD73.

In certain optional aspects an anti-CD39 agent can be for use to treat a cancer in an individual who is a non-responder, or who has experienced a partial or an incomplete response to treatment with an agent that neutralizes CD73, or whose disease has relapsed or progressed following treatment with an antibody that neutralizes CD73.

In certain optional aspects, patients can be identified for treatment with CD39-neutralizing agent and a CD73-neutralizing agent (e.g. any agent disclosed herein) by assessing whether the patient is a poor responder (has a poor prognosis for response) for a CD73-neutralizing agent. A poor responder can be treated with a combination of a CD39-neutralizing agent and the CD73-neutralizing agent.

In certain optional aspects, patients can be identified for treatment with a CD39-neutralizing agent (and optionally further a CD73-neutralizing agent) by assessing the presence in circulation and/or in a tumor sample (e.g., tumor tissue and/or tumor adjacent tissue) elevated levels of CD73 polypeptide expression and/or numbers of CD73-expressing cells.

In certain optional aspects, patients can be identified for treatment with a CD39-neutralizing agent and a CD73-neutralizing agent by assessing the presence in circulation and/or in a tumor sample (e.g., tumor tissue and/or tumor adjacent tissue) elevated levels of CD39 polypeptide expression and/or numbers of CD39-expressing cells.

In other aspects, pharmaceutical compositions and kits are provided, as well as methods for using them. In one aspect, provided is a pharmaceutical composition comprising a compound that neutralizes the ATPase activity of a human CD39 polypeptide and an agent that neutralizes the 5'-ectonucleotidase activity of a CD73 polypeptide. In one aspect, provided is a kit comprising a compound that neutralizes the inhibitory activity of a human CD39 polypeptide and an agent that neutralizes the 5'-ectonucleotidase activity of a CD73 polypeptide.

These aspects are more fully described in, and additional aspects, features, and advantages will be apparent from, the description of the invention provided herein.

### BRIEF DESCRIPTION OF THE DRAWINGS

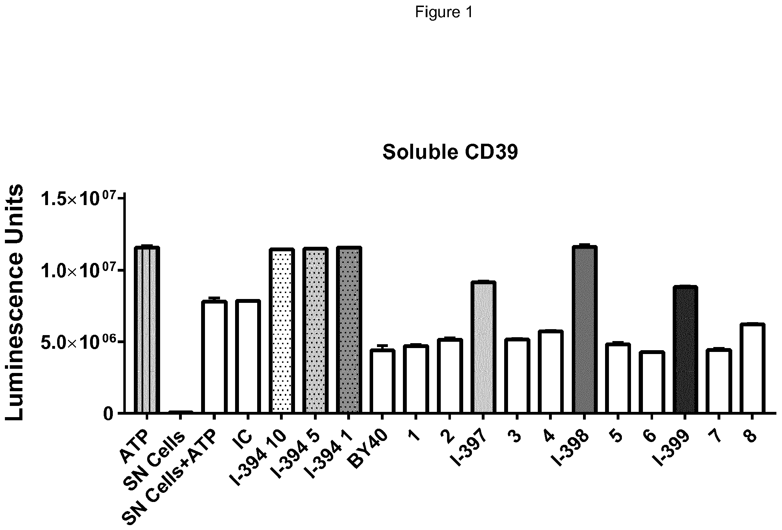

Figure 1 shows a representative screening result, showing antibodies I-397, I-398 and I-399 compared to positive control I-394 antibody.

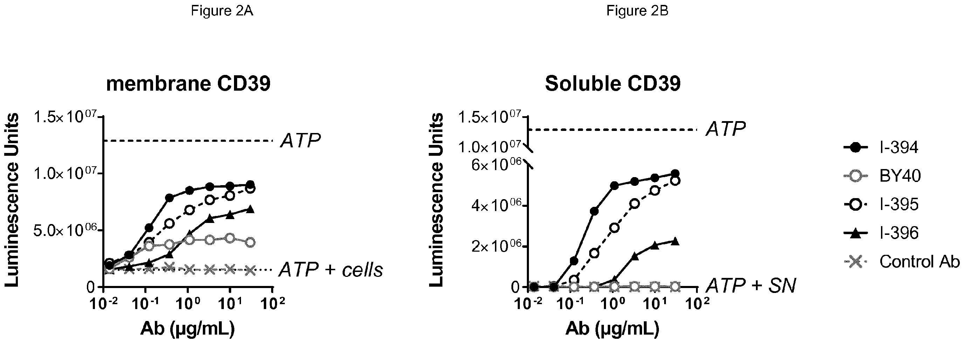

Figure 2A shows that antibodies BY40, I-394, I-395 and I-396 inhibit cell-membrane bound CD39, with both I-394 and I-395 showing greater potency at all concentrations as well as greater maximal inhibition of cellular CD39 compared to BY40. Figure 2B shows that antibodies I-395 and I-396 both inhibit soluble CD39 in comparison to negative control (BY40) and positive control (I-394) antibodies.

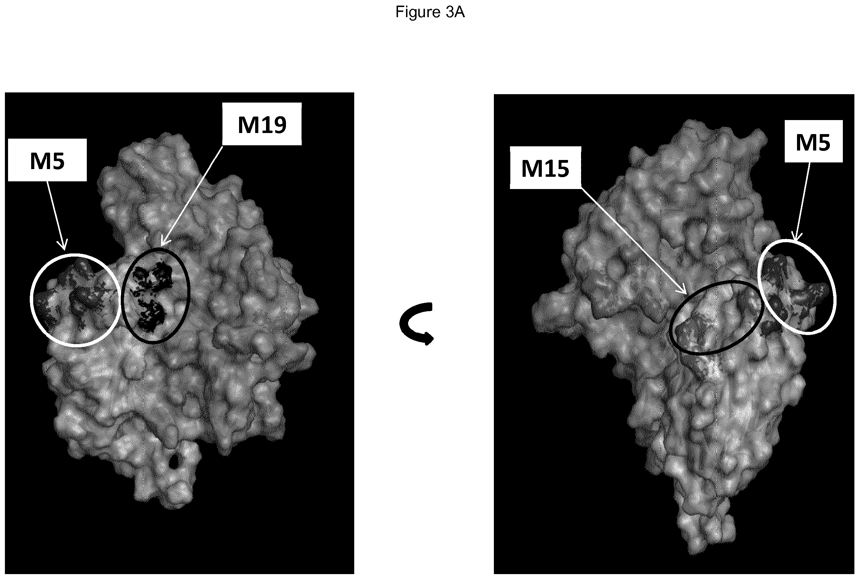

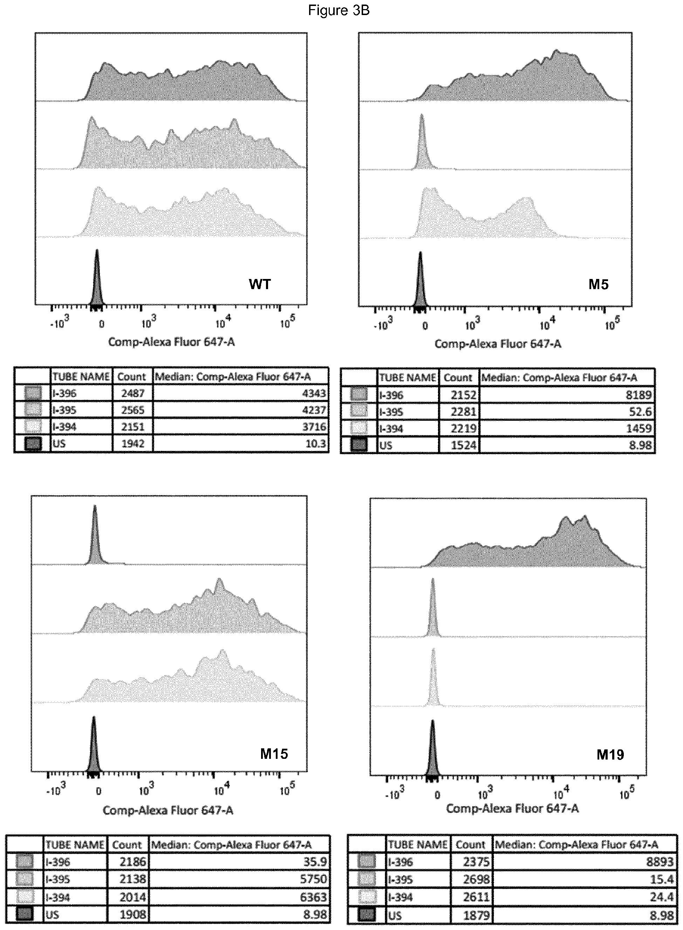

Figure 3A shows the position of residues mutated in mutants 5 (M5), 15 (M15) and 19 (M19) on the surface of the CD39 protein. Figure 3B shows results of binding to mutants 5, 15 and 19 for different antibodies.

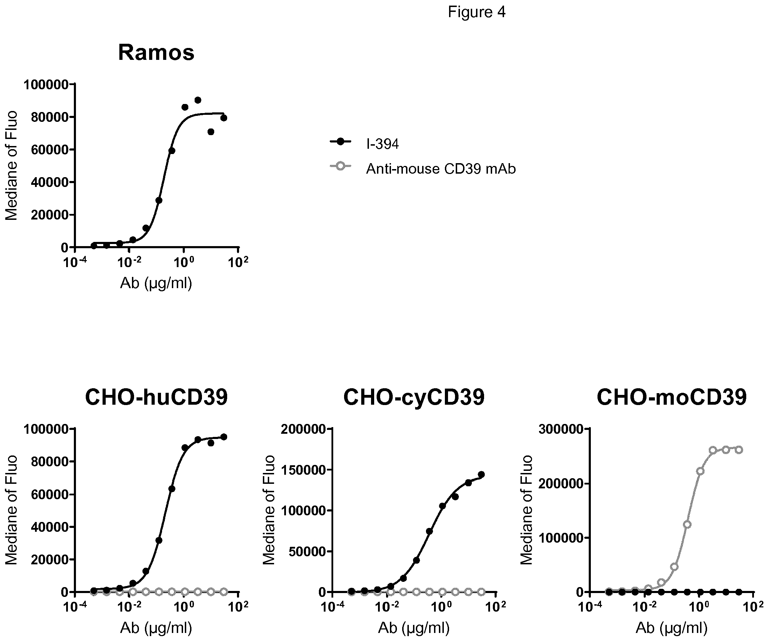

Figure 4 shows binding of antibody I-394 to cells expressing human CD39, as assessed by flow cytometry. I-394 binds cells expressing human CD39 (CHO-huCD39), cells expressing cynomolgus CD39 (CHO-cyCD39) and to Ramos lymphoma cells, but not to cells expressing murine CD39 (CHO-moCD39).

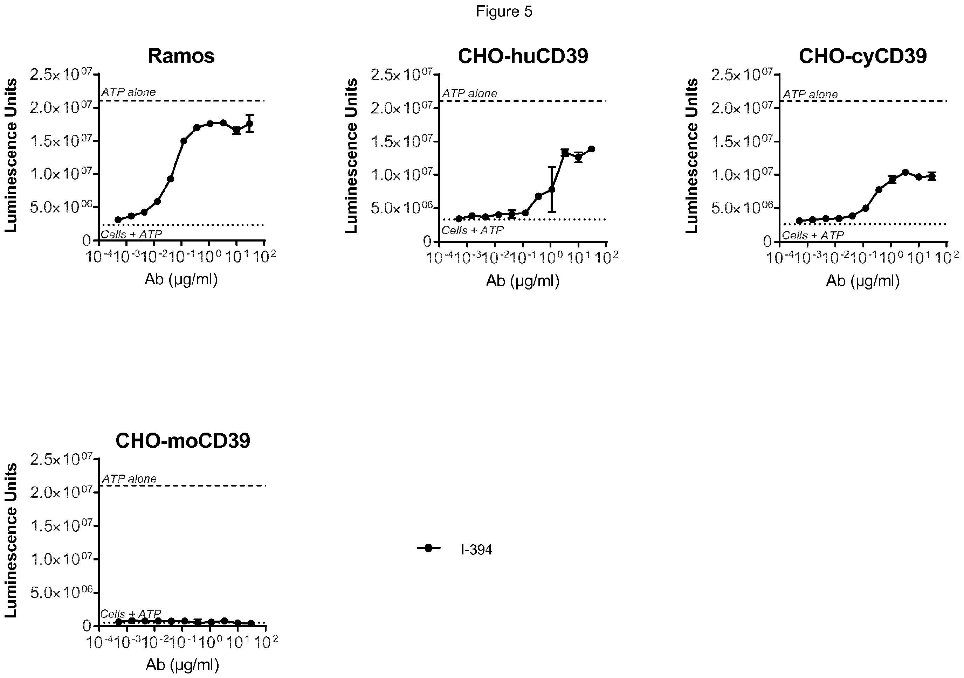

Figure 5 shows antibody I-394 is highly potent at blocking CD39 enzymatic activity in tumor (Ramos) cells, in cells expressing human CD39 (CHO-huCD39), and in cells expressing cynomolgus CD39 (CHO-cyCD39), as assessed by quantifying luminescence units which are proportional to the amount of ATP present.

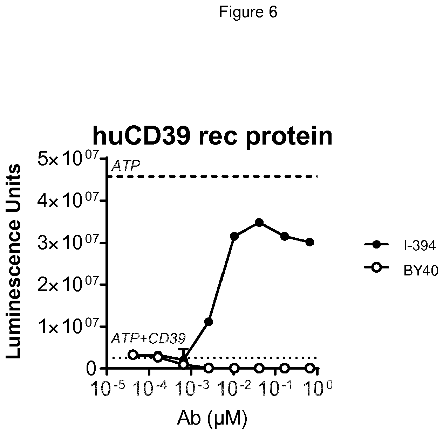

Figure 6 shows antibody I-394 is highly potent at blocking the enzymatic activity of soluble recombinant human CD39 protein, as assessed by quantifying luminescence units which are proportional to the amount of ATP present.

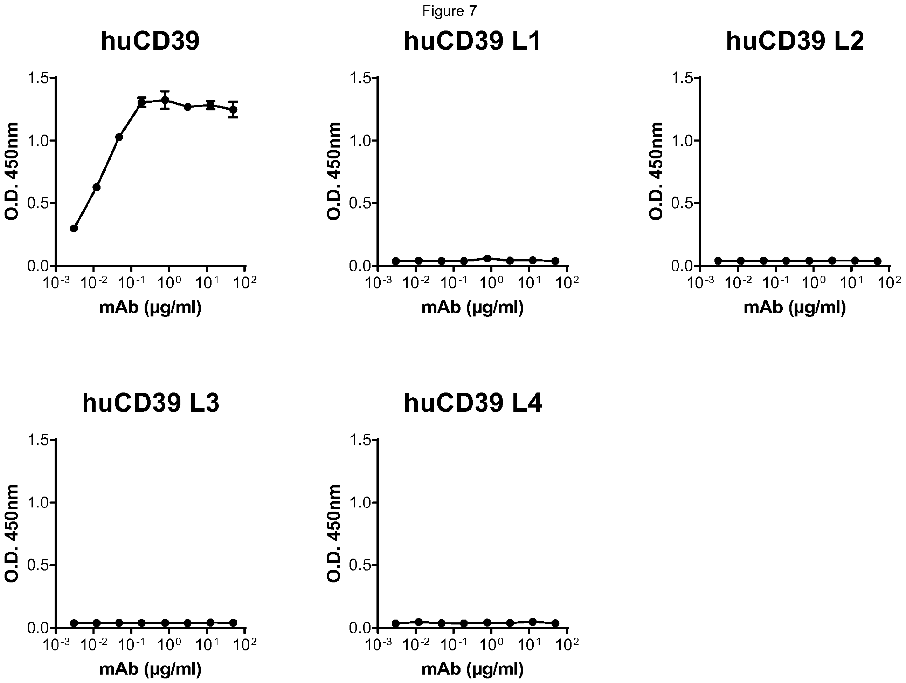

Figure 7 shows antibody I-394 binds to human CD39 but not to any of the human isoforms CD39-L1, -L2, -L3 or -L4, as assessed in an ELISA assay.

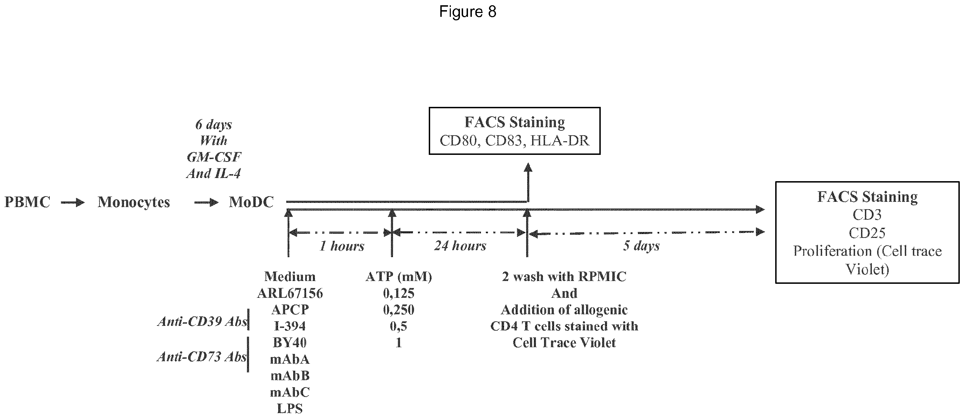

Figure 8 shows the experimental procedure for assessing the effect of ATP-mediated DC activation on CD4 T cells activation, ATP-activated DC were washed and then incubated with allogenic CD4 T cells (ratio 1 MoDC / 4 T cells) for a mixed lymphocytes reaction (MLR) during 5 days. T cells activation and proliferation were analyzed through CD25 expression and Cell Trace Violet dilution by flow cytometry.

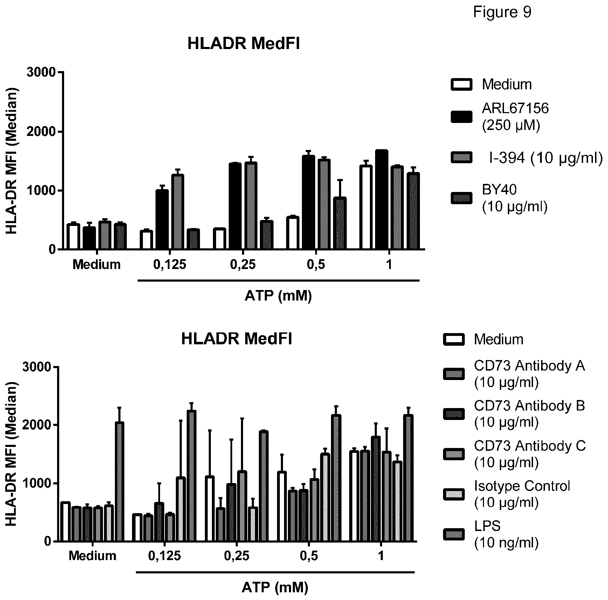

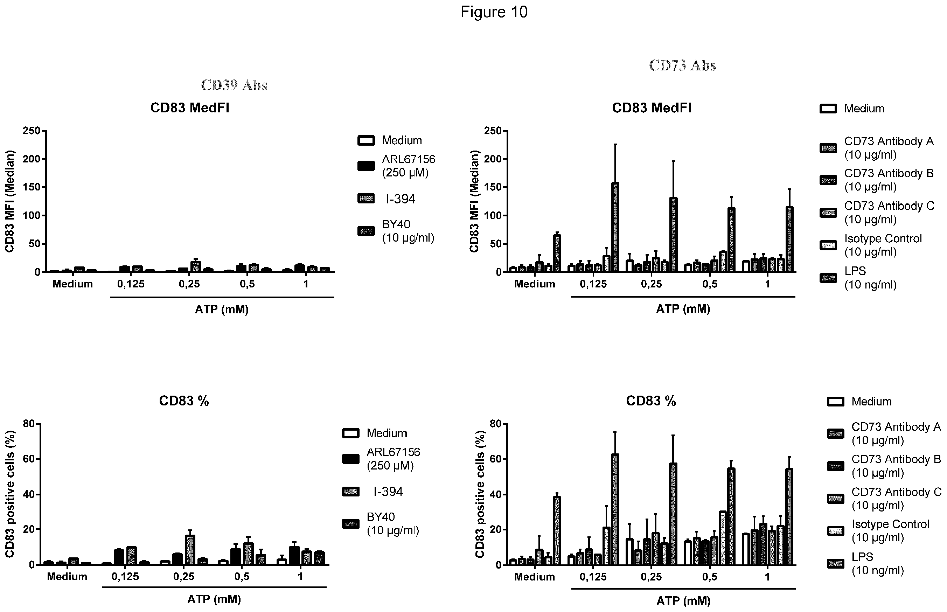

Figure 9 shows HLA-DR expression on moDC and Figure 10 shows CD83 expression on moDC. These figures show that the anti-CD39 blocking antibody I-394 and chemical inhibitors of CD39 lead to moDC activation at each of 0.125 mM, 0.25 mM or 0.5mM. However, anti-CD39 antibody BY40 or anti-CD73 antibodies were not able to favor ATP-induced activation of dendritic cell (DC), suggesting that antibodies are not able to block enzymatic activity sufficiently to avoid ATP catabolism. The legends, top to bottom, correspond to the bars in the graph, from left to right.

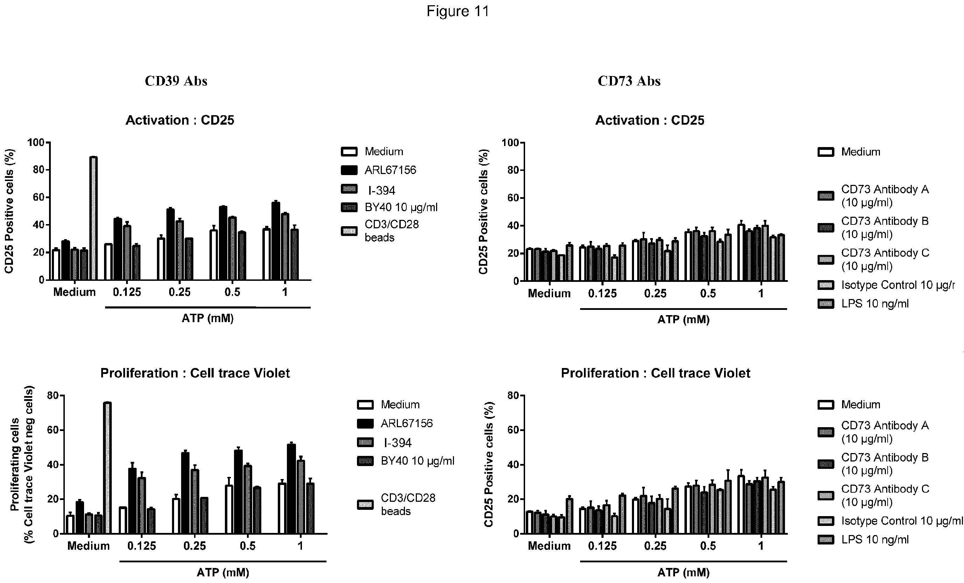

Figure 11 showing CD25 expression shows that MoDC activated in presence of ATP were able to induce T cell activation and proliferation in a MLR assay; the enhancement of ATP-mediated MoDC activation by anti-CD39 blocking antibody I-394 resulted in higher T cell proliferation and activation. The legends, top to bottom, correspond to the bars in the graph, from left to right.

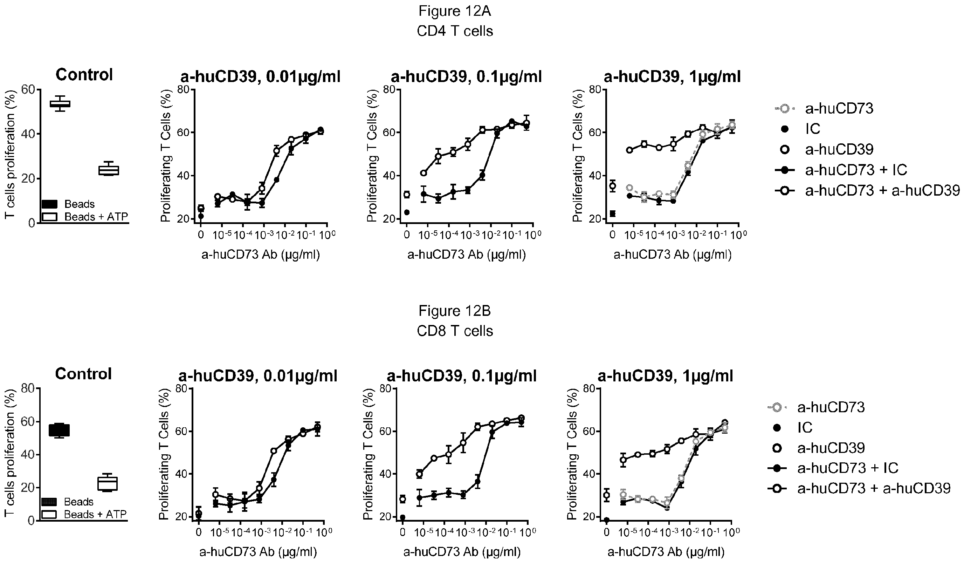

Figure 12A shows the dose range of anti-CD73 antibodies on CD4 T cell proliferation, in the presence of added ATP, at 3 different doses of anti-sCD39 antibodies, either 0.01 µg/ml, 0.1 µg/ml and 1 µg/ml. The anti-CD39 antibodies that are capable of neutralizing soluble human CD39 show a strong potentiation of anti-CD73 antibodies in restoring CD4 T cell proliferation. Figure 12B shows the dose range of anti-CD73 antibodies on CD8 T cell proliferation, in the presence of added ATP, anti-sCD39 antibodies show a strong potentiation of anti-CD73 antibodies in restoring CD8 T cell proliferation.

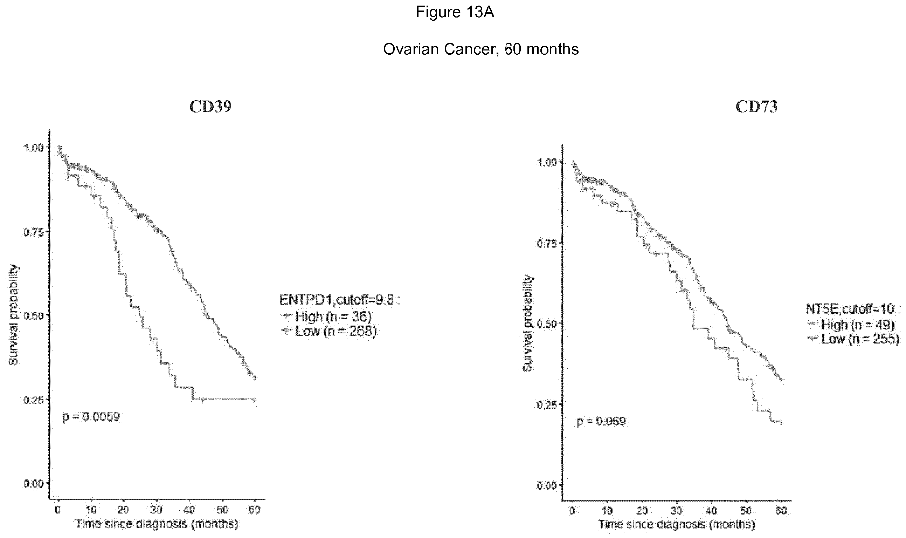

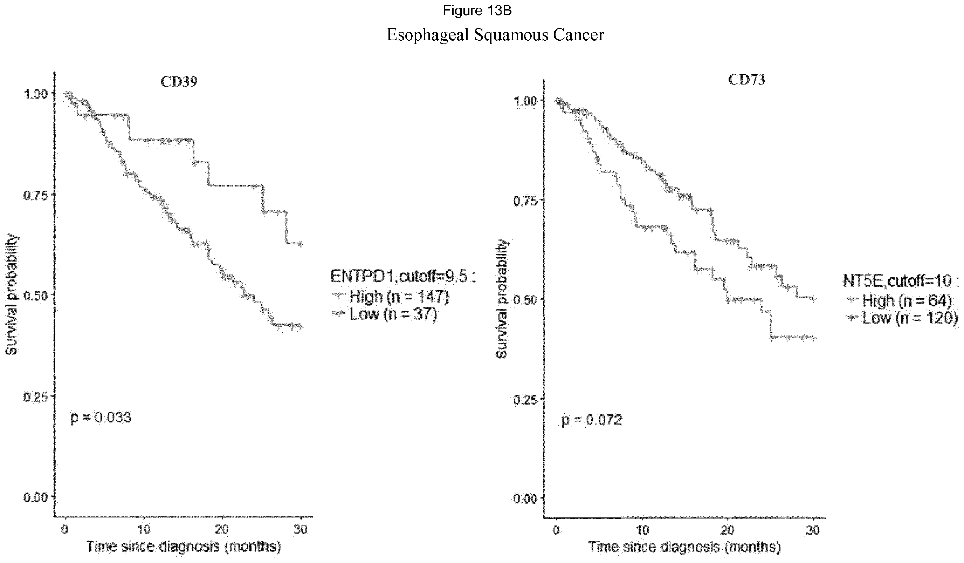

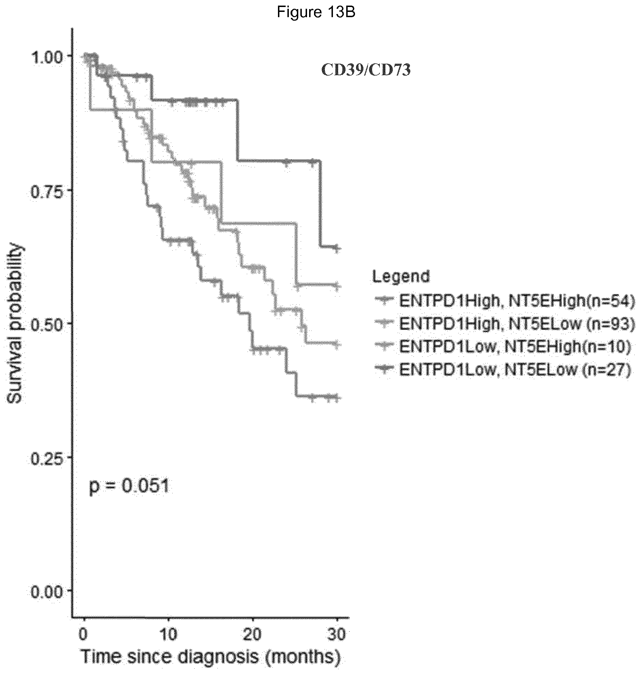

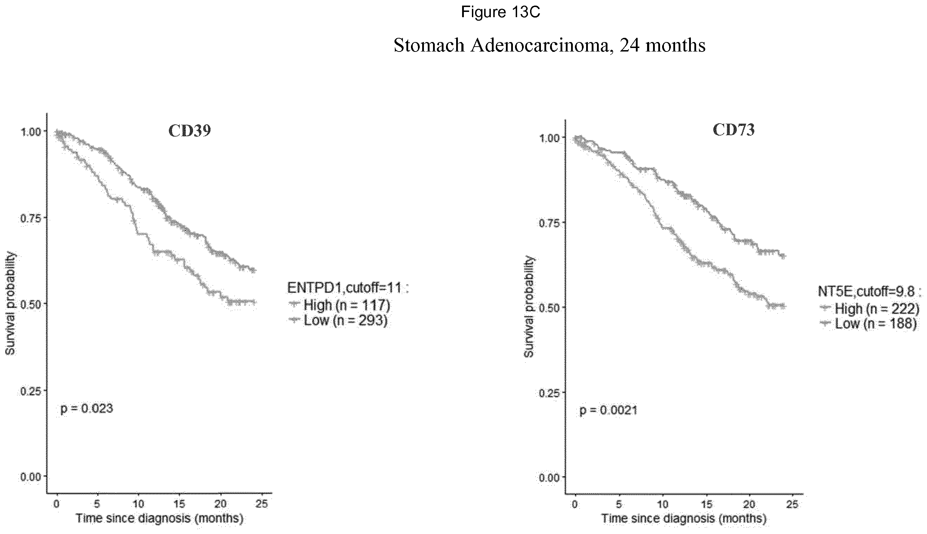

Figures 13A, 13B and 13C show a study correlating CD39 and CD73 gene expression in human cancer samples with survival, taking account of disease stage and time. Figure 13A shows results in ovarian cancer, notably low CD39 expression in cancer samples is correlated with higher survival probability in ovarian cancer; also shown is correlation of high expression CD73 with lower survival probability in ovarian cancer. Figure 13B shows results in esophageal cancer, notably low CD39 expression in cancer samples is correlated with higher survival probability in esophageal cancer; also shown is correlation of high expression CD73 with lower survival probability in esophageal cancer. Figure 13C shows results in stomach adenocarcinoma, notably low CD39 expression in cancer samples is correlated with higher survival probability in stomach cancer; also shown is correlation of high expression CD73 with lower survival probability in stomach cancer.

### DETAILED DESCRIPTION

### Definitions

As used in the specification, "a" or "an" may mean one or more. As used in the claim(s), when used in conjunction with the word "comprising", the words "a" or "an" may mean one or more than one. As used herein "another" may mean at least a second or more.

Where "comprising" is used, this can optionally be replaced by "consisting essentially of" or by "consisting of".

Human CD73, also known as ecto-5'-nucleotidase and as 5-prime-ribonucleotide phosphohydrolase, EC 3.1.3.5, encoded by the *NT5E* gene, exhibits 5'-nucleotidase, notably AMP-, NAD-, and NMN-nucleosidase, activities. CD73 catalyzes the conversion at neutral pH of purine 5-prime mononucleotides to nucleosides, the preferred substrate being AMP. The enzyme consists of a dimer of 2 identical 70-kD subunits bound by a glycosyl phosphatidyl inositol linkage to the external face of the plasma membrane The amino acid sequence of Human CD73 preprotein (monomer), including a signal sequence at amino acids 1-26, is shown in Genbank under accession number NP_002517, and as follows:

MCPRAARAPA TLLLALGAVL WPAAGAWELT ILHTNDVHSR LEQTSEDSSK CVNASRCMGG
VARLFTKVQQ IRRAEPNVLL LDAGDQYQGT IWFTVYKGAE VAHFMNALRY DAMALGNHEF
DNGVEGLIEP LLKEAKFPIL SANIKAKGPL ASQISGLYLP YKVLPVGDEV VGIVGYTSKE
TPFLSNPGTN LVFEDEITAL QPEVDKLKTL NVNKIIALGH SGFEMDKLIA QKVRGVDVVV
GGHSNTFLYT GNPPSKEVPA GKYPFIVTSD DGRKVPVVQA YAFGKYLGYL KIEFDERGNV
ISSHGNPILL NSSIPEDPSI KADINKWRIK LDNYSTQELG KTIVYLDGSS QSCRFRECNM
GNLICDAMIN NNLRHTDEMF WNHVSMCILN GGGIRSPIDE RNNGTITWEN LAAVLPFGGT
FDLVQLKGST LKKAFEHSVH RYGQSTGEFL QVGGIHVVYD LSRKPGDRVV KLDVLCTKCR
VPSYDPLKMD EVYKVILPNF LANGGDGFQM IKDELLRHDS GDQDINVVST YISKMKVIYP
AVEGRIKFST GSHCHGSFSL IFLSLWAVIF VLYQ
(SEQ ID NO: 1).

In the context herein, "inhibit", "inhibiting", "neutralize" or "neutralizing" when referring to the CD73 polypeptide (e.g., "neutralize CD73", "neutralize the activity of CD73" or "neutralize the enzymatic activity of CD73", etc.), refers to a process in which the 5'-nucleotidase (5'-ectonucleotidase) activity of CD73 is inhibited. This comprises, notably the inhibition of CD73-mediated generation of adenosine, i.e. the inhibition of CD73-mediated catabolism of AMP to adenosine. This can be measured for example in a cell-free assay that measures the capacity of a test compound to inhibit the conversion of AMP to adenosine, either directly or indirectly. In one embodiment, an antibody preparation causes at least a 50% decrease in the conversion of AMP to adenosine, at least a 70% decrease in the conversion of AMP to adenosine, or at least an 80% decrease in the conversion of AMP to adenosine, referring, for example, to the assays described herein.

Human CD39, also known as NTPdase1, ENTPD1, ATPDase and vascular ATP diphosphohydrolase, exhibits ATPase activity. CD39 is a membrane bound protein that hydrolyzes extracellular ATP and ADP to AMP, which is further converted to adenosine by another enzyme, 5-prime nucleotidase. The amino acid sequence of the human CD39 mature polypeptide chain is shown in Genbank under accession number P49961 and as follows:

MEDTKESNVK TFCSKNILAI LGFSSIIAVI ALLAVGLTQN KALPENVKYG IVLDAGSSHT
SLYIYKWPAE KENDTGVVHQ VEECRVKGPG ISKFVQKVNE IGIYLTDCME RAREVIPRSQ
HQETPVYLGA TAGMRLLRME SEELADRVLD VVERSLSNYP FDFQGARIIT GQEEGAYGWI
TINYLLGKFS QKTRWFSIVP YETNNQETFG ALDLGGASTQ VTFVPQNQTI ESPDNALQFR
LYGKDYNVYT HSFLCYGKDQ ALWQKLAKDI QVASNEILRD PCFHPGYKKV VNVSDLYKTP
CTKRFEMTLP FQQFEIQGIG NYQQCHQSIL ELFNTSYCPY SQCAFNGIFL PPLQGDFGAF
SAFYFVMKFL NLTSEKVSQE KVTEMMKKFC AQPWEEIKTS YAGVKEKYLS EYCFSGTYIL
SLLLQGYHFT ADSWEHIHFI GKIQGSDAGW TLGYMLNLTN MIPAEQPLST PLSHSTYVFL
MVLFSLVLFT VAIIGLLIFH KPSYFWKDMV

(SEQ ID NO: 2).

In the context herein, "inhibit", "inhibiting", "neutralize" or "neutralizing" when referring to the CD39 polypeptide (e.g., "neutralize CD39", "neutralize the activity of CD39" or "neutralize the enzymatic activity of CD39", etc.), refers to a process in which the ATP hydrolysis (ATPase) activity of CD39 is inhibited. This comprises, notably the inhibition of CD39-mediated generation of AMP and/or ADP, i.e., the inhibition of CD39-mediated catabolism of ATP to AMP and/or ADP. This can be measured for example in a cellular assay that measures the capacity of a test compound to inhibit the conversion of ATP to AMP and/or ADP, either directly or indirectly. For example, disappearance of ATP and/or generation of AMP can be assessed, as described herein. In one embodiment, an antibody preparation causes at least a 60% decrease in the conversion of ATP to AMP, at least a 70% decrease in the conversion of ATP to AMP, or at least an 80% or 90% decrease in the conversion of ATP to AMP, referring, for example, to the assays described herein (e.g., disappearance of ATP and/or generation of AMP).

"EC₅₀" with respect to an agent and a particular activity (e.g. binding to a cell, inhibition of enzymatic activity, activation or inhibition of an immune cell), refers to the efficient concentration of the agent which produces 50% of its maximum response or effect with respect to such activity. "EC₁₀₀" with respect to an agent and a particular activity refers to the efficient concentration of the agent which produces its substantially maximum response with respect to such activity.

The term "antibody," as used herein, refers to polyclonal and monoclonal antibodies. Depending on the type of constant domain in the heavy chains, antibodies are assigned to one of five major classes: IgA, IgD, IgE, IgG, and IgM. Several of these are further divided into subclasses or isotypes, such as IgG1, IgG2, IgG3, IgG4, and the like. An exemplary immunoglobulin (antibody) structural unit comprises a tetramer. Each tetramer is composed of two identical pairs of polypeptide chains, each pair having one "light" (about 25 kDa) and one "heavy" chain (about 50-70 kDa). The N-terminus of each chain defines a variable region of about 100 to 110 or more amino acids that is primarily responsible for antigen recognition. The terms variable light chain (V_{L}) and variable heavy chain (V_{H}) refer to these light and heavy chains respectively. The heavy-chain constant domains that correspond to the different classes of immunoglobulins are termed "alpha," "delta," "epsilon," "gamma" and "mu," respectively. The subunit structures and three-dimensional configurations of different classes of immunoglobulins are well known. IgG are the exemplary classes of antibodies employed herein because they are the most common antibodies in the physiological situation and because they are most easily made in a laboratory setting. Optionally the antibody is a monoclonal antibody. Particular examples of antibodies are humanized, chimeric, human, or otherwise-human-suitable antibodies. "Antibodies" also includes any fragment or derivative of any of the herein described antibodies.

The term "specifically binds to" means that an antibody can bind preferably in a competitive binding assay to the binding partner, e.g., CD39, CD73, as assessed using either recombinant forms of the proteins, epitopes therein, or native proteins present on the surface of isolated target cells. Competitive binding assays and other methods for determining specific binding are well known in the art. For example binding can be detected via radiolabels, physical methods such as mass spectrometry, or direct or indirect fluorescent labels detected using, e.g., cytofluorometric analysis (e.g., FACScan). Binding above the amount seen with a control, non-specific agent indicates that the agent binds to the target.

When an antibody is said to "compete with" a particular monoclonal antibody, it means that the antibody competes with the monoclonal antibody in a binding assay using either recombinant molecules (e.g., CD39, CD73) or surface expressed molecules (e.g., CD39, CD73). For example, if a test antibody reduces the binding of an antibody having a heavy chain variable region and a light chain variable region of any of antibodies 11E1, 6E1, 3C12 or 8C7 to a CD73 polypeptide or CD73-expressing cell in a binding assay, the antibody is said to "compete" respectively with such antibody. For example, if a test antibody reduces the binding of an antibody having a heavy chain of SEQ ID NO: 6 and a light chain of SEQ ID NO: 7 to a CD39 polypeptide or CD39-expressing cell in a binding assay, the antibody is said to "compete" respectively with such antibody.

The term "affinity", as used herein, means the strength of the binding of an antibody to an epitope. The affinity of an antibody is given by the dissociation constant Kd, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant Kₐ is defined by 1/Kd. Methods for determining the affinity of mAbs can be found in Harlow, et al., Antibodies: A Laboratory Manual, Cold Spring Harbor Laboratory Press, Cold Spring Harbor, N.Y., 1988), Coligan et al., eds., Current Protocols in Immunology, Greene Publishing Assoc. and Wiley Interscience, N.Y., (1992, 1993), and Muller, Meth. Enzymol. 92:589-601 (1983). One standard method well known in the art for determining the affinity of mAbs is the use of surface plasmon resonance (SPR) screening (such as by analysis with a BIAcore^{™} SPR analytical device).

Within the context herein a "determinant" designates a site of interaction or binding on a polypeptide.

The term "epitope" refers to an antigenic determinant, and is the area or region on an antigen to which an antibody binds. A protein epitope may comprise amino acid residues directly involved in the binding as well as amino acid residues which are effectively blocked by the specific antigen binding antibody or peptide, *i.e.*, amino acid residues within the "footprint" of the antibody. It is the simplest form or smallest structural area on a complex antigen molecule that can combine with e.g., an antibody or a receptor. Epitopes can be linear or conformational/structural. The term "linear epitope" is defined as an epitope composed of amino acid residues that are contiguous on the linear sequence of amino acids (primary structure). The term "conformational or structural epitope" is defined as an epitope composed of amino acid residues that are not all contiguous and thus represent separated parts of the linear sequence of amino acids that are brought into proximity to one another by folding of the molecule (secondary, tertiary and/or quaternary structures). A conformational epitope is dependent on the 3-dimensional structure. The term "conformational" is therefore often used interchangeably with "structural".

The term "deplete" or "depleting", with respect to CD73- or CD39-expressing cells, means a process, method, or compound that results in killing, elimination, lysis or induction of such killing, elimination or lysis, so as to negatively affect the number of such CD73- or CD39-expressing cells present in a sample or in a subject.

The term "internalization", used interchangeably with "intracellular internalization", refers to the molecular, biochemical and cellular events associated with the process of translocating a molecule from the extracellular surface of a cell to the intracellular surface of a cell. The processes responsible for intracellular internalization of molecules are well-known and can involve, *inter alia*, the internalization of extracellular molecules (such as hormones, antibodies, and small organic molecules); membrane-associated molecules (such as cell-surface receptors); and complexes of membrane-associated molecules bound to extracellular molecules (for example, a ligand bound to a transmembrane receptor or an antibody bound to a membrane-associated molecule). Thus, "inducing and/or increasing internalization" comprises events wherein intracellular internalization is initiated and/or the rate and/or extent of intracellular internalization is increased.

The term "agent" is used herein to denote a chemical compound, a mixture of chemical compounds, a biological macromolecule, or an extract made from biological materials. The term "therapeutic agent" refers to an agent that has biological activity.

For the purposes herein, a "humanized" or "human" antibody refers to an antibody in which the constant and variable framework region of one or more human immunoglobulins is fused with the binding region, e.g., the CDR, of an animal immunoglobulin. Such antibodies are designed to maintain the binding specificity of the non-human antibody from which the binding regions are derived, but to avoid an immune reaction against the non-human antibody. Such antibodies can be obtained from transgenic mice or other animals that have been "engineered" to produce specific human antibodies in response to antigenic challenge (see, e.g., Green et al. (1994) Nature Genet 7:13; Lonberg et al. (1994) Nature 368:856; Taylor et al. (1994) Int Immun 6:579). A fully human antibody also can be constructed by genetic or chromosomal transfection methods, as well as phage display technology, all of which are known in the art (see, e.g., McCafferty et al. (1990) Nature 348:552-553). Human antibodies may also be generated by in vitro activated B cells (see, e.g., U.S. Pat. Nos. 5,567,610 and 5,229,275).

A "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity.

The term "hypervariable region" when used herein refers to the amino acid residues of an antibody that are responsible for antigen binding. The hypervariable region generally comprises amino acid residues from a "complementarity-determining region" or "CDR" (e.g. residues 24-34 (L1), 50-56 (L2) and 89-97 (L3) in the light-chain variable domain and 31-35 (H1), 50-65 (H2) and 95-102 (H3) in the heavy-chain variable domain; Kabat et al. 1991) and/or those residues from a "hypervariable loop" (e.g. residues 26-32 (L1), 50-52 (L2) and 91-96 (L3) in the light-chain variable domain and 26-32 (H1), 53-55 (H2) and 96-101 (H3) in the heavy-chain variable domain; Chothia and Lesk, J. Mol. Biol 1987;196:901-917), or a similar system for determining essential amino acids responsible for antigen binding. Typically, the numbering of amino acid residues in this region is performed by the method described in Kabat et al., supra. Phrases such as "Kabat position", "variable domain residue numbering as in Kabat" and "according to Kabat" herein refer to this numbering system for heavy chain variable domains or light chain variable domains. Using the Kabat numbering system, the actual linear amino acid sequence of a peptide may contain fewer or additional amino acids corresponding to a shortening of, or insertion into, a FR or CDR of the variable domain. For example, a heavy chain variable domain may include a single amino acid insert (residue 52a according to Kabat) after residue 52 of CDR H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

By "framework" or "FR" residues as used herein is meant the region of an antibody variable domain exclusive of those regions defined as CDRs. Each antibody variable domain framework can be further subdivided into the contiguous regions separated by the CDRs (FR1, FR2, FR3 and FR4).

The terms "Fc domain," "Fc portion," and "Fc region" refer to a C-terminal fragment of an antibody heavy chain, e.g., from about amino acid (aa) 230 to about aa 450 of human γ (gamma) heavy chain or its counterpart sequence in other types of antibody heavy chains (e.g., α, δ, ε and µ for human antibodies), or a naturally occurring allotype thereof. Unless otherwise specified, the commonly accepted Kabat amino acid numbering for immunoglobulins is used throughout this disclosure (see Kabat et al. (1991 ) Sequences of Protein of Immunological Interest, 5th ed., United States Public Health Service, National Institute of Health, Bethesda, MD).

The terms "isolated", "purified" or "biologically pure" refer to material that is substantially or essentially free from components which normally accompany it as found in its native state. Purity and homogeneity are typically determined using analytical chemistry techniques such as polyacrylamide gel electrophoresis or high performance liquid chromatography. A protein that is the predominant species present in a preparation is substantially purified.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (nonrecombinant) form of the cell or express native genes that are otherwise abnormally expressed, under expressed or not expressed at all.

Within the context herein, the term antibody that "binds" a polypeptide or epitope designates an antibody that binds said determinant with specificity and/or affinity.

The term "identity" or "identical", when used in a relationship between the sequences of two or more polypeptides, refers to the degree of sequence relatedness between polypeptides, as determined by the number of matches between strings of two or more amino acid residues. "Identity" measures the percent of identical matches between the smaller of two or more sequences with gap alignments (if any) addressed by a particular mathematical model or computer program (i.e., "algorithms"). Identity of related polypeptides can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988).

Methods for determining identity are designed to give the largest match between the sequences tested. Methods of determining identity are described in publicly available computer programs. Computer program methods for determining identity between two sequences include the GCG program package, including GAP (Devereux et al., Nucl. Acid. Res. 12, 387 (1984); Genetics Computer Group, University of Wisconsin, Madison, Wis.), BLASTP, BLASTN, and FASTA (Altschul et al., J. Mol. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.

### Agents that inhibit sCD39 protein

The agent that binds and inhibits CD39 for use in accordance herein can be an antigen binding domain or a protein comprising such, optionally an antibody or antibody fragment, that binds to and inhibits or neutralizes the ATPase activity of a soluble CD39 protein (sCD39). In one aspect of the disclosure, the sCD39 protein lacks the two transmembrane domains (i.e. the transmembrane domains near the N- and C-terminal ends) found in membrane bound CD39. In one aspect of the disclosure, sCD39 is a non-membrane bound sCD39 protein found in circulation, e.g., in a human individual. In one aspect of the disclosure, sCD39 comprises or consists of the amino acid sequence of SEQ ID NO: 5 (optionally further comprising a C-terminal tag or another non-CD39-derived amino acid sequence). In one aspect of the disclosure, the protein, antibody or antibody fragment inhibits the ATPase activity of sCD39 when incubated with sCD39 in solution, e.g., according to the methods disclosed herein. In one embodiment, the protein, antibody or antibody fragment specifically binds the human CD39 protein, both in soluble (extracellular domain protein) and in membrane-bound form.

The antibodies that neutralize the activity of sCD39 (and memCD39) may, in addition to use as bivalent binders, also be effective as monovalent binders, whether they are targeting memCD39 in addition to sCD39. Consequently, in one aspect of the disclosure, an agent that inhibits CD39 may be an antigen binding protein that binds monovalently to a human CD39 protein (sCD39 and optionally further memCD39) and neutralizes the enzymatic (ATPase) activity thereof. The antigen binding protein can optionally be specified as binding to a single CD39 protein and/or bearing a single antigen binding domain capable of binding to a CD39 protein. In one aspect of the disclosure, provided is an antibody fragment, optionally a F(ab) fragment, a single chain antibody, a scFv, a multispecific antibody, that binds monovalently to a human CD39 protein (sCD39 and/or memCD39) and neutralizes the enzymatic (ATPase) activity thereof. In one embodiment, a CD39-neutralizing antigen binding protein that binds monovalently to a human CD39 protein is a multi-specific antigen binding protein, e.g., a multi-specific antibody, a bi-specific antibody, a tri-specific antibody, etc. In one aspect of the disclosure, a CD39-neutralizing antigen binding protein that binds monovalently to a human CD39 protein further binds to and inhibits the enzymatic activity of a human CD73 protein. In one aspect of the disclosure, provided is use of a CD39-neutralizing antigen binding protein that binds monovalently to a human CD39 protein comprises a first (or a single) antigen binding domain that binds CD39 (sCD39 and/or memCD39) and a second (and optionally further) antigen binding domain(s) that binds and inhibits a CD73 protein.

In one aspect of the disclosure, the anti-CD39 antibody does not increase or induce intracellular internalization of, or more generally down-modulation of, cell surface-expressed CD39 and/or does not depend thereupon for its CD39 inhibitory activity.

In one aspect, an anti-CD39 antibody is capable of: (a) inhibiting the enzymatic activity of membrane-bound CD39 protein (e.g., comprising an amino acid sequence of SEQ ID NO: 2) expressed at the surface of cells, and (b) inhibiting the enzymatic activity of soluble CD39 protein (e.g., having an amino acid sequence of SEQ ID NO: 5).

In one aspect of the disclosure, an anti-CD39 antibody does not substantially bind (e.g., via its Fc domain) to human Fcγ receptors (e.g., CD16, CD32a, CD32b, CD64) and/or C1q, and/or do not substantially directing ADCC and/or CDC toward a CD39-expressing cell. Optionally, the antibody retains an Fc domain (e.g. of human IgG isotype) and retains binding to human FcRn.

In one aspect of the disclosure, the CD39 neutralizing antibodies can be characterized by being capable of causing a decrease in the ATPase activity of a sCD39 polypeptide and/or of a monomeric CD39 polypeptide, optionally causing a decrease of AMP generation by a soluble monomeric human CD39 protein, e.g. a CD39 protein consisting of the amino acid sequence of SEQ ID NO: 5, by at least 50%, 60%, 70%, 80% or 90%.

In one aspect of the disclosure, the CD39 neutralizing antibodies can be characterized by being capable of causing a decrease in cells' ATPase activity of CD39, optionally causing a decrease of AMP generation by a CD39-expressing cell, by at least 50%, 60%, 70%, 80% or 90%. In one aspect of the disclosure, the CD39-neutralizing antibodies can be characterized by an EC₅₀ for inhibition of ATPase activity (e.g., EC₅₀ for inhibition of AMP generation by a CD39-expressing cell) of CD39 expressed by a cell of no more than 1 µg/ml, optionally no more than 0.5 µg/ml, optionally no more than 0.2 µg/ml.

An antigen-binding compound can be produced as further described herein, and at any desired stage be assessed for its ability to inhibit the enzymatic activity of CD39, notably to block the ATPase activity of sCD39 and to reduce the production of ADP and AMP (and, together with CD73, adenosine) by soluble CD39 protein and optionally further by a CD39-expressing cell, and in turn restore the activity of and/or relieve the adenosine-mediated inhibition of lymphocyte activation and/or proliferation.

The inhibitory activity (e.g., immune enhancing potential) of an antibody can be assessed for example, in an assay to detect the disappearance (hydrolysis) of ATP and/or the generation of AMP.

The ability of an antibody to inhibit soluble recombinant human CD39 protein can be tested by detecting ATP after incubating test antibody with soluble CD39 protein (e.g., the CD39 protein having the amino acid sequence of SEQ ID NO: 5, as produced in Example 1, optionally further comprising a purification tag or other functional or non-functional non-CD39-derived amino acid sequence). See, e.g., Example 1. Briefly, ATP can be quantified using the Cell Titer Glo^{™} (Promega), in an assay in which dose ranges of test antibody are incubated with soluble recombinant human CD39 protein described in Example 1, for 1 hour at 37°C. 20 µM ATP are added to the plates for 30 additional minutes at 37°C before addition of CTG reagent. Emitted light is quantified using an Enspire^{™} luminometer after a short incubation period of 5 min in the dark.

The ability of an antibody to inhibit cells expressing CD39 protein can be tested by detecting ATP after incubating test antibody with cells (e.g., Ramos cells, cells transfected with CD39, etc.). See, e.g., Example 1. Cells can be incubated for 1 hour at 37°C with test antibody. Cells are then incubated with 20 µM ATP for 1 additional hour at 37°C. Plates are centrifuged for 2 min at 400g and cell supernatant are transferred in a luminescence microplate (white wells). CTG is added to the supernatant and emitted light is quantified after a 5 min incubation in the dark using an Enspire^{™} luminometer. Anti-CD39 antibody efficacy is determined by comparing emitted light in presence of antibody with ATP alone (maximal light emission) and ATP together with cells (minimal light emission).

A decrease in hydrolysis of ATP into AMP, and/or an increase of ATP and/or a decrease in generation of AMP, in the presence of antibody indicate the antibody inhibits CD39. In one embodiment, an antibody preparation is capable of causing at least a 60% decrease in the enzymatic activity of a CD39 polypeptide expressed by a cell, preferably the antibody causes at least a 70%, 80% or 90% decrease in the enzymatic activity of a CD39 polypeptide in a cell, as assessed by detecting ATP using the Cell Titer Glo^{™} (Promega) after incubating cells expressing CD39 polypeptide (e.g., Ramos cells) with a test antibody, e.g., as in Examples herein. In one aspect of the disclosure, an antibody is characterized by an EC₅₀ for causing a decrease in the enzymatic activity of a CD39 polypeptide expressed by a cell, (e.g. as assessed by detecting ATP), of no more than the EC₅₀ observed with an anti-CD39 antibody described herein, e.g. I-394, I-395, I-396 or I-399, optionally an EC₅₀ of no more than 2-log or 1-log greater than that of an anti-CD39 antibody described herein, e.g. I-394, I-395, I-396 or I-399.

In one aspect of the disclosure, an antibody preparation is capable of causing at least a 60% decrease in the enzymatic activity of a soluble recombinant CD39 polypeptide, preferably at least a 70%, 80% or 90% decrease in the enzymatic activity of a soluble recombinant CD39 polypeptide, as assessed by detecting ATP using the Cell Titer Glo^{™} (Promega) after incubating soluble recombinant CD39 polypeptide with a test antibody, e.g., as in Example 1.

The activity of an antibody can also be measured in an indirect assay for its ability to modulate the activity of immune cells (e.g., adenosine receptor-expressing immune cells; A2A-receptor expressing cells), for example to relieve the adenosine-mediated inhibition of lymphocyte activity, or to cause the activation of lymphocyte activity. This can be addressed, for example, using a cytokine-release assay. In another example, an antibody can be evaluated in an indirect assay for its ability to modulate the proliferation of lymphocytes.

In one example, provided is a method for producing or identifying an anti-CD39 antibody or antigen binding domain capable of use in the methods of the disclosure (e.g. for use in treatment of CD73-positive cancers; for use combination with an agent that inhibits the enzymatic activity of CD73), the method comprising the steps of:
(a) providing a plurality of antibodies that bind a human CD39 polypeptide,
(b) bringing each of the antibodies into contact with soluble extracellular domain CD39 protein and assessing neutralization of ATPase activity thereof, and
(c) selecting an antibody of step (b) that results in a neutralization of ATPase activity, by at least 70%, optionally 80% or optionally 90%.

Optionally, the method further comprises the steps of:
(d) assessing the ability of the antibody selected in step (c) to potentiate the activity of an agent that inhibits the enzymatic activity of a CD73 protein; and
(e) selecting an antibody of step (d) that potentiates the activity of an agent that inhibits the enzymatic activity of a CD73 protein.

Optionally, step (d) comprises bringing the antibody selected in step (c) and an agent that inhibits the enzymatic activity of a CD73 protein into contact with cells, optionally human T cells, optionally CD4 T cells, optionally CD8 T cells, and assessing activation and/or proliferation of the cells. Optionally, the assay is conducted in the presence of ATP (e.g., exogenously added ATP).

Optionally, in any aspect herein, a neutralizing anti-CD39 antibody binds an antigenic determinant present on both sCD39 and CD39 expressed at the cell surface (memCD39).

Optionally, in any aspect herein, the binding molecule (e.g., anti-CD39 antibody) comprises the variable heavy chain domain (V_{H}) comprising a light chain CDR1, 2 and 3 as described herein, and a variable light chain domain (V_{L}) comprising a heavy chain CDR1, 2 and 3 as described herein. In one aspect, the antibody may comprise a heavy chain comprising the three CDRs of the heavy chain variable region (VH) of antibody I-394 and a light chain comprising the three CDRs of the light chain variable region (VL) of antibody I-394.

Optionally, in any aspect herein, an anti-CD39 antibody can be characterized as comprising a heavy chain comprising an amino acid sequence at least 60%, 70%, 80%, 85%, 90% or 95% amino acid identity to a heavy chain as described herein (e.g., the heavy chain of SEQ ID NO: 52), and a light chain comprising an amino acid sequence at least 60%, 70%, 80%, 85%, 90% or 95% amino acid identity to a light chain as described herein (e.g., the light chain of SEQ ID NO: 53).

Optionally, in any aspect herein, an anti-CD39 antibody can be characterized as being a function-conservative variant of any of the antibodies disclosed herein. "Function-conservative variants" are those in which a given amino acid residue in a protein or enzyme has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Amino acids other than those indicated as conserved may differ in a protein so that the percent protein or amino acid sequence similarity between any two proteins of similar function may vary and may be, for example, from 70% to 99% as determined according to an alignment scheme such as by the Cluster Method, wherein similarity is based on the MEGALIGN algorithm. A "function-conservative variant" also includes a polypeptide which has at least 60% amino acid identity as determined by BLAST or FASTA algorithms, preferably at least 75%, more preferably at least 85%, still preferably at least 90%, and even more preferably at least 95%, and which has the same or substantially similar properties or functions as the native or parent protein to which it is compared.

Optionally, in any aspect herein, anti-CD39 antibody comprises a human Fc domain that is modified (compared to a wild-type Fc domain of the same isotype) to reduce binding between the Fc domain and human CD16A, CD16B, CD32A, CD32B and/or CD64 polypeptides, optionally wherein the antibody comprises: (i) a heavy chain comprising CDR 1, 2 and 3 of the heavy chain variable region of SEQ ID NO: 6 and (ii) a light chain comprising CDR 1, 2 and 3 of the light chain variable region of SEQ ID NO: 7. In one aspect, the Fc domain is modified (compared to a wild-type Fc domain of the same isotype) to reduce binding between the Fc domain and human C1q polypeptide. In one embodiment, the antibody comprises an amino acid substitution in a heavy chain constant region at any one, two, three, four, five or more of residues selected from the group consisting of: 220, 226, 229, 233, 234, 235, 236, 237, 238, 243, 264, 268, 297, 298, 299, 309, 310, 318, 320, 322, 327, 330 and 331 (Kabat EU numbering). In one embodiment, the antibody has an amino acid substitution in a heavy chain constant region at any three, four, five or more of residues selected from the group consisting of: 234, 235, 237, 322, 330 and 331. In one embodiment, the antibody comprises an Fc domain comprising an amino acid sequence of any of SEQ ID NOS: 59-62.

In one aspect of the disclosure, an antibody comprises a heavy chain constant region comprising the amino acid sequence below, or an amino acid sequence at least 90%, 95% or 99% identical thereto but retaining the amino acid residues at Kabat positions 234, 235 and 331 (underlined):

A S T K G P S V F P L A P S S K S T S G G T A A L G C L V K D Y F P
E P V T V S W N S G A L T S G V H T F P A V L Q S S G L Y S L S S V
V T V P S S S L G T Q T Y I C N V N H K P S N T K V D K R V E P K S
C D K T H T C P P C P A P E A E G G P S V F L F P P K P K D T L M I
S R T P E V T C V V V D V S H E D P E V K F N W Y V D G V E V H N A
K T K P R E E Q Y N S T Y R V V S V L T V L H Q D W L N G K E Y K C
K V S N K A L P A S I E K T I S K A K G Q P R E P Q V Y T L P P S R
E E M T K N Q V S L T C L V K G F Y P S D I A V E W E S N G Q P E N
N Y K T T P P V L D S D G S F F L Y S K L T V D K S R W Q Q G N V F
S C S V M H E A L H N H Y T Q K S L S L S P G K (SEQ ID NO: 59).

In one aspect of the disclosure, an antibody comprises a heavy chain constant region comprising the amino acid sequence below, or an amino acid sequence at least 90%, 95% or 99% identical thereto but retaining the amino acid residues at Kabat positions 234, 235 and 331 (underlined):

A S T K G P S V F P L A P S S K S T S G G T A A L G C L V K D Y F P
E P V T V S W N S G A L T S G V H T F P A V L Q S S G L Y S L S S V
V T V P S S S L G T Q T Y I C N V N H K P S N T K V D K R V E P K S
C D K T H T C P P C P A P E F E G G P S V F L F P P K P K D T L M I
S R T P E V T C V V V D V S H E D P E V K F N W Y V D G V E V H N A
K T K P R E E Q Y N S T Y R V V S V L T V L H Q D W L N G K E Y K C
K V S N K A L P A S I E K T I S K A K G Q P R E P Q V Y T L P P S R
E E M T K N Q V S L T C L V K G F Y P S D I A V E W E S N G Q P E N
N Y K T T P P V L D S D G S F F L Y S K L T V D K S R W Q Q G N V F
S C S V M H E A L H N H Y T Q K S L S L S P G K (SEQ ID NO: 60).

In one aspect of the disclosure, an antibody comprises a heavy chain constant region comprising the amino acid sequence below, or an amino acid sequence at least 90%, 95% or 99% identical thereto but retaining the amino acid residues at Kabat positions 234, 235, 237, 330 and 331 (underlined):

A S T K G P S V F P L A P S S K S T S G G T A A L G C L V K D Y F P
E P V T V S W N S G A L T S G V H T F P A V L Q S S G L Y S L S S V
V T V P S S S L G T Q T Y I C N V N H K P S N T K V D K R V E P K S
C D K T H T C P P C P A P E A E G A P S V F L F P P K P K D T L M I
S R T P E V T C V V V D V S H E D P E V K F N W Y V D G V E V H N A
K T K P R E E Q Y N S T Y R V V S V L T V L H Q D W L N G K E Y K C
K V S N K A L P S S I E K T I S K A K G Q P R E P Q V Y T L P P S R
E E M T K N Q V S L T C L V K G F Y P S D I A V E W E S N G Q P E N
N Y K T T P P V L D S D G S F F L Y S K L T V D K S R W Q Q G N V F
S C S V M H E A L H N H Y T Q K S L S L S P G K (SEQ ID NO: 61).

In one aspect, an antibody comprises a heavy chain constant region comprising the amino acid sequence below, or a sequence at least 90%, 95% or 99% identical thereto but retaining the amino acid residues at Kabat positions 234, 235, 237 and 331 (underlined):

A S T K G P S V F P L A P S S K S T S G G T A A L G C L V K D Y F P
E P V T V S W N S G A L T S G V H T F P A V L Q S S G L Y S L S S V
V T V P S S S L G T Q T Y I C N V N H K P S N T K V D K R V E P K S
C D K T H T C P P C P A P E A E G A P S V F L F P P K P K D T L M I
S R T P E V T C V V V D V S H E D P E V K F N W Y V D G V E V H N A
K T K P R E E Q Y N S T Y R V V S V L T V L H Q D W L N G K E Y K C
K V S N K A L P A S I E K T I S K A K G Q P R E P Q V Y T L P P S R
E E M T K N Q V S L T C L V K G F Y P S D I A V E W E S N G Q P E N
N Y K T T P P V L D S D G S F F L Y S K L T V D K S R W Q Q G N V F
S C S V M H E A L H N H Y T Q K S L S L S P G K (SEQ ID NO: 62).

Binding of anti-CD39 antibody to cells transfected with CD39 mutants can be measured and compared to the ability of anti-CD39 antibody to bind wild-type CD39 polypeptide (e.g., SEQ ID NO: 2). A reduction in binding between an anti-CD39 antibody and a mutant CD39 polypeptide (e.g., a mutant of Table 1) means that there is a reduction in binding affinity (e.g., as measured by known methods such FACS testing of cells expressing a particular mutant, or by Biacore testing of binding to mutant polypeptides) and/or a reduction in the total binding capacity of the anti- CD39 antibody (e.g., as evidenced by a decrease in Bmax in a plot of anti-CD39 antibody concentration versus polypeptide concentration). A significant reduction in binding indicates that the mutated residue is directly involved in binding to the anti-CD39 antibody or is in close proximity to the binding protein when the anti-CD39 antibody is bound to CD39.

In some aspects, a significant reduction in binding means that the binding affinity and/or capacity between an anti-CD39 antibody and a mutant CD39 polypeptide is reduced by greater than 40 %, greater than 50 %, greater than 55 %, greater than 60 %, greater than 65 %, greater than 70 %, greater than 75 %, greater than 80 %, greater than 85 %, greater than 90% or greater than 95% relative to binding between the antibody and a wild type CD39 polypeptide. In certain aspects, binding is reduced below detectable limits. In some aspects, a significant reduction in binding is evidenced when binding of an anti-CD39 antibody to a mutant CD39 polypeptide is less than 50% (e.g., less than 45%, 40%, 35%, 30%, 25%, 20%, 15% or 10%) of the binding observed between the anti-CD39 antibody and a wild-type CD39 polypeptide.

In one aspect, the antibody binds an epitope on CD39 comprising an amino acid residue (e.g., one, two or three of the residues) selected from the group consisting of R138, M139 and E142 (with reference to SEQ ID NO: 2).

In one aspect, an anti-CD39 antibody exhibits reduced binding (e.g. substantially complete loss of binding) to a CD39 polypeptide having a mutation at one, two or three of the residues selected from the group consisting of: R138, M139 and E142 (with reference to SEQ ID NO: 2), compared to a wild-type CD39 polypeptide (a CD39 polypeptide of SEQ ID NO: 2); optionally, the mutant CD39 polypeptide has the mutations: R138A, M139A and E142K. In one optional aspect, the antibody does not have a loss of binding to any of the mutant CD39 polypeptide of Table 1 other than mutant 19.

An anti-CD39 antibody may for example comprise: a HCDR1 comprising an amino acid sequence: DYNMH (SEQ ID NO: 8); a HCDR2 comprising an amino acid sequence: YIVPLNGGSTFNQKFKG (SEQ ID NO: 9); a HCDR3 comprising an amino acid sequence: GGTRFAY (SEQ ID NO: 10); a LCDR1 comprising an amino acid sequence: RASESVDNFGVSFMY (SEQ ID NO: 11); a LCDR2 region comprising an amino acid sequence: GASNQGS (SEQ ID NO: 12) ; and a LCDR3 region of I-394 comprising an amino acid sequence: QQTKEVPYT (SEQ ID NO: 13). CDR positions may be according to Kabat numbering.

An exemplary anti-CD39 VH and VL pair of an antibody that inhibits the enzymatic activity of human sCD39 protein is that of antibody I-394, the amino acid sequence of the heavy chain variable region of which is listed below (SEQ ID NO: 6), and the amino acid sequence of the light chain variable region of which is listed below (SEQ ID NO: 7). The CDRs according to Kabat numbering are underlined in SEQ ID NOS: 6 and 7. Optionally, the VH and VL comprise (e.g., are modified to incorporate) human acceptor frameworks. In one embodiment, an anti-CD39 antibody of the disclosure comprises the VH CDR1, CDR2 and/or CDR3 (e.g., according to Kabat numbering) of the heavy chain variable region having the amino acid sequence of SEQ ID NO: 6. In one embodiment, an anti-CD39 antibody of the disclosure comprise the VL CDR1, CDR2 and/or CDR3 (e.g., according to Kabat numbering) of the light chain variable region having the amino acid sequence of SEQ ID NO: 7.
I-394 VH: 
EVQLQQSGPELVKPGASVKMSCKASGYTFTDYNMHWVKQSHGRTLEWIGYIVPLNGGSTF
NQKFKGRATLTVNTSSRTAYMELRSLTSEDSAAYYCARGGTRFAYWGQGTLVTVSA (SEQ
ID NO: 6).
I-394 VL:

DIVLTQSPASLAVSLGQRATISCRASESVDNFGVSFMYWFQQKPGQPPNLLIYGASNQGSG
VPARFRGSGSGTDFSLNIHPMEADDTAMYFCQQTKEVPYTFGGGTKLEIK (SEQ ID NO:
7).

Another exemplary anti-CD39 VH and VL pair according to the disclosure referred to in the examples is that of antibody I-395, the amino acid sequence of the heavy chain variable region of which is listed below (SEQ ID NO: 14), and the amino acid sequence of the light chain variable region of which is listed below (SEQ ID NO: 15). The CDRs according to Kabat numbering are underlined in SEQ ID NOS: 14 and 15. Optionally, the VH and VL comprise (e.g., are modified to incorporate) human acceptor frameworks. In one embodiment, an anti-CD39 antibody of the disclosure comprises the VH CDR1, CDR2 and/or CDR3 (e.g., according to Kabat numbering) of the heavy chain variable region having the amino acid sequence of SEQ ID NO: 14. In one embodiment, an anti-CD39 antibody of the disclosure comprise the VL CDR1, CDR2 and/or CDR3 (e.g., according to Kabat numbering) of the light chain variable region having the amino acid sequence of SEQ ID NO: 15.
I-395 VH: 
EVQLQQSGPELVKPGASVRMSCKASGYTFTDYNMHWVKKNHGKGLEWIGYINPNNGGTT
YNQKFKGKATLTVNTSSKTAYMELRSLTSEDSAVYYCTRGGTRFASWGQGTLVTVSA
(SEQ ID NO: 14).
I-395 VL:

NIVLTQSPASLAVSLGQRATISCRASESVDNYGISFMYWFQQKPGQPPKLLIYAASTQGSG
VPARFSGSGSGTDFSLNIHPMEEDDTAMYFCQQSKEVPFTFGSGTKLEIK
(SEQ ID NO:15).

Another exemplary anti-CD39 VH and VL pair according to the disclosure referred to in the examples is that of antibody 1-396, the amino acid sequence of the heavy chain variable region of which is listed below (SEQ ID NO: 22), and the amino acid sequence of the light chain variable region of which is listed below (SEQ ID NO: 23). The CDRs according to Kabat numbering are underlined in SEQ ID NOS: 22 and 23. Optionally, the VH and VL comprise (e.g., are modified to incorporate) human acceptor frameworks. In one embodiment, an anti-CD39 antibody of the disclosure comprises the VH CDR1, CDR2 and/or CDR3 (e.g., according to Kabat numbering) of the heavy chain variable region having the amino acid sequence of SEQ ID NO: 22. In one embodiment, an anti-CD39 antibody of the disclosure comprise the VL CDR1, CDR2 and/or CDR3 (e.g., according to Kabat numbering) of the light chain variable region having the amino acid sequence of SEQ ID NO: 23.
I-396 VH: 
EVQLQQSGAELVKPGASVKLSCIVSGFNIKDTYINWVKQRPEQGLEWIGRIDPANGNTKYD
PKFQGKATMTSDTSSNTAYLHLSSLTSDDSAVYYCARWGYDDEEADYFDSWGQGTTLTV
SS
(SEQ ID NO: 22).
I-396 VL:

DIVLTQSPASLAVSLGQRATISCRASESVDNYGISFMNWFQQKPGQPPKLLIYAASNQGSG
VPARFSGSGSGTDFSLNILPMEEVDAAMYFCHQSKEVPWTFGGGTKLEIK
(SEQ ID NO: 23).

Another exemplary anti-CD39 VH and VL pair according to the disclosure referred to in the examples is that of antibody I-399, the amino acid sequence of the heavy chain variable region of which is listed below (SEQ ID NO: 30), and the amino acid sequence of the light chain variable region of which is listed below (SEQ ID NO: 31). The CDRs according to Kabat numbering are underlined in SEQ ID NOS: 30 and 31. Optionally, the VH and VL comprise (e.g., are modified to incorporate) human acceptor frameworks. In one embodiment, an anti-CD39 antibody of the disclosure comprises the VH CDR1, CDR2 and/or CDR3 (e.g., according to Kabat numbering) of the heavy chain variable region having the amino acid sequence of SEQ ID NO: 30. In one embodiment, an anti-CD39 antibody of the disclosure comprise the VL CDR1, CDR2 and/or CDR3 (e.g., according to Kabat numbering) of the light chain variable region having the amino acid sequence of SEQ ID NO: 31.
I-399 VH: 
PVQLQQPGAEVVMPGASVKLSCKASGYTFTSFWMNWMRQRPGQGLEWIGEIDPSDFYTN
SNQRFKGKATLTVDKSSSTAYMQLSSLTSEDSAVYFCARGDFGWYFDVWGTGTSVTVSS
(SEQ ID NO: 30).
I-399 VL:

EIVLTQSPTTMTSSPGEKITFTCSASSSINSNYLHWYQQKPGFSPKLLIYRTSNLASGVPTRF
SGSGSGTSYSLTIGTMEAEDVATYYCQQGSSLPRTFGGGTKLEIK
(SEQ ID NO: 31).

In any aspect, the specified variable region, FR and/or CDR sequences may comprise one or more sequence modifications, e.g., a substitution (1, 2, 3, 4, 5, 6, 7, 8 or more sequence modifications). In one aspect, the substitution is a conservative modification.

In another aspect, the anti-CD39 compound comprises a VH domain having at least about 60%, 70% or 80% sequence identity, optionally at least about 85%, 90%, 95%, 97%, 98% or 99% identity, to the VH domain of an antibody disclosed herein. In another aspect, the anti-CD39 antibody comprises a VL domain having at least about 60%, 70% or 80% sequence identity, optionally at least about 85%, 90%, 95%, 97%, 98% or 99% identity, to the VL domain of an antibody disclosed herein.

### Agents that inhibit CD73

Inhibition or neutralization of the ecto-5' nucleotidase activity of the CD73 enzyme can advantageously involve use of an agent (e.g., a small molecule organic compound, an antibody, a polypeptide fused to an Fc domain, an immunoadhesin, etc.) that binds CD73 and inhibits the ecto-5' nucleotidase activity of the CD73 enzyme.

Each subunit of the CD73 dimer consists of two structural domains: the N-terminal domain (residues 27-317, numbering as referenced in Knapp et al. (2012)) and the C-terminal domain (residues 337-549), with the larger N-terminal domain of CD73 containing the metal ion binding site and having a four-layered a/b-b-b-a structure, including two sandwiched mixed b sheets. The C-terminal domain contains the substrate binding site and dimerization interface, and has a four-layered structure of the composition a/b-b-a-b. The two domains are linked by a single a helix (residues 318-336) comprising a small hinge region, which enables the enzyme to undergo large domain movements and thereby switch between the open and closed conformations. The active site, observed in the closed conformation, is located at the interface between the N- and C-terminal domains and is formed from residues of both domains. See, e.g., Knapp et al. (2012) Structure 20, 2161-2173.

A variety of different CD73 binding agents are known in the art that function to inhibit the ecto-5' nucleotidase activity of CD73 by different mechanisms of action. Small molecule agents such as purines and ADP analogs such as the non-hydrolysable ADP analog APCP (adenosine 5'-[α,β-methylene]diphosphate) act as competitive inhibitors of ADP (e.g. by binding the ADP binding site of CD73). Other agents, including both antibodies and small molecule agents, can act as non-competitive inhibitors. For example, antibodies such as BMS-986179 have been reported that inhibit the enzymatic activity of CD73 by inducing intracellular internalization of CD73 (see, e.g. PCT publication no. WO2016/081748). In other examples, both small molecule and/or antibody agents can act as allosteric inhibitors, for example by binding CD73 at a site that leads to impairment of domain motion needed for enzymatic activity. For example, computation biology permitted the design of small molecules combining rigid scaffolds such as five- or six membered aromatic rings as a tri-branched based molecules that can target the dimerization interface of CD73, thereby non-competitively inhibiting CD73 (see Rahimova et al. (2018) PLOS Computational Biology; https://doi.org/10.1371/journal.pcbi.1005943), and 2-alkoxy-3-(sulfonylarylaminomethylene)-chroman-4-one derivatives have been reported as non-competitive inhibitors (see Al-Rashida and Iqbal (2014) Med Res Rev 34: 703-743) . Antibody MEDI9447 (oleclumab; see also PCT publication no. WO2016/075099 sterically blocks CD73 and prevents CD73 from adopting a catalytically active conformation (see Geoghegan et al. (2016) mAbs 8: 454-467). Antibodies that act as non-competitive inhibitors of cell membrane-bound CD73 (without reliance on internalization) and additionally inhibit the enzymatic activity of soluble CD73 can additionally optionally further inhibit soluble CD73 polypeptide without reliance on internalization have also been reported (see PCT publication no. WO2016/055609). Other agents may include nucleic acid (e.g. RNA) based inhibitors of CD73 expression.

Examples of antibody agents reported to inhibit the enzymatic activity of CD73 are disclosed in PCT publication nos. WO2016/055609; WO2016/131950; WO2017/064043; WO2017/100670; WO2016/075099; and WO2016/081748. Examples of small molecule organic compounds reported to inhibit the enzymatic activity of CD73 are disclosed in PCT publication nos. WO2015/049447 and WO2015/164573 (purine derivatives), WO2018094148, WO2017/120508, WO2017/153952 and WO2017/098421.

Antibodies preferably bind an epitope present on CD73 expressed at the surface of cells, including tumor cells, and inhibit the enzymatic (ecto-5' nucleotidase) activity of the CD73 enzyme (e.g. membrane-bound CD73 protein expressed at the surface of cells). In one aspect, these antibodies can be used as pure CD73 blocking antibodies, e.g., they inhibit the enzymatic activity of membrane-bound CD73 protein expressed at the surface of cells without substantially binding Fcγ receptors and/or without substantially directing ADCC toward a CD73-expressing cell. Optionally, the antibodies retain an Fc domain and retain binding to human FcRn. Optionally the antibodies comprise a modified Fc domain, e.g. to decrease protease sensitivity (e.g. toward proteases such as MMPs in the tumor environment) and/or to decrease binding to human Fcγ receptors (e.g., CD16).

Optionally, the antibodies are administered in an amount effective to inhibit the enzymatic activity of CD73 for a desired period of time, e.g. 1 week, 2 weeks, a month, until the next successive administration of anti-CD73 antibody.

Examples of anti-CD73 antibodies are provided in PCT publication nos. WO2017/064043; WO2017/100670; WO2016/075099; and WO2016/081748.

In one example, the anti-CD73 antibodies used in accordance of the disclosure neutralize the enzymatic activity of CD73 by causing or inducing intracellular internalization of, or more generally down-modulation of, cell surface-expressed CD73. Antibody BMS-986179 (PCT publication no. WO2016/081748 is an example of such antibody; the antibody functions by binding to a conformational epitope on CD73, where the antibody binds to a site that includes amino acid residues in the segments 65-83 and 157-172 of human CD73 (reference to SEQ ID NO: 1).

In one aspect, anti-CD73 antibodies may bind an epitope on CD73 that is present on CD73 not only in the "open" conformation when not bound to substrate but also in the "closed" conformation when bound to a substrate (e.g. a natural substrate such as AMP or an inhibitor or other compound that binds the active site such as an AMP analogue adenosine 5'-(a,β-methylene)diphosphate (APCP)). In one aspect, an anti-CD73 antibody does not compete with a substrate of CD73 for binding to a CD73 polypeptide. Examples of substrates of CD73 include, e.g. a natural substrate such as AMP or an inhibitor or other compound that binds the active site such as an AMP analogue adenosine 5'-(α,β-methylene)diphosphate (APCP).

In one aspect, these antibodies do not inhibit the enzymatic activity of CD73 as a soluble recombinant CD73 protein, e.g. the antibodies are not capable of inhibiting the enzymatic activity of soluble human dimeric CD73 polypeptide when the antibodies are in a setting/configuration where they not capable of forming oligomers, e.g. when they are provided at a substantial molar excess (e.g. at least 10-fold, 20-fold, 100-fold, etc.) to the CD73 polypeptide dimers. For example, antibodies that inhibit CD73 by causing intracellular internalization of CD73 (e.g. BMS-986179) or antibodies designed to inhibit membrane-bound CD73 but not necessarily soluble CD73 (e.g. oleculmab and/or humanized 1E9 antibody) may not capable of inhibiting the enzymatic activity of soluble human dimeric CD73 polypeptide when the antibodies are in a setting/configuration where they not capable of forming oligomers. Because residual CD73 enzymatic activity can result in sufficient adenosine generation to mediate immunosuppressive effects, high levels of antibody-mediated enzyme blockade are advantageous in order to mediate a therapeutic effect. The treatment methods and compositions herein that make use of combinations with antibodies that inhibit sCD39 can be particularly useful to enhance the activity of such antibodies.

In one aspect, the anti-CD73 antibody is MEDI9447 (oleclumab; see PCT publication no. WO2016/075099), or an antibody that shares a common determinant or epitopic site on CD73 therewith. In one aspect, the therapeutic anti-CD73 antibody is BMS-986179 (see PCT publication no. WO2016/081748), or an antibody that shares a common determinant or epitopic site on CD73 therewith. In one aspect, the therapeutic anti-CD73 antibody is humanized 1E9 (see PCT publication no. WO2017/100670), or an antibody that shares a common determinant or epitopic site on CD73 therewith.

In one example, the anti-CD73 antibodies used in accordance of the disclosure do not cause intracellular internalization of, or more generally down-modulation of, cell surface-expressed CD73 and/or do not depend thereupon for their CD73-neutralizing activity. Examples of such antibodies are described in PCT publication nos. WO2016/075099 (e.g. antibody MEDI9447, oleclumab), WO2016/055609 (e.g. antibodies 11E1, 6E1, 3C12 and 8C7) and WO2016/131950. Oleclumab binds to CD73 at residue in amino acid segments 158-171 and 206-211, e.g. residues V170 K206 and N211 (reference to SEQ ID NO: 1). Antibodies 11E1, 6E1, 3C12 and 8C7 lose binding to CD73 mutants having a substitution at residue K136 (with reference to the CD73 polypeptide of SEQ ID NO: 1). Antibodies 11E1, 6E1, 3C12 and 8C7 also lose binding to mutants having substitutions at residues A99, E129, K133, E134 and A135 (with reference to the CD73 polypeptide of SEQ ID NO: 1), as well as to mutants having a substitution at residues K97, E125, Q153 and K330 (with reference to the CD73 polypeptide of SEQ ID NO: 1).

The anti-CD73 antibodies used in accordance of the disclosure can optionally inhibit the enzymatic activity of CD73 as a soluble recombinant CD73 protein (e.g., the antibodies are capable of inhibiting the enzymatic activity of soluble human dimeric CD73 polypeptide when the antibodies are in a setting/configuration where they not capable of forming oligomers, e.g. when they are provided at a substantial molar excess (e.g. at least 10-fold, 20-fold, 100-fold, etc.) to the CD73 polypeptide dimers). Antibodies 11E1, 6E1, 3C12 and 8C7 are examples of such antibodies. Assays using soluble CD73 that can be used to identify such CD73 function blocking antibodies, are provided in PCT publication nos. WO2016/055609 and WO2016/131950. Such antibodies, when used in combinations with antibodies that inhibit sCD39 can provide the highest degree of inhibition of adenosine generation and/or immunosuppression.

Accordingly, an antibody may be an allosteric inhibitor of the CD73 polypeptide, e.g. the antibody binds human CD73 polypeptide expressed at the surface of a cell, including but limited to tumor cells, and inhibits the enzymatic (ecto-5' nucleotidase) activity CD73 polypeptide, without interfering with the ability of a substrate of the CD73 polypeptide to bind the CD73 polypeptide.

Exemplary antibodies are described herein that bind to an epitope on CD73 that is present on the same face when CD73 is present as a CD73 dimer, e.g., potentially permitting an antibody to bind bivalently to one CD73 dimer, notably in "closed" position where the binding sites are spatially further apart. In view of binding to ligand-bound CD73, the antibodies described herein may be useful for binding to CD73 when bound to AMP, e.g., in the tumor environment where upstream ADP and/or AMP are present at significant levels prior to treatment). The tumor microenvironment can be characterized by any appropriate parameter, for example high levels of ADP (e.g. generated by dying cells), taken up by CD39 on stromal and cellular infiltrate (e.g. TReg cells) to yield high levels of AMP, as well as more generally by AMP, adenosine, by presence or levels of CD39 expression or CD39-expressing cells, by presence or levels of CD73 expression or CD73-expressing cells, by presence or levels of adenosine receptor expression or adenosine-receptor expressing cells. Thus, CD73 molecules in the tumor environment may be in the substrate-bound conformation and the ability to bind and inhibit substrate-bound cellular CD73 (e.g. cells expressing CD73 pre-incubated with substrate such as AMP) in addition to non-substrate bound CD73 may provide greater ability to inhibit CD73 in vivo. Optionally, levels of ADP or AMP (and/or ATP or adenosine) can be assessed in the tumor environment prior to treatment. The antibodies may have a particular advantage for treatment in an individual having significant levels (e.g. high levels, compared to a reference) ADP, AMP, ATP or adenosine in the tumor sample.

Exemplary antibodies may bind a human CD73 polypeptide expressed at the surface of cells and that inhibits the enzymatic (ecto-5' nucleotidase) activity of the CD73 polypeptide, wherein the antibody is capable of binding bivalently to a single CD73 polypeptide dimer (a soluble CD73 polypeptide dimer or a CD73 polypeptide dimer expressed by a cell). Optionally, the antibody binds with a first antigen binding domain to a first CD73 polypeptide within the dimer and with a second antigen binding domain to a second CD73 polypeptide.

An exemplary antibody may bind a human CD73 polypeptide expressed at the surface of cells and inhibits the enzymatic (ecto-5' nucleotidase) activity of the CD73 polypeptide, wherein the antibody is capable of binding the CD73 polypeptide in the substrate-bound conformation.

An agent (e.g. a CD73-binding compound, an anti-CD73 antibody) can be assessed and selected for its ability to inhibit the enzymatic activity of CD73, notably to block the 5'-nucleotidase activity of CD73 and to reduce the production of adenosine by a CD73-expressing cell, and in turn restore the activity of and/or relieve the adenosine-mediated inhibition of lymphocytes.

The ability of an antibody to inhibit the enzymatic activity of CD73 can be tested in a cell-free assay using recombinant soluble human CD73 (as dimers) and AMP, where conversion of AMP to adenosine (and/or inhibition thereof) is detected directly (e.g. by measurement of substrates and products, i.e. AMP, adenosine and/or phosphate), or indirectly. In one example, AMP and/or adenosine are detected via HPLC before and after incubation of the test compound with recombinant CD73. Recombinant CD73 is described, e.g., in WO2016/055609 and WO2016/131950.

The inhibitory activity of an antibody can also be assessed in any of a number of other ways. For example, in an indirect assay, a luciferase-based reagent is used (e.g. CellTiter-Glo^{®} system available from Promega), to detect the disappearance of AMP. The luciferase reaction in the assay is inhibited by AMP. Adding the CD73 enzyme to the reaction degrades the AMP, and relieves the inhibition, producing a detectable signal.

The assays using soluble CD73 can advantageously involve testing at conditions where the antibodies are provided at a substantial molar excess (e.g. 10-fold, 20-fold, 50-fold, 100-fold, etc.) to the CD73 polypeptide dimers. When provided in molar excess to the enzyme, the anti-CD73 antibodies will no longer be capable of forming multimeric complexes of antibodies and CD73 dimers; antibodies that retain inhibition of the enzymatic activity of CD73 can then be selected.

The ability of an antibody to inhibit the 5'-ectonucleotidase enzymatic activity of CD73 can alternatively or in addition also be tested in a cellular assay (using cells that express CD73). Advantageously, antibodies can be tested or screened first in the cell-free assay to identify antibodies that block the activity of the enzyme to reduce likelihood of selecting antibodies that inhibit CD73 by causing internalization of CD73, and then tested as purified antibody in cellular assays. Cellular assays can be carried out as shown in WO2016/055609. For example, a CD73-expressing cell line (e.g. MDA-MB-231 cell line) are plated in flat-bottom 96 well plates in presence of anti-CD73 antibodies and incubated. AMP is added to the cells and incubated at 4°C (to avoid CD73 down-modulation). Plates are then centrifuged and supernatant is transferred to flat bottom 96 well culture plate. Free phosphate produced by the hydrolysis of AMP into adenosine is then quantified. A decrease in hydrolysis of AMP into adenosine in the presence of antibody indicate the antibody inhibits cellular CD73.

In one aspect, an antibody preparation causes at least a 50% decrease in the enzymatic activity of a CD73 polypeptide, preferably at least a 60%, 70% or 80% decrease in the enzymatic activity of a CD73 polypeptide (e.g. a soluble homodimeric CD73 polypeptide; CD73 expressed by cells).

The activity of an antibody can also be measured in an indirect assay for its ability to modulate the activity of lymphocytes, for example to relieve the adenosine-mediated inhibition of lymphocyte activity, or to cause the activation of lymphocyte activity. This can be addressed, for example, using a cytokine-release assay. In another example, an antibody can be evaluated in an indirect assay for its ability to modulate the proliferation of lymphocytes.

The antibody can be tested for its ability to internalize or to induce down-modulation of CD73, e.g. whether by internalization or induction of CD73 shedding from the cell surface. Whether an anti-CD73 antibody internalizes upon binding CD73 on a mammalian cell, or whether a CD73 polypeptide undergoes intracellular internalization (e.g. upon being bound by an antibody) can be determined by various assays including those described in WO2016/055609, for example.

For example, antibodies can be selected for the ability to inhibit the enzymatic activity of soluble human dimeric CD73 polypeptide when the antibodies are in a setting/configuration where they not capable of forming oligomers, e.g. when they are provided at a substantial molar excess (e.g. at least 10-fold, 20-fold, 100-fold, etc.) to the CD73 polypeptide dimers. Antibodies that function by causing oligomerization fail to inhibit CD73 when the antibodies provided at a substantial molar excess to the CD73 polypeptide dimers. The antibodies furthermore bind an epitope on CD73 that is maintained when CD73 is expressed at the cell surface. Through use of this assay, antibodies can also be identified that bind bivalently to a single CD73 dimer; such antibodies may have improved CD73-binding and CD73 blocking activity in vitro and vivo in CD73-expressing cells. The antibodies identified by these methods were then tested in cellular enzymatic activity assays using purified antibody, and found to neutralize the enzymatic activity of cellular CD73. Antibodies that inhibit CD73 by inducing internalization or that lose significant binding to cellular CD73 were less potent and were not able to neutralize enzymatic activity, providing at best only partial inhibition of the enzymatic activity of CD73 in cells.

The epitope on CD73 bound by these antibodies is present on CD73 polypeptides as expressed by a range of cells, e.g. cancer cells, CD4 T cells, CD8 T cells, B cells, transfected cells, and binds with high affinity as determined by flow cytometry. For example, an antibody can be characterized by an EC₅₀, as determined by flow cytometry, that is comparable to, or of no more than 2-log, optionally 1-log, greater than that of an anti-CD73 antibody described herein (e.g., antibody 6E1), or of no more than 5 µg/ml, optionally no more than 2 µg/ml, no more than 1 µg/ml, no more than 0.5 µg/ml, no more than 0.1 µg/ml or no more than 0.05 µg/ml, for binding to cells that express at their surface a CD73 polypeptide. In one aspect the cells are cells that are made to express CD73 at their surface. In one aspect the cells are cells that endogenously express CD73 at their surface, e.g. cancer cells, leukemia cells, bladder cancer cells, glioma cells, glioblastoma cells, ovarian cancer cells, melanoma cells, prostate cancer cells, thyroid cancer cells, esophageal cancer cells or breast cancer cells.

In one aspect, the CD73 neutralizing antibodies can be characterized by being capable of causing a decrease in cells' 5'-ectonucleotidase activity of CD73 by at least 60%, 75% or 80%. In one aspect, the CD73-neutralizing antibodies can be characterized by an EC₅₀ for inhibition of 5'-ectonucleotidase activity of CD73 expressed by a cell that is comparable to, or of no more than that of an antibody described herein, of no more than 2-log, optionally 1-log, greater than that of an anti-CD73 antibody described herein (e.g., antibody 6E1), or no more than 1 µg/ml, optionally no more than 0.5 µg/ml, optionally no more than 0.2 µg/ml.

Optionally, inhibition of 5'-ectonucleotidase activity of CD73 expressed by a cell is determined by assessing neutralization of 5' ectonucleotidase activity in MDA-MB-231 cells by quantifying hydrolysis of AMP to adenosine (see, e.g., Example 5 of WO2016/055609).

The epitope on CD73 bound by the neutralizing antibodies disclosed herein does not result in the down-modulation of CD73 expression on cells (and, e.g., does not cause clustering and internalization of the antibody-CD73 complex), including when full length antibodies are used that bind CD73 in bivalent manner. The anti-CD73 antibody thus remains bound, together with CD73, at the cell surface. In view of the broad tissue expression of CD73, antibodies that do not trigger CD73 down-modulation and/or internalization may provide improved pharmacological properties and greater amounts of antibody in the tumor microenvironment.

In one aspect, provided is an isolated antibody that specifically binds human CD73 (e.g. a polypeptide comprising the amino acid sequence of SEQ ID NO: 1) and which neutralizes the 5'-ectonucleotidase activity of a homodimeric human CD73 polypeptide in solution. In one aspect, provided is an antibody that binds and inhibits the enzymatic activity of a soluble human CD73 polypeptide, notably an antibody that neutralizes the CD73-mediated catabolism of AMP to adenosine. In one aspect, the antibody binds CD73 in bivalent manner. In one aspect, the antibody is a non-depleting antibody e.g., an Fc silent antibody. In one aspect, the antibody neutralizes CD73 in solution without reliance on induction of CD73 polypeptide : anti-CD73 antibody oligomers.

In one aspect, an antibody specifically binds human CD73 at the surface of a cell and that is capable of neutralizing the 5'-ectonucleotidase activity of a soluble human CD73 polypeptide. In one aspect, the antibody does not induce the oligomerization of the soluble CD73.

In one aspect, an antibody specifically binds human CD73 at the surface of a cell and is capable of neutralizing the 5'-ectonucleotidase activity of cellular CD73 (CD73 expressed by cells). In one aspect, an antibody specifically binds and neutralizes the 5'-ectonucleotidase activity of a human CD73 at the surface of a cell, and is not internalized into CD73-expressing cells upon binding to CD73. The antibody does not cause multimerization and subsequence internalization of CD73. In one aspect, an antibody binds and is capable of inhibiting the enzymatic activity of a recombinant human CD73 polypeptide in solution, wherein said antibody is not internalized into CD73-expressing cells. In one aspect, the non-internalizing antibody binds CD73 in bivalent manner. In one aspect, the antibody is a non-depleting antibody, e.g., an Fc silent antibody. The antibody is capable of neutralizing the 5'-ectonucleotidase activity of a dimeric human CD73 polypeptide in solution, moreover without reliance on induction of oligomers of CD73 polypeptides anti-CD73 antibodies.

In one aspect, an antibody specifically binds bivalently to human CD73 polypeptides and inhibits the enzymatic activity of cellular human CD73 (and optionally further recombinant soluble human CD73), wherein said antibody is not internalized into CD73-expressing cells. Preferably, the antibody substantially lacks Fcγ receptor binding (e.g. via its Fc domain).

In one aspect, an antibody specifically binds to human CD73 polypeptides and inhibits the enzymatic activity of cellular human CD73 (and optionally further recombinant soluble human CD73), wherein said antibody increases or induces intracellular internalization of CD73 in CD73-expressing cells. Preferably, the antibody substantially lacks Fcγ receptor binding (e.g. via its Fc domain).

In one aspect, an antibody specifically binds human CD73 at the surface of a cell pre-incubated with AMP, and is capable of neutralizing the 5'-ectonucleotidase activity thereof. Optionally, neutralizing the 5'-ectonucleotidase activity is determined by assessing neutralization of 5' ectonucleotidase activity in MDA-MB-231 cells by quantifying hydrolysis of AMP to adenosine (see, e.g., Example 5 of WO2016/055609).

Optionally, an anti-CD73 antibody can bind to a common antigenic determinant present on both soluble CD73 and CD73 expressed at the cell surface.

Optionally, an anti-CD73 antibody binds a common antigenic determinant present on CD73 when it is "open" conformation (when CD73 active site is not occupied by/bound to a substrate, e.g. AMP, APCP) and "closed" CD73 when it is "closed" conformation (when CD73 active site is occupied by/bound to a substrate, e.g. AMP, APCP).

In one aspect, an anti-CD73 antibody binds an antigenic determinant within each CD73 polypeptide chain within a CD73 dimer, e.g., wherein the antigenic determinants are present on a common face of the CD73 dimer.

In one aspect, an anti-CD73 antibody has reduced binding to a CD73 polypeptide having an amino acid substitution at a residue in segment 158-171 and/or at a residue in segment 206-211, e.g. a CD73 polypeptide having an amino acid substitution at any one or more residues V170 K206 and N211 (reference to SEQ ID NO: 1).

In one aspect, an anti-CD73 antibody has reduced binding to a CD73 polypeptide having an amino acid substitution at a residue in segment 65-83 and/or at a residue in segment 157-172 (reference to SEQ ID NO: 1).

In one aspect, an anti-CD73 antibody binds an epitope on CD73 comprising residue K136 (with reference to SEQ ID NO: 1).

In one aspect, an anti-CD73 antibody binds an epitope on CD73 comprising one, two, three or four of the residues selected from the group consisting of K97, E125, Q153 and K330 (with reference to SEQ ID NO: 1).

In one aspect, an anti-CD73 antibody binds that bind an epitope on CD73 comprising one, two, three, four or five of the residues selected from the group consisting of A99, E129, K133, E134, and A135 (with reference to SEQ ID NO: 1).

In one aspect, an anti-CD73 antibody binds at least partly within a domain or segment of amino acid residues on a human CD73 protein (e.g. a CD73 homodimer protein) comprising the amino acid residues K97, A99, E125, E129, K133, E134, A135, K136, Q153 and K330 (with reference to SEQ ID NO: 1). In one aspect, an anti-CD73 antibody binds an epitope on CD73 comprising at least one, two, three, four or five, or more, of the residues selected from the group consisting of K97, A99, E125, E129, K133, E134, A135, K136, Q153 and K330 (with reference to SEQ ID NO: 1).

In one aspect, an anti-CD73 antibody has reduced binding to a CD73 polypeptide having a mutation at a residue K136 (with reference to SEQ ID NO: 1); optionally, the mutant CD73 polypeptide has the mutation: K136A.

In one aspect, an anti-CD73 antibody has reduced binding to a CD73 polypeptide having a mutation at a residue selected from the group consisting of: K97, E125, Q153 and K330 (with reference to SEQ ID NO: 1); optionally, the mutant CD73 polypeptide has the mutations: K97A, E125A, Q153A and/or K330A (e.g., K97A, E125A and K330A; K97A, E125A and/or Q153A).

In one aspect, an anti-CD73 antibody has reduced binding to a CD73 polypeptide having a mutation at a residue selected from the group consisting of: A99, E129, K133, E134, and A135 (with reference to SEQ ID NO: 1); optionally, the mutant CD73 polypeptide has the mutations: A99S, E129A, K133A, E134N, and A135S.

In one aspect an anti-CD73 antibody competes for binding to an epitope on CD73 bound by oleclumab, humanized 1E9, BMS-986179, 11E1, 8C7, 3C12 and/or 6E1, (e.g., that competes for binding to an epitope on a CD73 polypeptide with an antibody having the heavy and light chain CDRs or variable regions of said antibody).

In one aspect of any of the embodiments herein, an antigen-binding compound binds the same epitope and/or competes for binding to an epitope on a CD73 polypeptide with monoclonal antibodies 11E1, 8C7, 3C12 and/or 6E1 (e.g., that competes for binding to a CD73 polypeptide with an antibody having the heavy and light chain CDRs or variable regions of any of 11E1, 8C7, 3C12 or 6E1). In one embodiment, an antigen-binding compound binds the same epitope and/or competes for binding to an epitope on a CD73 polypeptide with an antibody selected from the group consisting of:
(a) an antibody having respectively a VH and VL region of SEQ ID NOS: 3 and 4 (6E1);
(b) an antibody having respectively a VH and VL region of SEQ ID NOS: 40 and 41 (11E1);
(c) an antibody having respectively a VH and VL region of SEQ ID NOS: 42 and 43 (8C7); and
(d) an antibody having respectively a VH and VL region of SEQ ID NOS: 44 and 45 (3C12).

In one aspect, an anti-CD73 antibody binds an epitope comprising one, two or three amino acid residues selected from the group consisting of the amino acid residues on CD73 bound by 11E1, 6E1, 3C12 or 8C7.

In one aspect of any of the aspects herein, the antibody may have a heavy and/or light chain having one, two or three CDRs of the respective heavy and/or light chain of an antibody selected from the group of antibodies consisting of oleclumab, humanized 1E9, BMS-986179, 11E1, 6E1, 3C12 and 8C7.

In any of the aspects herein, the anti-CD73 antibodies can be characterized by binding to human CD73 polypeptides expressed on the surface of a cell (e.g. a tumor cell, a cell made to express CD73, e.g. an MDA-MB-231 tumor cell line, or a recombinant host cell made to express CD73, as shown in WO2016/055609), and optionally further wherein the antibody binds with high affinity as determined by flow cytometry. For example, an antibody can be characterized by an in vitro EC₅₀, as determined by flow cytometry, of no more than 5 µg/ml, optionally no more than 1 µg/ml, no more than 0.5 µg/ml, no more than 0.1 µg/ml or no more than 0.05 µg/ml, for binding to cells that express at their surface a CD73 polypeptide, e.g. tumor cells expressing CD73, cells expressing at their surface a CD73 polypeptide, lymphocytes expressing CD73, etc. Optionally, an antigen-binding compound has an in vitro EC₅₀ of no more than 1 µg/ml, optionally no more than 0.5 µg/ml, no more than 0.1 µg/ml, or no more than 0.05 µg/ml for binding to (i) cells expressing at their surface human CD73 (e.g. a polypeptide having the amino acid sequence of SEQ ID NO: 1) and/or (ii) cells expressing at their surface human non-human primate CD73 (e.g. a cynomolgus monkey CD73).

In one aspect of any of the aspects herein, the anti-CD73 antibody is a tetrameric antibody comprising two heavy and two light chains, the heavy chains comprising Fc regions of human isotype and which substantially lack binding to human Fcγ receptors (e.g. CD16A, CD16B, CD32A, CD32B and/or CD64). In one aspect, anti- CD73 antibody comprises an Fc domain that is modified (compared to a wild-type Fc domain of the same isotype) to reduce binding between the Fc domain and human CD16A, CD16B, CD32A, CD32B and/or CD64 polypeptides. In one embodiment, the antibody comprises an amino acid substitution in a heavy chain constant region at any one, two, three, four, five or more of residues selected from the group consisting of: 220, 226, 229, 233, 234, 235, 236, 237, 238, 243, 264, 268, 297, 298, 299, 309, 310, 318, 320, 322, 327, 330 and 331 (Kabat EU numbering). In one aspect, the antibody has an amino acid substitution in a heavy chain constant region at any three, four, five or more of residues selected from the group consisting of: 234, 235, 237, 322, 330 and 331. In one embodiment, the antibody comprises an Fc domain comprising an amino acid sequence of any of SEQ ID NOS: 59-62.

The amino acid sequence of the heavy and light chain variable regions of antibodies 11E1, 6E1, 3C12 and 8C7 are listed in Table A. In a specific aspect, the disclosure provides an antibody that binds the same or essentially the same epitope or determinant as monoclonal antibodies 11E1, 6E1, 3C12 or 8C7; optionally the antibody comprises the hypervariable region of antibody 11E1, 6E1, 3C12 or 8C7. In any of the aspects herein, antibody 11E1 can be characterized by the amino acid sequences and/or nucleic acid sequences encoding it. In one aspect, the monoclonal antibody comprises the Fab or F(ab')₂ portion of 11E1, 6E1, 3C12 or 8C7. Also provided is a monoclonal antibody that comprises the heavy chain variable region of 11E1, 6E1, 3C12 or 8C7. According to one aspect, the monoclonal antibody comprises the three CDRs (e.g., according to Kabat, Chothia or IGMT numbering) of the heavy chain variable region of 11E1, 6E1, 3C12 or 8C7. Also provided is a monoclonal antibody that further comprises the variable light chain variable region of 11E1, 6E1, 3C12 or 8C7 or one, two or three of the CDRs (e.g., according to Kabat, Chothia or IGMT numbering) of the light chain variable region of 11E1, 6E1, 3C12 or 8C7. Optionally any one or more of said light or heavy chain CDRs may contain one, two, three, four or five or more amino acid modifications (e.g. substitutions, insertions or deletions). Optionally, provided is an antibody where any of the light and/or heavy chain variable regions comprising part or all of an antigen binding region of antibody 11E1, 6E1, 3C12 or 8C7 are fused to an immunoglobulin constant region of the human IgG type, optionally a human constant region, optionally a human IgG1, IgG2, IgG3 or IgG4 isotype. In one aspect, a human constant region optionally further comprising an amino acid substitution to reduce effector function (binding to human Fcγ receptors). In one aspect, a human constant region (optionally a hinge region) optionally further comprising an amino acid substitution to increase or induce intracellular internalization of CD73.

An anti-CD73 antibody may for example comprise: a HCDR1 of 6E1 comprising an amino acid sequence: SYNMY (SEQ ID NO: 46), or a sequence of at least 4 contiguous amino acids thereof, optionally wherein one or more of these amino acids may be substituted by a different amino acid; a HCDR2 of 6E1 comprising an amino acid sequence: YIDPYNGGSSYNQKFKG (SEQ ID NO: 47), or a sequence of at least 4, 5, 6, 7, 8, 9 or 10 contiguous amino acids thereof, optionally wherein one or more of these amino acids may be substituted by a different amino acid; a HCDR3 of 6E1 comprising an amino acid sequence: GYNNYKAWFAY (SEQ ID NO: 48), or a sequence of at least 4, 5, 6, 7, 8, 9 or 10 contiguous amino acids thereof, optionally wherein one or more of these amino acids may be substituted by a different amino acid; a LCDR1 of 6E1 comprising an amino acid sequence: KASQSVTNDVA (SEQ ID NO: 49), or a sequence of at least 4, 5, 6, 7, 8, 9 or 10 contiguous amino acids thereof, optionally wherein one or more of these amino acids may be substituted by a different amino acid; a LCDR2 of 6E1 comprising an amino acid sequence: YASNRYT (SEQ ID NO: 50) or a sequence of at least 4, 5 or 6 contiguous amino acids thereof, optionally wherein one or more of these amino acids may be substituted by a different amino acid; and/or a LCDR3 of 6E1 comprising an amino acid sequence: QQDYSSLT (SEQ ID NO: 51), or a sequence of at least 4, 5, 6, 7 or 8 contiguous amino acids thereof, optionally wherein one or more of these amino acids may be deleted or substituted by a different amino acid. CDR positions may be according to Kabat numbering.

In another aspect of any of the aspects herein, any of the CDRs 1, 2 and/or 3 of the heavy and light chains may be characterized by a sequence of at least 4, 5, 6, 7, 8, 9 or 10 contiguous amino acids thereof, and/or as having an amino acid sequence that shares at least 50%, 60%, 70%, 80%, 85%, 90% or 95% sequence identity with the particular CDR or set of CDRs listed in the corresponding SEQ ID NO.

In any of the antibodies, e.g., 11E1, 8C7, 3C12 or 6E1, the specified variable region and CDR sequences may comprise sequence modifications, e.g. a substitution (1, 2, 3, 4, 5, 6, 7, 8 or more sequence modifications). In one embodiment, a CDRs 1, 2 and/or 3 of the heavy and light chains comprises one, two, three or more amino acid substitutions, where the residue substituted is a residue present in a sequence of human origin. In one embodiment the substitution is a conservative modification. A conservative sequence modification refers to an amino acid modification that does not significantly affect or alter the binding characteristics of the antibody containing the amino acid sequence. Such conservative modifications include amino acid substitutions, additions and deletions. Modifications can be introduced into an antibody by standard techniques known in the art, such as site-directed mutagenesis and PCR-mediated mutagenesis. Conservative amino acid substitutions are typically those in which an amino acid residue is replaced with an amino acid residue having a side chain with similar physicochemical properties. Specified variable region and CDR sequences may comprise one, two, three, four or more amino acid insertions, deletions or substitutions. Where substitutions are made, preferred substitutions will be conservative modifications. Families of amino acid residues having similar side chains have been defined in the art. These families include amino acids with basic side chains (*e.g.*, lysine, arginine, histidine), acidic side chains (*e.g.*, aspartic acid, glutamic acid), uncharged polar side chains (*e.g*. glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (*e.g*. threonine, valine, isoleucine) and aromatic side chains (*e.g*., tyrosine, phenylalanine, tryptophan, histidine). Thus, one or more amino acid residues within the CDR regions of an antibody can be replaced with other amino acid residues from the same side chain family and the altered antibody can be tested for retained function (*i.e.*, the properties set forth herein) using the assays described herein.

The sequences of the variable regions of the antibodies are listed in Table A below (if leader sequences are present any antibody chain can be specified to start at the amino acid position immediately following the end of the leader sequence). In any embodiment herein, a VL or VH sequence can be specified or numbered so as to contain or lack a signal peptide or any part thereof. The HCDR1, 2, 3 and LCDR1, 2, 3 of an antibody having the VH and VL sequences indicated in Table A can optionally be specified as all (or each, independently) being those of the Kabat numbering system, those of the Chothia numbering system, those of the IMGT numbering system, or any other suitable numbering system.

**Table A**

| | **SEQ ID NO:** | **Amino Acid Sequence of Anti-CD73 antibodies** |
|---|---|---|
| 6E1 VH | 3 | EFQLQQSGPELVKPGASVKVSCKASGYAFTSYNMYWVKQSHGKRLEWIG YIDPYNGGSSYNQKFKGKATLTVDKSSSTAYMHLNNLTSEDSAVYYCAR GYNNYKAWFAYWGQGTLVTVSA |
| 6E1 VL | 4 | SIVMTQTPKFLLVSAGDRVTITCKASQSVTNDVAWYQQKPGQSPKLLIY YASNRYTGVPDRFTGSGYGTDFTFTISTMQAEDLAVYFCQQDYSSLTFG AGTKLELK |
| 11E1 VH | 40 | EIQLQQSGPELVKPGASVKVSCKASGYAFTSYNMYWVKQSHGKSLEWIG YIDPYNGGTSYNQKFKGKATLTVDKSSSTAYMHLNSLTSEDSAVYYCAR GYGNYKAWFAYWGQGTLVTVSA |
| 11E1 VL | 41 | DAVMTQTPKFLLVSAGDRVTITCKASQSVTNDVAWYQQKPGQSPKLLIY YASNRYTGVPDRFTGSGYGTDFTFTISTVQAEDLAVYFCQQDYSSLTFG AGTKLELK |
| 8C7 VH | 42 | EVQLQQSGPELVKPGASVKVSCKASGYAFASYNMNWVKQSHGKSLDWIG YIDPYNGGSSYNLTFKGKATLTVDKSSTTAYMHLNSLTSEDSAVYYCAR GYGNYKAWFAYWGQGTLVTVSAASTKGP |
| 8C7 VL | 43 | SIVMTPTPKFLLVSAGDRVTITCKASQSVSNDVAWYQQKPGQSPKLLIY YASTRYTGVPDRFTGSGYGTDFTFTISTVQAEDLAVYFCQQDYSSLTFG AGTKLELKRTVAAP |
| 3C12 VH | 44 | QIQLQQSGPELVKPGASVKVSCKASGYAFASYNMNWVKQSHGKSLDWIG YIDPYNGGSSYNLTFKGKATLTVDKSSTTAYMHLNSLTSEDSAVYYCAR GYGNYKAWFAYWGQGTLVTVSAASTKGP |
| 3C12 VL | 45 | DVVMTQTPKFLLVSAGDRVTITCKASQSVSNDVAWYQQKPGQSPKLLIY YASTRYTGVPDRFTGSGYGTDFTFTISTVQAEDLAVYFCQQDYSSLTFG AGTKLELKRTVAAP |

In one aspect, the antibodies are to be administered to an individual having a cancer in an amount and frequency sufficient to inhibit the activity of CD73 in the tumor microenvironment. In one aspect, the antibodies are to be administered in an amount and frequency sufficient to decrease the generation and/or concentration of adenosine in the tumor microenvironment. In one aspect, the antibodies are to be administered in an amount and frequency sufficient to increase the generation and/or concentration of ATP in the tumor microenvironment. In one aspect, the antibodies are to be administered in an amount and frequency sufficient to neutralize the activity of CD73 expressed by tumor cells. In one aspect, the antibodies are to be administered in an amount and frequency sufficient to neutralize the activity of CD73 expressed by CD4 T cells, CD8 T cells and/or B cells.

### Production of antibodies

The anti-CD73 and anti-CD39 antibodies may be produced by any of a variety of techniques known in the art. Typically, they are produced by immunization of a non-human animal, for example a mouse, with an immunogen comprising a CD73 or CD39 polypeptide, respectively, or by screening a library of candidate binding domains with a CD73 or CD39 polypeptide. The CD39 or CD73 polypeptide may comprise the full length sequence of a human CD39 or CD73 polypeptide, respectively, or a fragment or derivative thereof, typically an immunogenic fragment, i.e., a portion of the polypeptide comprising an epitope exposed on the surface of cells expressing a CD39 or CD73 polypeptide. Such fragments typically contain at least about 7 consecutive amino acids of the mature polypeptide sequence, even more preferably at least about 10 consecutive amino acids thereof. Fragments typically are essentially derived from the extra-cellular domain of the receptor. In one embodiment, the immunogen comprises a wild-type human CD39 or CD73 polypeptide in a lipid membrane, typically at the surface of a cell. In a specific embodiment, the immunogen comprises intact cells, particularly intact human cells, optionally treated or lysed. In another embodiment, the polypeptide is a recombinant CD39 or CD73 polypeptide.

The step of immunizing a non-human mammal with an antigen may be carried out in any manner well known in the art for stimulating the production of antibodies in a mouse (see, for example, E. Harlow and D. Lane, Antibodies: A Laboratory Manual., Cold Spring Harbor Laboratory Press, Cold Spring Harbor, NY (1988)). The immunogen is suspended or dissolved in a buffer, optionally with an adjuvant, such as complete or incomplete Freund's adjuvant. Methods for determining the amount of immunogen, types of buffers and amounts of adjuvant are well known to those of skill in the art and are not limiting in any way. These parameters may be different for different immunogens, but are easily elucidated.

Similarly, the location and frequency of immunization sufficient to stimulate the production of antibodies is also well known in the art. In a typical immunization protocol, the non-human animals are injected intraperitoneally with antigen on day 1 and again about a week later. This is followed by recall injections of the antigen around day 20, optionally with an adjuvant such as incomplete Freund's adjuvant. The recall injections are performed intravenously and may be repeated for several consecutive days. This is followed by a booster injection at day 40, either intravenously or intraperitoneally, typically without adjuvant. This protocol results in the production of antigen-specific antibody-producing B cells after about 40 days. Other protocols may also be used as long as they result in the production of B cells expressing an antibody directed to the antigen used in immunization.

For monoclonal antibodies, splenocytes are isolated from the immunized non-human mammal and the subsequent fusion of those splenocytes with an immortalized cell in order to form an antibody-producing hybridoma. The isolation of splenocytes from a non-human mammal is well-known in the art and typically involves removing the spleen from an anesthetized non-human mammal, cutting it into small pieces and squeezing the splenocytes from the splenic capsule through a nylon mesh of a cell strainer into an appropriate buffer so as to produce a single cell suspension. The cells are washed, centrifuged and resuspended in a buffer that lyses any red blood cells. The solution is again centrifuged and remaining lymphocytes in the pellet are finally resuspended in fresh buffer.

Once isolated and present in single cell suspension, the lymphocytes can be fused to an immortal cell line. This is typically a mouse myeloma cell line, although many other immortal cell lines useful for creating hybridomas are known in the art. Murine myeloma lines include, but are not limited to, those derived from MOPC-21 and MPC-11 mouse tumors available from the Salk Institute Cell Distribution Center, San Diego, U. S. A., X63 Ag8653 and SP-2 cells available from the American Type Culture Collection, Rockville, Maryland U. S. A. The fusion is effected using polyethylene glycol or the like. The resulting hybridomas are then grown in selective media that contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Hybridomas are typically grown on a feeder layer of macrophages. The macrophages are preferably from littermates of the non-human mammal used to isolate splenocytes and are typically primed with incomplete Freund's adjuvant or the like several days before plating the hybridomas. Fusion methods are described in Goding, "Monoclonal Antibodies: Principles and Practice," pp. 59-103 (Academic Press, 1986).

The cells are allowed to grow in the selection media for sufficient time for colony formation and antibody production. This is usually between about 7 and about 14 days.

The hybridoma colonies are then assayed for the production of antibodies that specifically bind to CD39 or CD73 polypeptide gene products. The assay is typically a colorimetric ELISA-type assay, although any assay may be employed that can be adapted to the wells that the hybridomas are grown in. Other assays include radioimmunoassays or fluorescence activated cell sorting. The wells positive for the desired antibody production are examined to determine if one or more distinct colonies are present. If more than one colony is present, the cells may be re-cloned and grown to ensure that only a single cell has given rise to the colony producing the desired antibody. Typically, the antibodies will also be tested for the ability to bind to CD39 or CD73 polypeptides, e.g., CD39- or CD73-expressing cells.

Hybridomas that are confirmed to produce a monoclonal antibody can be grown up in larger amounts in an appropriate medium, such as DMEM or RPMI-1640. Alternatively, the hybridoma cells can be grown in vivo as ascites tumors in an animal.

After sufficient growth to produce the desired monoclonal antibody, the growth media containing monoclonal antibody (or the ascites fluid) is separated away from the cells and the monoclonal antibody present therein is purified. Purification is typically achieved by gel electrophoresis, dialysis, chromatography using protein A or protein G-Sepharose, or an anti-mouse Ig linked to a solid support such as agarose or Sepharose beads (all described, for example, in the Antibody Purification Handbook, Biosciences, publication No. 18-1037-46, Edition AC). The bound antibody is typically eluted from protein A/protein G columns by using low pH buffers (glycine or acetate buffers of pH 3.0 or less) with immediate neutralization of antibody-containing fractions. These fractions are pooled, dialyzed, and concentrated as needed.

Positive wells with a single apparent colony are typically re-cloned and re-assayed to insure only one monoclonal antibody is being detected and produced.

Antibodies may also be produced by selection of combinatorial libraries of immunoglobulins, as disclosed for instance in (Ward et al. Nature, 341 (1989) p. 544).

The identification of one or more antibodies that bind(s) to the antigen of interest, i.e. CD39 or CD73, particularly substantially or essentially the same epitope as monoclonal antibody 11E1, 8C7 or 6E1 with respect to CD73, or particularly substantially or essentially the same epitope as monoclonal antibody I-394, I-395, I-396 or I-399 with respect to CD39, can be readily determined using any one of a variety of immunological screening assays in which antibody competition can be assessed. Many such assays are routinely practiced and are well known in the art (see, e. g., U. S. Pat. No. 5,660,827, issued Aug. 26, 1997). It will be understood that actually determining the epitope to which an antibody described herein binds is not in any way required to identify an antibody that binds to the same or substantially the same epitope as the monoclonal antibody described herein.

For example, where the test antibodies to be examined are obtained from different source animals, or are even of a different Ig isotype, a simple competition assay may be employed in which the control (I-394, I-395, I-396 or I-399, for example) and test antibodies are admixed (or pre-adsorbed) and applied to a sample containing CD39 or CD73 polypeptides. Protocols based upon western blotting and the use of BIACORE analysis are suitable for use in such competition studies.

In certain aspects, one pre-mixes the control antibodies (e.g., 1-394, 1-395, I-396 or I-399 for example) with varying amounts of the test antibodies (e.g., about 1:10 or about 1:100) for a period of time prior to applying to the CD39 antigen sample. In other embodiments, the control and varying amounts of test antibodies can simply be admixed during exposure to the CD39 antigen sample. As long as one can distinguish bound from free antibodies (e. g., by using separation or washing techniques to eliminate unbound antibodies) and I-394, I-395, I-396 or I-399 from the test antibodies (e. g., by using species-specific or isotype-specific secondary antibodies or by specifically labeling I-394, I-395, I-396 or I-399 with a detectable label) one can determine if the test antibodies reduce the binding of I-394, I-395, I-396 or I-399 to the antigens, indicating that the test antibody compete for binding to the same site on CD39 as I-394, I-395, I-396 or I-399. The binding of the (labelled) control antibodies in the absence of a completely irrelevant antibody can serve as the control high value. The control low value can be obtained by incubating the labelled (I-394, I-395, I-396 or I-399) antibodies with unlabelled antibodies of exactly the same type (I-394, I-395, I-396 or I-399), where competition would occur and reduce binding of the labelled antibodies. In a test assay, a significant reduction in labelled antibody reactivity in the presence of a test antibody is indicative of a test antibody that recognizes substantially the same epitope, i.e., one that "cross-reacts" or competes with the labelled (I-394, I-395, I-396 or I-399) antibody. Any test antibody that reduces the binding of I-394, I-395, I-396 or I-399 to CD39 antigens by at least about 50%, such as at least about 60%, or more preferably at least about 80% or 90% (e. g., about 65-100%), at any ratio of I-394, I-395, I-396 or I-399:test antibody between about 1:10 and about 1:100 is considered to be an antibody that competes for binding to substantially the same epitope or determinant as I-394, I-395, I-396 or I-399. Preferably, such test antibody will reduce the binding of I-394, I-395, I-396 or I-399 to the CD39 antigen by at least about 90% (e.g., about 95%).

Competition can also be assessed by, for example, a flow cytometry test. In such a test, cells bearing a given CD39 polypeptide can be incubated first with I-394, I-395, I-396 or I-399 (or a CD73 polypeptide is incubated with 11E1, 8C7, 3C12 or 6E1), for example, and then with the test antibody labelled with a fluorochrome or biotin. The antibody is said to compete with I-394, I-395, I-396 or I-399 if the binding obtained upon preincubation with a saturating amount of I-394, I-395, I-396 or I-399 is about 80%, preferably about 50%, about 40% or less (e.g., about 30%, 20% or 10%) of the binding (as measured by mean of fluorescence) obtained by the antibody without preincubation with I-394, I-395, I-396 or I-399. Alternatively, an antibody is said to compete with I-394, I-395, I-396 or I-399 if the binding obtained with a labelled 1-394, 1-395, I-396 or I-399 antibody (by a fluorochrome or biotin) on cells preincubated with a saturating amount of test antibody is about 80%, preferably about 50%, about 40%, or less (e. g., about 30%, 20% or 10%) of the binding obtained without preincubation with the test antibody.

A simple competition assay in which a test antibody is pre-adsorbed and applied at saturating concentration to a surface onto which a CD39 antigen (or CD73 for anti-CD73 antibodies) is immobilized may also be employed. The surface in the simple competition assay is preferably a BIACORE chip (or other media suitable for surface plasmon resonance analysis). The control antibody (e.g., I-394, I-395, I-396 or I-399) is then brought into contact with the surface at a CD39-saturating concentration and the CD39 and surface binding of the control antibody is measured. This binding of the control antibody is compared with the binding of the control antibody to the CD39-containing surface in the absence of test antibody. In a test assay, a significant reduction in binding of the CD39-containing surface by the control antibody in the presence of a test antibody can be indicative that the test antibody competes for binding to the same determinant or epitope as the control antibody such that the test antibody "cross-reacts" with the control antibody. Any test antibody that reduces the binding of control (such as I-394, I-395, I-396 or I-399) antibody to a CD39 antigen by at least about 30% or more, preferably about 40%, can be considered to be an antibody that binds to substantially the same epitope or determinant as a control (e.g., I-394, I-395, I-396 or I-399). Preferably, such a test antibody will reduce the binding of the control antibody (e.g., I-394, I-395, I-396 or I-399) to the CD39 antigen by at least about 50% (e. g., at least about 60%, at least about 70%, or more). It will be appreciated that the order of control and test antibodies can be reversed: that is, the control antibody can be first bound to the surface and the test antibody is brought into contact with the surface thereafter in a competition assay. Preferably, the antibody having higher affinity for the CD73 antigen is bound to the surface first, as it will be expected that the decrease in binding seen for the second antibody (assuming the antibodies are cross-reacting) will be of greater magnitude. Further examples of such assays are provided in, e.g., Saunal (1995) J. Immunol. Methods 183: 33-41.

In one aspect, the antibodies are validated in an immunoassay to test their ability to bind to, respectively, CD39 or CD73-expressing cells, respectively. For example, a blood sample or tumor biopsy is performed and tumor cells or tumor infiltrating cells are collected. The ability of a given antibody to bind to the cells is then assessed using standard methods well known to those in the art. Antibodies may bind for example to a substantial proportion (e.g., 20%, 30%, 40%, 50%, 60%, 70%, 80% or more) of cells known to express respectively CD39 or CD73, e.g. tumor cells, from a significant percentage of individuals or patients (e.g., 10%, 20%, 30%, 40%, 50% or more). Antibodies can be used for diagnostic purposes to determine the presence or level of malignant cells in a patient, for example as a biomarker to assess whether a patient is suitable for treatment with an anti-CD73 agent, or for use in the herein-described therapeutic methods. To assess the binding of the antibodies to the cells, the antibodies can either be directly or indirectly labelled. When indirectly labelled, a secondary, labelled antibody is typically added.

Determination of whether an antibody binds within an epitope region can be carried out in ways known to the person skilled in the art. As one example of such mapping/characterization methods, an epitope region for an anti-CD73 or anti-CD39 antibody may be determined by epitope "foot-printing" using chemical modification of the exposed amines/carboxyls in the respective CD73 or CD39 protein. One specific example of such a foot-printing technique is the use of HXMS (hydrogen-deuterium exchange detected by mass spectrometry) wherein a hydrogen/deuterium exchange of receptor and ligand protein amide protons, binding, and back exchange occurs, wherein the backbone amide groups participating in protein binding are protected from back exchange and therefore will remain deuterated. Relevant regions can be identified at this point by peptic proteolysis, fast microbore high-performance liquid chromatography separation, and/or electrospray ionization mass spectrometry. See, e. g., Ehring H, Analytical Biochemistry, Vol. 267 (2) pp. 252-259 (1999) Engen, J. R. and Smith, D. L. (2001) Anal. Chem. 73, 256A-265A. Another example of a suitable epitope identification technique is nuclear magnetic resonance epitope mapping (NMR), where typically the position of the signals in two-dimensional NMR spectra of the free antigen and the antigen complexed with the antigen binding peptide, such as an antibody, are compared. The antigen typically is selectively isotopically labeled with 15N so that only signals corresponding to the antigen and no signals from the antigen binding peptide are seen in the NMR-spectrum. Antigen signals originating from amino acids involved in the interaction with the antigen binding peptide typically will shift position in the spectrum of the complex compared to the spectrum of the free antigen, and the amino acids involved in the binding can be identified that way. See, e. g., Ernst Schering Res Found Workshop. 2004; (44): 149-67; Huang et al., Journal of Molecular Biology, Vol. 281 (1) pp. 61-67 (1998); and Saito and Patterson, Methods. 1996 Jun; 9 (3): 516-24.

Epitope mapping/characterization also can be performed using mass spectrometry methods. See, e.g., Downard, J Mass Spectrom. 2000 Apr; 35 (4): 493-503 and Kiselar and Downard, Anal Chem. 1999 May 1; 71 (9): 1792-1801. Protease digestion techniques also can be useful in the context of epitope mapping and identification. Antigenic determinant-relevant regions/sequences can be determined by protease digestion, e.g. by using trypsin in a ratio of about 1:50 to CD73 or o/n digestion at and pH 7-8, followed by mass spectrometry (MS) analysis for peptide identification. The peptides protected from trypsin cleavage by the anti-CD73 or anti-CD39 binder can subsequently be identified by comparison of samples subjected to trypsin digestion and samples incubated with antibody and then subjected to digestion by e.g. trypsin (thereby revealing a footprint for the binder). Other enzymes like chymotrypsin, pepsin, etc., also or alternatively can be used in similar epitope characterization methods. Moreover, enzymatic digestion can provide a quick method for analyzing whether a potential antigenic determinant sequence is within a region of the CD73 or CD39 polypeptide that is not surface exposed and, accordingly, most likely not relevant in terms of immunogenicity/antigenicity.

Site-directed mutagenesis is another technique useful for elucidation of a binding epitope. For example, in "alanine-scanning", each residue within a protein segment is replaced with an alanine residue, and the consequences for binding affinity measured. If the mutation leads to a significant reduction in binding affinity, it is most likely involved in binding. Monoclonal antibodies specific for structural epitopes (i.e., antibodies which do not bind the unfolded protein) can be used to verify that the alanine-replacement does not influence over-all fold of the protein. See, e.g., Clackson and Wells, Science 1995; 267:383-386; and Wells, Proc Natl Acad Sci USA 1996; 93:1-6.

Electron microscopy can also be used for epitope "foot-printing". For example, Wang et al., Nature 1992; 355:275-278 used coordinated application of cryoelectron micros-copy, three-dimensional image reconstruction, and X-ray crystallography to determine the physical footprint of a Fab-fragment on the capsid surface of native cowpea mosaic virus.

Other forms of "label-free" assay for epitope evaluation include surface plasmon resonance (SPR, BIACORE) and reflectometric interference spectroscopy (RifS). See, e.g., Fägerstam et al., Journal Of Molecular Recognition 1990;3:208-14; Nice et al., J. Chromatogr. 1993; 646:159-168; Leipert et al., Angew. Chem. Int. Ed. 1998; 37:3308-3311; Kröger et al., Biosensors and Bioelectronics 2002; 17:937-944.

It should also be noted that an antibody binding the same or substantially the same epitope as an antibody can be identified in one or more of the exemplary competition assays described herein.

Typically, an anti-CD73 or anti-CD39 antibody provided herein has an affinity for a respective CD73 (e.g. as a CD73 homodimer) or CD39 polypeptide in the range of about 10⁴ to about 10¹¹ M⁻¹ (e.g., about 10⁸ to about 10¹⁰ M⁻¹). For example, in a particular aspect the anti-CD73 or anti-CD39 antibody that have an average disassociation constant (K_{D}) of less than 1 × 10⁻⁹ M with respect to CD73 or CD39, respectively, as determined by, e.g., surface plasmon resonance (SPR) screening (such as by analysis with a BIAcore^{™} SPR analytical device). In a more particular exemplary aspect, the anti-CD73 or anti-CD39 antibodies that have a KD of about 1 × 10⁻⁸ M to about 1 × 10⁻¹⁰ M, or about 1 × 10⁻⁹ M to about 1 × 10⁻¹¹ M, for CD73 or CD39, respectively. In one embodiment, binding is monovalent binding. In one embodiment, binding is bivalent binding.

Antibodies can be characterized for example by a mean KD of no more than about (i.e. better affinity than) 100, 60, 10, 5, or 1 nanomolar, preferably sub-nanomolar or optionally no more than about 500, 200, 100 or 10 picomolar. KD can be determined for example for example by immobilizing recombinantly produced human CD73 or CD39 proteins on a chip surface, followed by application of the antibody to be tested in solution. In one embodiment, the method further comprises a step of selecting antibodies from (b) that are capable of competing for binding to CD73 with antibody 11E1, 8C7, 3C12 or 6E1, or that are capable of competing for binding to CD39 with antibody I-394, I-395, I-396 or I-399.

In one aspect of any of the embodiments, the antibodies prepared according to the present methods are monoclonal antibodies. In another aspect, the non-human animal used to produce antibodies according to the methods herein is a mammal, such as a rodent, bovine, porcine, fowl, camelid, horse, rabbit, goat, or sheep.

DNA encoding an antibody that binds an epitope present on a CD73 or CD39 polypeptide is isolated from a hybridoma and placed in an appropriate expression vector for transfection into an appropriate host. The host is then used for the recombinant production of the antibody, or variants thereof, such as a humanized version of that monoclonal antibody, active fragments of the antibody, chimeric antibodies comprising the antigen recognition portion of the antibody, or versions comprising a detectable moiety.

DNA encoding the monoclonal antibodies of the disclosure, e.g., antibody I-394, I-395, I-396 or I-399, 11E1, 8C7, 3C12 or 6E1, can be readily isolated and sequenced using conventional procedures (e. g., by using oligonucleotide probes that are capable of binding specifically to genes encoding the heavy and light chains of murine antibodies). Once isolated, the DNA can be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. As described elsewhere in the present specification, such DNA sequences can be modified for any of a large number of purposes, e.g., for humanizing antibodies, producing fragments or derivatives, or for modifying the sequence of the antibody, e.g., in the antigen binding site in order to optimize the binding specificity of the antibody. Recombinant expression in bacteria of DNA encoding the antibody is well known in the art (see, for example, Skerra et al., Curr. Opinion in Immunol., 5, pp. 256 (1993); and Pluckthun, Immunol. 130, p. 151 (1992).

Fragments and derivatives of antibodies (which are encompassed by the term "antibody" or "antibodies" as used in this application, unless otherwise stated or clearly contradicted by context) can be produced by techniques that are known in the art. "Fragments" comprise a portion of the intact antibody, generally the antigen binding site or variable region. Examples of antibody fragments include Fab, Fab', Fab'-SH, F (ab') 2, and Fv fragments; diabodies; any antibody fragment that is a polypeptide having a primary structure consisting of one uninterrupted sequence of contiguous amino acid residues (referred to herein as a "single-chain antibody fragment" or "single chain polypeptide"), including without limitation (1) single-chain Fv molecules (2) single chain polypeptides containing only one light chain variable domain, or a fragment thereof that contains the three CDRs of the light chain variable domain, without an associated heavy chain moiety and (3) single chain polypeptides containing only one heavy chain variable region, or a fragment thereof containing the three CDRs of the heavy chain variable region, without an associated light chain moiety; and multispecific (e.g. bispecific) antibodies formed from antibody fragments. Included, *inter alia*, are a nanobody, domain antibody, single domain antibody or a "dAb".

In one aspect, the agent is an antibody selected from a fully human antibody, a humanized antibody, and a chimeric antibody.

In one aspect, the agent is a fragment of an antibody comprising a constant domain selected from IgG1, IgG2, IgG3 and IgG4. In one aspect, the agent is an antibody fragment selected from a Fab fragment, a Fab' fragment, a Fab'-SH fragment, a F(ab)2 fragment, a F(ab')2 fragment, an Fv fragment, a Heavy chain Ig (a llama or camel Ig), a V_{HH} fragment, a single domain FV, and a single-chain antibody fragment. In one aspect, the agent is a synthetic or semisynthetic antibody-derived molecule selected from a scFV, a dsFV, a minibody, a diabody, a triabody, a kappa body, an IgNAR; and a multispecific antibody. In one aspect, the antibody is in at least partially purified form. In one aspect, the antibody is in essentially isolated form.

An anti-CD39 or anti-CD73 agent such as an antibody can be incorporated in a pharmaceutical formulation comprising in a concentration from 1 mg/ml to 500 mg/ml, wherein said formulation has a pH from 2.0 to 10.0. The formulation may further comprise a buffer system, preservative(s), tonicity agent(s), chelating agent(s), stabilizers and surfactants. In one embodiment, the pharmaceutical formulation is an aqueous formulation, i.e., formulation comprising water. Such formulation is typically a solution or a suspension. In a further aspect, the pharmaceutical formulation is an aqueous solution. The term "aqueous formulation" is defined as a formulation comprising at least 50 %w/w water. Likewise, the term "aqueous solution" is defined as a solution comprising at least 50 %w/w water, and the term "aqueous suspension" is defined as a suspension comprising at least 50 %w/w water.

In another aspect, the pharmaceutical formulation is a freeze-dried formulation, whereto the physician or the patient adds solvents and/or diluents prior to use.

In another aspect, the pharmaceutical formulation is a dried formulation (e.g., freeze-dried or spray-dried) ready for use without any prior dissolution.

In a further aspect, the pharmaceutical formulation comprises an aqueous solution of such an antibody, and a buffer, wherein the antibody is present in a concentration from 1 mg/ml or above, and wherein said formulation has a pH from about 2.0 to about 10.0.

In a another aspect, the pH of the formulation is in the range selected from the list consisting of from about 2.0 to about 10.0, about 3.0 to about 9.0, about 4.0 to about 8.5, about 5.0 to about 8.0, and about 5.5 to about 7.5.

In a further aspect, the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative embodiment of the invention.

In a further aspect, the formulation further comprises a pharmaceutically acceptable preservative. In a further embodiment, the formulation further comprises an isotonic agent. In a further embodiment, the formulation also comprises a chelating agent. In a further embodiment of the invention the formulation further comprises a stabilizer. In a further embodiment, the formulation further comprises a surfactant. For convenience reference is made to Remington: The Science and Practice of Pharmacy, 19th edition, 1995.

It is possible that other ingredients may be present in the peptide pharmaceutical formulation of the present invention. Such additional ingredients may include wetting agents, emulsifiers, antioxidants, bulking agents, tonicity modifiers, chelating agents, metal ions, oleaginous vehicles, proteins (e.g., human serum albumin, gelatine or proteins) and a zwitterion (e.g., an amino acid such as betaine, taurine, arginine, glycine, lysine and histidine). Such additional ingredients, of course, should not adversely affect the overall stability of the pharmaceutical formulation of the present invention.

Administration of pharmaceutical compositions according to the invention may be through several routes of administration, for example, intravenous. Suitable antibody formulations can also be determined by examining experiences with other already developed therapeutic monoclonal antibodies. Several monoclonal antibodies have been shown to be efficient in clinical situations, such as Rituxan (Rituximab), Herceptin (Trastuzumab) Xolair (Omalizumab), Bexxar (Tositumomab), Campath (Alemtuzumab), Zevalin, Oncolym and similar formulations may be used with the antibodies of this invention.

Also provided are kits which include a pharmaceutical composition containing an anti-CD39 antibody, an anti-CD73 antibody, and a pharmaceutically-acceptable carrier, in a therapeutically effective amount adapted for use in the preceding methods. The kits optionally also can include instructions, e.g., comprising administration schedules, to allow a practitioner (e.g., a physician, nurse, or patient) to administer the composition contained therein to administer the composition to a patient having cancer (e.g., a solid tumor). The kit also can include a syringe.

Optionally, the kits include multiple packages of the single-dose pharmaceutical compositions each containing an effective amount of the anti-CD39 or anti-CD73 antibody for a single administration in accordance with the methods provided above. Instruments or devices necessary for administering the pharmaceutical composition(s) also may be included in the kits. For instance, a kit may provide one or more pre-filled syringes containing an amount of the anti-CD39 antibody and syringes containing an amount of anti-CD73 antibody, or syringes containing amounts of both anti-CD39 and anti-CD73 antibodies.

In one aspect, the present invention provides a kit for treating a cancer in a human patient, the kit comprising:
(a) a dose of an anti-CD39 antibody that neutralizes the activity of sCD39, optionally wherein the antibody comprises the hypervariable region (e.g. CDR1, CDR2 and CDR3 domains) of a heavy chain variable region of antibody I-394, I-395, I-396 or I-399, and the hypervariable region (e.g. CDR1, CDR2 and CDR3 domains) of a light chain variable region of antibody I-394, I-395, I-396 or I-399;
(b) a dose of an agent that binds CD73 and that neutralizes the activity of CD73, optionally wherein the agent is an anti-CD73 antibody; and
(c) optionally, instructions for using the anti-CD39 antibody and CD73 binding agent in any of the methods described herein.

### Diagnostics, prognostics, and treatment of malignancies

Described are methods useful in the diagnosis, prognosis, monitoring, treatment and prevention of a cancer in an individual. While the treatment regimens and methods described herein are particularly useful for the treatment of solid tumors, the treatment regimens and methods described herein can also be used for a variety of hematological cancers. The compositions of the present invention are utilized for example the treatment of a variety of cancers and other proliferative diseases including, but not limited to: carcinoma, including that of the bladder, breast, colon, kidney, liver, lung, ovary, uterus, prostate, pancreas, stomach, cervix, thyroid, head and neck (head and neck squamous cell carcinoma, and skin (e.g. melanoma); hematopoietic tumors of lymphoid lineage, including leukemia, acute lymphocytic leukemia, chronic lymphocytic leukemia, acute lymphoblastic leukemia, B-cell lymphoma, T-cell lymphoma, Hodgkins lymphoma, non-Hodgkins lymphoma, hairy cell lymphoma and Burketts lymphoma, and multiple myeloma; hematopoietic tumors of myeloid lineage, including acute and chronic myelogenous leukemias, promyelocytic leukemia, and myelodysplastic syndrome; tumors of mesenchymal origin, including fibrosarcoma and rhabdomyoscarcoma; other tumors, including melanoma, seminoma, terato-carcinoma, neuroblastoma and glioma; tumors of the central and peripheral nervous system, including astrocytoma, neuroblastoma, glioma, and schwannomas; tumors of mesenchymal origin, including fibrosarcoma, rhabdomyoscaroma, and osteosarcoma; and other tumors, including melanoma, xeroderma pigmentosum, keratoacanthoma, seminoma, and thyroid follicular cancer.

In one aspect, the anti-CD39 antibodies described herein can be used advantageously to treat a cancer that is CD73-positive.

A CD73-positive cancer is a cancer known to be generally characterized by presence of CD73-expressing cells in the tumor or tumor environment. Accordingly, an individual having a cancer can be treated with the anti-CD39 antibody with our without a prior detection step to assess expression of CD73 on cells in the tumor microenvironment (e.g. on tumor cells, CD4 T cells, CD8 T cells, B cells).

Optionally, the treatment can comprise a step of detecting a CD73 nucleic acid or polypeptide in a biological sample of a tumor from an individual (e.g., in cancer tissue, tissue proximal to or at the periphery of a cancer, cancer adjacent tissue, adjacent non-tumorous tissue or normal adjacent tissue). A determination that a biological sample is characterized by CD73 polypeptide, e.g. comprises cells expressing CD73, indicates that the patient has a cancer that may have a strong benefit from treatment with an agent that inhibits sCD39 (optionally further in combination with an agent that inhibits CD73). A patient having a cancer suitable for treatment according to the disclosure may be determined to have tumor that prominently expresses CD73; that expresses CD73 at a high level (e.g. compared to a reference value, compared to a healthy individual, at a level corresponding to individuals that are poor responders for treatment with an anti-CD73 agent, at a level corresponding to individuals whose tumor are resistant to one or more immunotherapies), that show high intensity of staining with an anti-CD73 antibody.

In one aspect, the method comprises determining the level of expression of a CD73 nucleic acid or polypeptide in a biological sample and comparing the level to a reference level corresponding to a healthy individual. A determination that a biological sample comprises cells expressing CD73 nucleic acid or polypeptide at a level that is increased compared to the reference level indicates that the patient has a cancer that can be treated with an anti-CD39 antibody. Optionally, detecting a CD73 polypeptide in a biological sample comprises detecting CD73 polypeptide expressed on the surface of a malignant cell, a CD4 T cell, CD8 T cell, B cell. In one aspect, a determination that a biological sample comprises cells that prominently expresses CD73 nucleic acid or polypeptide indicates that the patients has a cancer that can be treated with an anti-CD39 antibody. "Prominently expressed", when referring to a CD73 polypeptide, means that the CD73 polypeptide is expressed in a substantial number of cells taken from a given patient. While the definition of the term "prominently expressed" is not bound by a precise percentage value, in some examples a receptor said to be "prominently expressed" will be present on at least 10%, 20% 30%, 40%, 50°%, 60%, 70%, 80%, or more of the tumor cells or the cells in tumor tissue or tumoradjacent tissue sample (e.g. biopsy) taken from a patient.

Determining whether an individual has a cancer characterized by cells that express a CD73 polypeptide can for example comprise obtaining a biological sample (e.g. by performing a biopsy) from the individual that comprises cells from the cancer environment (e.g. tumor or tumor adjacent tissue), bringing said cells into contact with an antibody that binds an CD73 polypeptide, and detecting whether the cells express CD73 on their surface. Optionally, determining whether an individual has cells that express CD73 comprises conducting an immunohistochemistry assay.

Combination therapies for the treatment of cancer provided herein involve administration of a neutralizing anti-CD39 agent (e.g. an antibody) and a CD73-neutralizing agent (e.g. an antibody), to treat subjects afflicted with cancer. In one aspect, the present disclosure provides an anti-CD39 antibody and an anti-CD73 antibody, for use in combination, to treat subjects having a solid tumor (e.g., a solid tumor, an advanced refractory solid tumor) or subjects having a hematological tumor.

As used herein, adjunctive or combined administration (co-administration) includes simultaneous administration of the compounds in the same or different dosage form, or separate administration of the compounds (e.g., sequential administration). Thus, the anti-CD39 and anti-CD73 antibodies can be simultaneously administered in a single formulation. Alternatively, the anti-CD39 and anti-CD73 antibodies can be formulated for separate administration and are administered concurrently or sequentially.

A patient having a cancer can be treated with the anti-CD39 agent and anti-CD73 agent with or without a prior detection step to assess tumoral ATPase activity, 5'-ectonucleotidase activity, tumoral adenosine accumulation (e.g., intratumoral adenosine concentration), and/or CD39 and/or CD73 expression on cells (e.g., circulating and/or tumor-infiltrating leukocytes, T Reg cells, B cells and/or on tumor cells). Optionally, the treatment can comprise a step of detecting tumoral ATPase activity and/or tumoral adenosine accumulation (e.g., elevated intratumoral adenosine concentration) in a biological sample from an individual having a tumor (e.g., a sample comprising tumor tissue and/or tumor adjacent tissue). A determination that a biological sample has elevated tumoral ATPase activity or tumoral adenosine accumulation (e.g., intratumoral adenosine concentration), for example compared to a reference, indicates that the individual has a cancer that may have a strong benefit from treatment with an agent that inhibits CD39 in combination with an agent that inhibits CD73. Optionally, the treatment can comprise a step of detecting a CD39 nucleic acid or polypeptide in a biological sample of a tumor (e.g., on a tumor cell) from an individual. A determination that a biological sample expresses CD39 (e.g., tumor cells, tumor infiltrating cells or generally TReg cells express CD39, cells express CD39 at a high level, a high number of cells are CD39-positive, high intensity of staining with an anti-CD39 antibody, compared to a reference) indicates that the individual has a cancer that may have a strong benefit from treatment with an agent that inhibits soluble CD39 protein, optionally in combination with an agent that inhibits CD73.

Optionally, the treatment can comprise a step of detecting a CD39 nucleic acid or polypeptide in a biological sample from an individual. Examples of biological samples include any suitable biological fluid (for example serum, lymph, blood), cell sample, or tissue sample. Any determination that cells in a biological sample (e.g., cancer cells, lymphocytes, e.g. TReg cells, B cells, T cells) express CD39 at a high level, or that a high number of cells in the sample are CD39-positive, or show high intensity of staining with an anti-CD39 antibody, compared to a reference) can indicate that the individual has a cancer that may have a strong benefit from treatment with an agent that inhibits CD39 in combination with an agent that inhibits CD73. In one aspect, the treatment can comprise a step of detecting a CD39 nucleic acid or polypeptide in a biological sample of a tumor (e.g., on a tumor-infiltrating cell) from an individual.

In the use for treatment methods, the anti-CD39 antibody and the CD73-neutralizing agent (e.g. anti-CD73 antibodies) can be administered separately, together or sequentially, or in a cocktail. In some aspects, the anti-CD39 antibody is to be administered prior to the administration of the CD73-neutralizing agent. For example, the anti-CD39 antibody can be administered approximately 0 to 30 days prior to the administration of the CD73-neutralizing agent. In some aspects, an anti-CD39 antibody is to be administered from about 30 minutes to about 2 weeks, from about 30 minutes to about 1 week, from about 1 hour to about 2 hours, from about 2 hours to about 4 hours, from about 4 hours to about 6 hours, from about 6 hours to about 8 hours, from about 8 hours to 1 day, or from about 1 to 5 days prior to the administration of the CD73-neutralizing agent. In some aspects, an anti-CD39 antibody is to be administered concurrently with the administration of the CD73-neutralizing agent. In some asects, an anti-CD39 antibody is to be administered after the administration of the anti-CD73 antibody. For example, an anti-CD39 antibody can be administered approximately 0 to 30 days after the administration of the CD73-neutralizing agent. In some aspects, an anti-CD39 antibody is to be administered from about 30 minutes to about 2 weeks, from about 30 minutes to about 1 week, from about 1 hour to about 2 hours, from about 2 hours to about 4 hours, from about 4 hours to about 6 hours, from about 6 hours to about 8 hours, from about 8 hours to 1 day, or from about 1 to 5 days after the administration of the CD73-neutralizing agent.

Suitable treatment protocols for treating a human having cancer include, for example, administering to the patient an effective amount of each of an antibody that inhibits the activity of CD39 and an antibody that neutralizes the activity of human CD73, wherein the method comprises at least one administration cycle in which at least one dose of the anti-CD39 antibody is administered at a dose of 1-20 mg/kg body weight and at least one dose of the anti-CD73 antibody is administered at a dose of 1-20 mg/kg body weight. In one embodiment, the administration cycle is between 2 weeks and 8 weeks.

In one aspect, the use for treating comprises at least one administration cycle, wherein the cycle is a period of eight weeks or less, wherein for each of the at least one cycles, two, three or four doses of the anti-CD39 antibody are administered at a dose of 1-20 mg/kg body weight and two, three or four doses of the anti-CD73 antibody are administered at a dose of 1-20 mg/kg body weight.

In one aspect, the anti-CD39 antibodies are to be administered in an amount effective to neutralize the enzymatic activity of sCD39 and/or memCD39 for a desired period of time, e.g., 1 week, 2 weeks, a month, until the next successive administration of anti-CD39 antibody. In one aspect, the antibodies are to be administered at a dosage and/or frequency that provides a blood concentration of antibody equal to at least the EC₅₀, EC₇₀ or EC₁₀₀ for inhibition of ATPase activity of sCD39 protein, optionally wherein the concentration is maintained for at least 1 week, 2 weeks, a month, or until the next successive administration of the anti-CD39 antibody.

In one aspect the anti-CD73 antibody and the anti-CD39 antibody are to be administered by i.v. In one aspect the anti-CD73 antibody and the anti-CD39 antibody are to be administered on the same day, optionally further once about every two weeks, optionally further by i.v.

In any aspect herein, the use for treating can comprise administering to the individual an anti-CD39 antibody that neutralizes the enzymatic activity of CD39 for at least one administration cycle in which the anti-CD39 antibody is administered at least once, optionally at least twice, in an amount effective to achieve, and/or to maintain between two successive administrations of the anti-CD39 antibody, a concentration in blood (serum) or an extravascular tissue (e.g. tumor environment) that corresponds to at least the in vitro EC₅₀ (e.g. an EC₅₀ between 0.01 and 0.5 µg/ml), optionally the EC₇₀ or optionally the EC₁₀₀, for neutralization of the enzymatic activity of CD39 (e.g. an EC₁₀₀ between 0.05 and 1 µg/ml, between 0.1 and 1 µg/ml). The in vitro EC₅₀, EC₇₀ or EC₁₀₀ can be determined, for example, according to the methods disclosed herein for testing the neutralizing activity of an anti-CD39 antibody. The antibody can for example be administered in an amount to achieve and/or maintained a concentration in circulation or in an extravascular tissue (e.g. tumor environment) of at least about 0.1 µg/ml, 0.5 µg/ml, 1 µg/ml or 2 µg/ml). For example, to achieve a concentration in an extravascular tissue of between 0.05 and 1 µg/ml, or between 0.1 and 1 µg/ml, the anti-CD39 antibody is administered in amounts effective to achieve a concentration in circulation of the anti-CD39 antibody of between 0.5 and 10 µg/ml, or between 1 and 10 µg/ml. Optionally, the anti-CD39 antibody is administered at least twice and in amounts effective to maintain the concentration of the anti-CD39 antibody at least the aforementioned concentration for at least 1 week, 2 weeks, 3 weeks, 4 weeks, between two successive administrations of the anti-CD39 antibody and/or throughout the administration cycle.

In any aspect herein, the use for treating can comprise administering to the individual an anti-CD73 antibody that neutralizes the enzymatic activity of CD73 for at least one administration cycle in which the anti-CD73 antibody is administered at least once, optionally at least twice, in an amount effective to achieve, and/or to maintain between two successive administrations of the anti-CD73 antibody, a concentration in blood (serum) or an extravascular tissue (e.g. tumor environment) that corresponds to at least the in vitro EC₅₀ (e.g. an EC₅₀ between 0.01 and 0.5 µg/ml), optionally the EC₇₀ or optionally the EC₁₀₀, for neutralization of the enzymatic activity of CD73 (e.g. an EC₁₀₀ between 0.05 and 1 µg/ml, between 0.1 and 1 µg/ml). The antibody can for example be administered in an amount to achieve and/or maintained a concentration in circulation or in an extravascular tissue (e.g. tumor environment) of at least about 0.1 µg/ml, 0.5 µg/ml, 1 µg/ml or 2 µg/ml). For example, to achieve a concentration in an extravascular tissue of between 0.05 and 1 µg/ml, or between 0.1 and 1 µg/ml, the anti-CD73 antibody is administered in amounts effective to achieve a concentration in circulation of the anti-CD73 antibody of between 0.5 and 10 µg/ml, or between 1 and 10 µg/ml. Optionally, the anti-CD73 antibody is administered at least twice and in amounts effective to maintain the concentration of the anti-CD73 antibody at least the aforementioned concentration for at least 1 week, 2 weeks, 3 weeks, 4 weeks, between two successive administrations of the anti-CD73 antibody and/or throughout the administration cycle.

In certain aspects an anti-CD39 agent and anti-CD73 agent can be used to treat a cancer in an individual having immune effector cells characterized by one or more markers of exhaustion and/or immunosuppression.

In certain aspects an anti-CD39 agent in combination with an anti-CD73 agent can be for use to treat a cancer in an individual having a poor disease prognosis for response to a an anti-CD73 agent, for example a poor prognosis evidenced by one or more markers indicative of lack of a sufficient anti-tumor immune response, indicative of immune exhaustion, and/or indicative of immunosuppression notably a poor prognosis for response to treatment with an agent that neutralizes CD73 (e.g., an anti-CD73 antibody). An individual having a poor disease prognosis, e.g., is at a higher risk of progression, based on one or more predictive factors. In one aspect, the predictive factor(s) comprises possessing or lacking a mutation in one or more genes. In one aspect, the predictive factor(s) comprises level(s) of expression of one or more genes or proteins (e.g., a gene signature).

In one aspect, a predictive factor(s) comprises presence (e.g., numbers) of cells in circulation or in the tumor environment expressing CD39 and/or CD73, and/or expression levels of CD39 and/or CD73 on cells in circulation or in the tumor environment; in one embodiment, the cells are tumor cells; in one embodiment the cells are leukocytes, e.g., B cells, regulatory T cells (Treg). Presence of elevated expression of CD39 and/or CD73, and/or elevated levels of CD39- and/or CD73-expressing cells can indicate an individual has a poor prognosis for response to treatment with an antibody that neutralizes CD73.

In one aspect, an anti-CD39 agent can be for use to treat a cancer in an individual who is a non-responder, or who has experienced a partial or an incomplete response to treatment with an agent that neutralizes CD73 (e.g., an anti-CD73 antibody), or whose disease has progressed following treatment with an agent that neutralizes CD73. In one aspect, the individual is treated with with an anti-CD39 agent in combination with an agent that neutralizes CD73.

In one aspect, an anti-CD39 agent can be for use to treat a cancer in an individual who has a poor prognosis for response to an agent (e.g., an antibody) that inhibits CTLA-4 or the PD-1 axis, or who is a non-responder, or who has experienced a partial or an incomplete response to treatment with an agent (e.g., an antibody) that inhibits CTLA-4 or the PD-1 axis, or whose disease has progressed following treatment with an agent (e.g., an antibody) that inhibits CTLA-4 or the PD-1 axis. In one aspect, the individual is treated with with an anti-CD39 agent in combination with an antibody that neutralizes CD73.

The anti-CD39 antibody compositions further in combination with an agent that binds and inhibits CD73 may optionally be combined (further combined) treatments with one or more other treatment or therapeutic agents. Such therapeutic agents include, but are not limited to anti-cancer agents and chemotherapeutic agents.

In one aspect, the additional therapeutic agent is an agent or treatment that induces the death of tumor cells, e.g., an agent or treatment that is capable of inducing the extracellular release of ATP from tumor cells, an agent or treatment that induces immunogenic cancer cell death. Extracellular ATP is released from tumor cells in case of stress (mechanical, hypotonic or hypoxic) or in case of cell death. Necrosis favors the passive release of ATP through the release of total cellular content, whereas apoptosis favors the release of ATP by activation of caspases 3 and 9 which cleave and activate Panexin1 (ATP transporter). Examples of agents that induce the extracellular release of ATP from tumor cells can include chemotherapy, radiotherapy, and, more generally, agents that induce apoptosis and thereby favor ATP release. Agents that induce the extracellular release of ATP have been shown to induce immunogenic cell death. For example, substantial ATP release is induced by anthracyclines, oxaliplatin, cisplatin and X-rays. Further example of agents that induce the extracellular release of ATP can include taxanes, anthracyclines, camptothecins, epothilones, mytomycins, combretastatins, vinca alkaloids, nitrogen mustards, maytansinoids, calicheamycins, duocarmycins, tubulysins, dolastatins and auristatins, enediynes, amatoxins, pyrrolobenzodiazepines, ethylenimines, radioisotopes, therapeutic proteins and peptides, and toxins or fragments thereof. The agents can be in any suitable configuration or formulation, including for example as free compound or as part of a conjugate. Agents can conveniently be conjugated to a targeting moiety, such as in an immunoconjugate. The terms "immunoconjugate" and "antibody conjugate" are used interchangeably and refer to an antigen binding agent, e.g., an antibody binding protein or an antibody that is conjugated to another moiety (e.g., a cytotoxic agent). An immunoconjugate comprising an antigen binding agent conjugated to a cytotoxic agent can also be referred to as a "antibody drug conjugate" or an "ADC".

In one aspect, the additional therapeutic agent is an agent (e.g., an antibody) that inhibits CTLA-4 or the PD-1 axis (i.e. inhibits PD-1 or PD-L1). Antibodies that bind CTLA-4, PD1 or PD-L1 can be used, for example, at the exemplary the doses and/or frequencies that such agents are used as monotherapy, e.g., as described below.

In one aspect, the second or additional second therapeutic agent is an agent (e.g., an antibody) that inhibits CTLA-4 or the PD-1 axis (i.e. inhibits PD-1 or PD-L1). Antibodies that bind CTLA-4, PD1 or PD-L1 can be used, for example, at the exemplary the doses and/or frequencies that such agents are used as monotherapy, e.g., as described below.

PD-1 is an inhibitory member of the CD28 family of receptors that also includes CD28, CTLA-4, ICOS and BTLA. PD-1 is expressed on activated B cells, T cells, and myeloid cells Okazaki et al. (2002) Curr. Opin. Immunol. 14: 391779-82; Bennett et al. (2003) J Immunol 170:711-8). Two ligands for PD-1 have been identified, PD- L1 and PD-L2, that have been shown to downregulate T cell activation upon binding to PD-1 (Freeman et al. (2000) J Exp Med 192:1027-34; Latchman et al. (2001) Nat Immunol 2:261-8; Carter et al. (2002) Eur J Immunol 32:634-43). PD-L1 is abundant in a variety of human cancers (Dong et al. (2002) Nat. Med. 8:787-9). The interaction between PD-1 and PD-L1 results in a decrease in tumor infiltrating lymphocytes, a decrease in T-cell receptor mediated proliferation, and immune evasion by the cancerous cells. Immune suppression can be reversed by inhibiting the local interaction of PD-1 with PD-L1, and the effect is additive when the interaction of PD-1 with PD-L2 is blocked as well. Blockade of PD-1 can advantageously involve use of an antibody that prevents PD-L1-induced PD-1 signalling, e.g. by blocking the interaction with its natural ligand PD-L1. In one aspect the antibody binds PD-1 (an anti-PD-1 antibody); such antibody may block the interaction between PD-1 and PD-L1 and/or between PD-1 and PD-L2. In another aspect the antibody binds PD-L1 (an anti-PD-L1 antibody) and blocks the interaction between PD-1 and PD-L1.

There are currently at least six agents blocking the PD-1/PD-L1 pathway that are marketed or in clinical evaluation, any of these may be useful in combination with the anti-CD73 antibodies of the disclosure. One agent is BMS-936558 (Nivolumab/ONO-4538, Bristol- Myers Squibb; formerly MDX-1106). Nivolumab, (Trade name Opdivo^{®}) is an FDAapproved fully human IgG4 anti-PD-L1 mAb that inhibits the binding of the PD-L1 ligand to both PD-1 and CD80 and is described as antibody 5C4 in WO 2006/121168. For melanoma patients, the most significant OR was observed at a dose of 3 mg/kg, while for other cancer types it was at 10 mg/kg. Nivolumab is generally dosed at 10 mg/kg every 3 weeks until cancer progression.

Another agent is durvalumab (Imfinzi^{®}, MEDI-4736), an anti-PD-L1 developed by AstraZeneca/Medimmune and described in WO2011/066389 and US2013/034559.

Another agent is MK-3475 (human IgG4 anti-PD1 mAb from Merck), also referred to as lambrolizumab or pembrolizumab (Trade name Keytruda^{®}) has been approved by the FDA for the treatment of melanoma and is being tested in other cancers. Pembrolizumab was tested at 2 mg/kg or 10 mg/kg every 2 or 3 weeks until disease progression.

Another agent is atezolizumab (Tecentriq^{®}, MPDL3280A/RG7446, Roche/Genentech), a human anti-PD-L1 mAb that contains an engineered Fc domain designed to optimize efficacy and safety by minimizing FcyR binding and consequential antibody-dependent cellular cytotoxicity (ADCC). Doses of ≤1, 10, 15, and 25 mg/kg MPDL3280A were administered every 3 weeks for up to 1 year. In phase 3 trial, MPDL3280A is administered at 1200 mg by intravenous infusion every three weeks in NSCLC.

Further known PD-1 antibodies and other PD-1 inhibitors include cemiplimab (Sanofi and Regeneron Pharmaceuticals), MGA012 (Macrogenics inc. and Incyte Corp.), pidlizumab (CT-011; CureTech) (humanized IgG1 anti-PD1 mAb from CureTech/Teva, see e.g., WO2009/101611), AMP-224 (a B7-DC/IgG1 fusion protein licensed to GSK), AMP- 514 described in WO 2012/145493, antibody YW243.55.S70 (an anti-PD-L1) described in WO2010/077634, MDX-1105, also known as BMS-936559, is an anti-PD-L1 antibody developed by Bristol-Myers Squibb described in WO2007/005874, and antibodies and inhibitors described in WO2006/121168, WO2009/014708, WO2009/114335 and WO2013/019906. Further examples of anti-PD1 antibodies are disclosed in WO2015/085847 (Shanghai Hengrui Pharmaceutical Co. Ltd.). Antibodies that compete with any of these antibodies for binding to PD-1 or PD-L1 also can be used.

CTLA-4 (cytotoxic T-lymphocyte-associated protein 4), also known as CD152 is another inhibitory member of the CD28 family of receptors, and is expressed on T cells. Antibodies that bind and inhibit CTLA-4 are known in the art. In one example, the antibody is ipilimumab (trade name Yervoy^{®}, Bristol-Myers Squibb), a human IgG antibody. In one example, the antibody is tremelimumab (CP-675,206; Medimmune/AstraZeneca). An exemplary administration regimen for Yervoy is 3 mg/kg invtravenously over 90 minutes every three weeks. In one example, the antibody used in combination with the anti-CD73 antibodies of the disclosure is an antibody that competes with ipilimumab or tremelimumab for binding to CTLA-4.

### Examples

### Methods

### Generation of CD39 mutants

CD39 mutants were generated by PCR. The sequences amplified were run on agarose gel and purified using the Macherey Nagel PCR Clean-Up Gel Extraction kit (reference 740609). The purified PCR products generated for each mutant were then ligated into an expression vector, with the ClonTech InFusion system. The vectors containing the mutated sequences were prepared as Miniprep and sequenced. After sequencing, the vectors containing the mutated sequences were prepared as Midiprep using the Promega PureYield^{™} Plasmid Midiprep System. HEK293T cells were grown in DMEM medium (Invitrogen), transfected with vectors using Invitrogen's Lipofectamine 2000 and incubated at 37°C in a CO2 incubator for 48 hours prior to testing for transgene expression. Mutants were transfected in Hek-293T cells, as shown in the table below. The targeted amino acid mutations in the table 1 below are shown using numbering of SEQ ID NO: 2.

**Table 1**

| ***Mutant*** | ***Substitutions*** | | | | | |
|---|---|---|---|---|---|---|
| **1** | V77G | H79Q | Q444K | G445D | | |
| **2A** | V81S | E82A | R111A | V115A | | |
| **2B** | E110A | R113T | E114A | | | |
| **3** | R118A | S119A | Q120K | Q122H | E123A | |
| **4** | D150A | E153S | R154A | S157K | N158A | L278F |
| **5** | Q96A | N99A | E143A | R147E | | |
| **6** | K188R | Replacement of the residues 190 to 207 by KTPGGS | | | | |
| **7** | A273S | N275A | I2775 | R279A | | |
| **8** | S294A | K298G | K303A | E306A | T308K | Q312A |
| **9** | K288E | K289A | V290A | E315R | | |
| **10A** | Q354A | D356S | E435A | H436Q | | |
| **10B** | H428A | T430A | A431D | D432A | | |
| **11** | N371K | L372K | E375A | K376G | lnsertion377V | V377S |
| **12** | K388N | Q392K | P393S | E396A | | |
| **13** | A402P | G403A | K405A | E406A | | |
| **15** | K87A | E100A | D107A | | | |
| **16** | Q323A | Q324A | Q327A | E331K | | |
| **17** | N334A | S336A | Y337G | N346A | | |
| **18** | Q228A | I230S | D234A | Q238A | | |
| **19** | R138A | M139A | E142K | | | |

### Cloning, production and purification of soluble huCD39

### Molecular Biology

The huCD39 protein was cloned from human PBMC cDNA using the following primers TACGACTCACAAGCTTGCCGCCACCATGGAAGATACAAAGGAGTC (SEQ ID NO: 38) (Forward), and CCGCCCCGACTCTAGATCACTTGTCATCGTCATCTTTGTAATCGA CATAGGTGGAGTGGGAGAG (SEQ ID NO: 56) (Reverse). The purified PCR product was then cloned into an expression vector using the InFusion cloning system. A M2 tag (FLAG tag, underlined in SEQ ID NO: 39) was added in the C-terminal part of the protein for the purification step; it will be appreciated that a CD39 extracellular domain protein (e.g., of SEQ ID NO: 54) can in any embodiment optionally be specified to lack the M2 tag.

### Expression and purification of the huCD39 proteins

After validation of the sequence cloned, CHO cells were nucleofected and the producing pool was then sub-cloned to obtain a cell clone producing the huCD39 protein. Supernatant from the huCD39 clone grown in roller was harvested and purified using M2 chromatography column and eluted using the M2 peptide. The purified proteins were then loaded onto a S200 size exclusion chromatography column. The purified protein corresponding to a monomer was formulated in a TBS PH7.5 buffer. The amino acid sequence of the CD39-M2 extracellular domain recombinant protein without M2 tag was as follows:

MEDTKESNVKTFCSKNILAILGFSSIIAVIALLAVGLTQNKALPENVKYGIVLDAGSSHTSLYIY
KWPAEKENDTGVVHQVEECRVKGPGISKFVQKVNEIGIYLTDCMERAREVIPRSQHQETPV
YLGATAGMRLLRMESEELADRVLDVVERSLSNYPFDFQGARIITGQEEGAYGWITINYLLGK
FSQKTRWFSIVPYETNNQETFGALDLGGASTQVTFVPQNQTIESPDNALQFRLYGKDYNVY
THSFLCYGKDQALWQKLAKDIQVASNEILRDPCFHPGYKKVVNVSDLYKTPCTKRFEMTLP
FQQFEIQGIGNYQQCHQSILELFNTSYCPYSQCAFNGIFLPPLQGDFGAFSAFYFVMKFLNL
TSEKVSQEKVTEMMKKFCAQPWEEIKTSYAGVKEKYLSEYCFSGTYILSLLLQGYHFTADS
WEHIHFIGKIQGSDAGWTLGYMLNLTNMIPAEQPLSTPLSHSTYV
(SEQ ID NO: 5).

The final amino acid sequence of the CD39-M2 extracellular domain recombinant protein with the M2 tag was as follows:

MEDTKESNVKTFCSKNILAILGFSSIIAVIALLAVGLTQNKALPENVKYGIVLDAGSSHTSLYIY
KWPAEKENDTGVVHQVEECRVKGPGISKFVQKVNEIGIYLTDCMERAREVIPRSQHQETPV
YLGATAGMRLLRMESEELADRVLDVVERSLSNYPFDFQGARIITGQEEGAYGWITINYLLGK
FSQKTRWFSIVPYETNNQETFGALDLGGASTQVTFVPQNQTIESPDNALQFRLYGKDYNVY
THSFLCYGKDQALWQKLAKDIQVASNEILRDPCFHPGYKKVVNVSDLYKTPCTKRFEMTLP
FQQFEIQGIGNYQQCHQSILELFNTSYCPYSQCAFNGIFLPPLQGDFGAFSAFYFVMKFLNL
TSEKVSQEKVTEMMKKFCAQPWEEIKTSYAGVKEKYLSEYCFSGTYILSLLLQGYHFTADS
WEHIHFIGKIQGSDAGWTLGYMLNLTNMIPAEQPLSTPLSHSTYVDYKDDDDK
(SEQ ID NO: 54).

### Inhibition of the enzymatic activity of soluble CD39

The inhibition by antibodies of the enzymatic activity of soluble CD39 protein produced was evaluated using Cell Titer Glo^{™} (Promega, reference G7571) that allows assessment of ATP hydrolysis through use of a reagent that generates a luminescent signal proportional to the amount of ATP present. In this way, inhibition of the soluble-CD39-meidatd ATP hydrolysis can be assessed. Briefly, dose ranges of anti-CD39 antibodies from 100 µg/ml to 6×10⁻³ µg/ml were incubated with 400 ng/ml of soluble recombinant human CD39 protein having the amino acid sequence described in the Methods section (SEQ ID NO: 54), for 1h at 37°C. 20 µM ATP was added to the plates for 30 additional minutes at 37°C before addition of CTG (Cell Titer Glo) reagent. Emitted light was quantified using an Enspire^{™} luminometer after a short incubation period of 5 min in the dark. Anti-CD39 antibody efficacy was determined by comparing emitted light in presence of antibody with ATP alone (maximal light emission) and ATP together with soluble CD39 protein (minimal light emission).

### Inhibition of the enzymatic activity of cellular CD39

The inhibition of the CD39 enzymatic activity in CD39-expressing cells by antibodies was evaluated using Cell Titer Glo^{™} (Promega, reference G7571) that allows assessment of ATP hydrolysis through use of a reagent that generates a luminescent signal proportional to the amount of ATP present. The assay was thus designed to permit assessment of the inhibition of ATP hydrolyzed by CD39 in the cell culture supernatant. Briefly, 5×10⁴ Ramos human lymphoma cells, 5×10³ human CD39-, cynomolgus CD39 - and mouse CD39-expressing CHO cells, were incubated 1 hour at 37°C with anti-CD39 antibodies from 30 µg/ml to 5×10⁻⁴ µg/ml. Cells were then incubated with 20 µM ATP for 1 additional hour at 37°C. Plates were centrifuged for 2 min at 400g and 50 µl cell supernatant are transferred in a luminescence microplate (white wells). 50 µl CellTiter-Glo^{®} Reagent (CTG) was added to the supernatant and emitted light was quantified after a 5 min incubation in the dark using a Enspire^{™} luminometer. Anti-CD39 antibody efficacy was determined by comparing emitted light in presence of antibody with ATP alone (maximal light emission) and ATP together with cells (minimal light emission).

### Generation of antibodies: Immunization and screening in mice

To obtain anti-human CD39 antibodies, Balb/c mice were immunized with the recombinant human CD39-M2 extracellular domain recombinant protein described above. Mice received one primo-immunization with an emulsion of 50 µg CD39 protein and Complete Freund Adjuvant, intraperitoneally, a 2nd immunization with an emulsion of 50 µg CD39 protein and Incomplete Freund Adjuvant, intraperitoneally, and finally a boost with 10 µg CD39 protein, intravenously. Immune spleen cells were fused 3 days after the boost with X63.Ag8.653 immortalized B cells, and cultured in the presence of irradiated spleen cells. Hydridomas were plated in semi-solid methylcellulose-containing medium and growing clones were picked using a clonepix 2 apparatus (Molecular Devices).

### Example 1: Epitope mapping of known neutralizing CD39 mAbs

In order to gain insight into the function of antibodies that inhibit the enzymatic (ATPase) activity of cellular CD39, we investigated the epitopes bound by antibodies that have been reported to inhibit the ATPase activity of CD39 in cellular assays: BY40 disclosed in PCT publication no. WO2009/095478.

In order to define the epitopes of anti-CD39 antibodies, we designed CD39 mutants defined by substitutions of amino acids exposed at the molecular surface over the surface of CD39. Mutants were transfected in Hek-293T cells, as shown in Table 1, using numbering of SEQ ID NO: 2.

Dose-ranges of I-394 (10 - 2.5 - 0.625 - 0.1563 - 0.0391 - 0.0098 - 0.0024 - 0.0006 µg/ml) were tested on the 20 generated mutants by flow cytometry. BY40 antibodies both had complete loss of binding to cells expressing mutant 5 of CD39, without loss of binding to any other mutant. Mutant 5 contains amino acid substitutions at residues Q96, N99, E143 and R147. The position of Mutant 5 on the surface of CD39 is shown in **Figure 3A****.**

### Example 2: Known neutralizing CD39 mAbs are unable to inhibit the ATPase activity of recombinant soluble CD39 protein

The two antibodies that have been reported to inhibit the ATPase activity of CD39 in cellular assays (BY40 and BY12) were assessed to determine whether are able to inhibit the ATPase activity of recombinant soluble CD39 protein. The inhibition by antibodies of the enzymatic activity of soluble CD39 protein produced as described above was evaluated using Cell Titer Glo^{™} (Promega, reference G7571). The inhibition by antibodies of the enzymatic activity of cellular CD39 protein was evaluated as indicated above.

As expected, BY40 inhibited the ATPase activity of CD39 protein in cells. However, BY40 was unable to inhibit the enzymatic activity of soluble CD39 protein. Figure 2B shows a comparison of BY40 with the new antibodies identified herein.

### Example 3: Screening for new mAbs to block sCD39 activity

A series of immunizations were carried out in order to seek antibodies that neutralize the ATPase activity of sCD39. To obtain anti-human CD39 antibodies, animals were immunized with the recombinant human CD39-M2 extracellular domain recombinant protein described above. In total, 15 series of immunizations were carried out using different protocols and in different animals. Included were different mice strains, rats and rabbits.

In initial immunization protocols, the primary screen involved testing supernatant (SN) of growing clones by flow cytometry using wild type CHO and CHO expressing huCD39 cell lines. Cells were stained with 0.1 µM and 0.005µM CFSE, respectively. For the flow cytometry screening, all cells were equally mixed and the presence of reacting antibodies in supernatants was revealed by Goat anti-mouse polyclonal antibody (pAb) labeled with APC. For antibodies that bound huCD39, supernatants were then screened for inhibition of the enzymatic activity of soluble CD39 using the screening assay developed and described above (Methods).

Results showed that while numerous specific CD39-binding antibodies could be obtained, none of the antibodies from any of these immunizations showed any inhibition of the enzymatic activity of soluble CD39. One possibility is that dominant epitopes on CD39 do not include any epitopes suitably positioned at or near that catalytic site of CD39. In view of the few antibodies available that inhibit cellular CD39 and the known difficulties in inhibiting the catalytic sites of enzymes using antibodies, the absence of antibodies that neutralize sCD39 may indicate that it is not possible to obtain antibodies that inhibit soluble (extracellular domain) CD39. Other possibilities relate to non-functional screening assays and/or improperly folded or functioning soluble CD39 protein, particularly since the lack of any antibody that can inhibit soluble CD39 hampers validation of sCD39 blockade assays.

In view of the absence of antibodies able to inhibit soluble CD39, a further immunization was carried out with a screening protocol designed to favor the generation of antibodies that bind the active site of CD39 as identified by the epitope of antibody BY40. Briefly, the primary screen involved testing supernatant (SN) of growing clones by flow cytometry using wild type CHO and CHO expressing huCD39 cell lines, as in the preceding immunizations, followed by screening for loss of binding Hek-293T cells expressing CD39 mutant 5, compared to wild-type CD39, as shown in Table 1. Mutant 5 has substitutions at residues Q96, N99, E143 and R147. However, again results showed that while numerous specific CD39-binding antibodies could be obtained that showed loss of binding to mutant 5, none of the antibodies from any of the initial immunizations showed any inhibition of the enzymatic activity of soluble CD39.

### Example 4: Identification of a first antibody that inhibits sCD39 activity as part of an epitope-directed screen

We sought to identify anti-CD39 antibodies that do not bind the Q96, N99, E143 and R147 region (defined by mutant 5) in order to have antibodies that do not compete with BY40-like antibodies. Such antibodies which need not have any ability to block the ATPase activity of CD39 can be useful for pharmacology studies of antibodies that inhibit cellular CD39 which bind to the BY40 binding site, e.g., to detect and quantify free CD39 proteins on cells in the presence of BY40 or BY40-like antibodies that inhibit cellular CD39.

Starting from the results of the immunization of Example 3 in which hybridomas were screened for loss of binding to CD39 mutant 5, a hybridoma was selected that was not among those that showed loss of binding to CD39 mutant 5. This hybridoma (1-394) was among the broader pool possibly due to inconclusive data indicating possible partial decrease in binding to mutant 5, but I-394 did not lose binding to mutant 5 and was therefore not initially retained.

In the context of ongoing screening of supernatants from further immunizations for inhibition of the enzymatic activity of soluble CD39, the antibody I-394 that had been cloned and produced was included as a control. Surprisingly, despite antibody I-394 not being among the clones retained in the epitope-directed screen, this antibody showed strong inhibition of the enzymatic activity of soluble CD39 in the assay described above (Methods).

I-394 was produced with human constant regions of IgG1 isotype, with a modified Fc domain having the mutations L234A/L235E/G237A/A330S/P331S (Kabat EU numbering) which results in lack of binding to human Fcγ receptors CD16A, CD16B, CD32A, CD32B and CD64, Briefly, the VH and Vk sequences of the I-394 antibody (the VH and Vk variable regions shown in SEQ ID NOS: 6 and 7, respectively) were cloned into expression vectors containing the hulgG1 constant domains harboring the aforementioned mutations and the huCk constant domain respectively. The two obtained vectors were co-transfected into the CHO cell line. The established pool of cell was used to produce the antibody in the CHO medium. The antibody was then purified using protein A. The amino acid sequences of the respective heavy and light chain variable domains of I-394 are shown below (Kabat CDRs underlined).

I-394 heavy chain variable domain sequence:
EVQLQQSGPELVKPGASVKMSCKASGYTFTDYNMHWVKQSHGRTLEWIGYIVPLNGGSTFNQKFKGRA
TLTVNTSSRTAYMELRSLTSEDSAAYYCARGGTRFAYWGQGTLVTVSA (SEQ ID NO: 6).

I-394 light chain variable domain sequence:
DIVLTQSPASLAVSLGQRATISCRASESVDNFGVSFMYWFQQKPGQPPNLLIYGASNQGSGVPARFRG
SGSGTDFSLNIHPMEADDTAMYFCQQTKEVPYTFGGGTKLEIK (SEQ ID NO: 7).

The heavy and light chain sequences of I-394 with human IgG1 constant regions, with L234A/L235E/G237A/A330S/P331S substitutions (retaining N297-linked glycosylation) are shown below:
I-394 heavy chain sequence: 
EVQLQQSGPELVKPGASVKMSCKASGYTFTDYNMHWVKQSHGRTLEWIGYIVPLNGGSTFNQKFKGRA
TLTVNTSSRTAYMELRSLTSEDSAAYYCARGGTRFAYWGQGTLVTVSAASTKGPSVFPLAPSS
KSTSGGTAALGCLVKDYFPEPVTVSWNSGALTSGVHTFPAVLQSSGLYSLSSVVTVPSSSLGTQTYIC
NVNHKPSNTKVDKRVEPKSCDKTHTCPPCPAPEAEGAPSVFLFPPKPKDTLMISRTPEVTCVVVDVSH
EDPEVKFNWYVDGVEVHNAKTKPREEQYNSTYRVVSVLTVLHQDWLNGKEYKCKVSNKALPSSIEKTI
SKAKGQPREPQVYTLPPSREEMTKNQVSLTCLVKGFYPSDIAVEWESNGQPENNYKTTPPVLDSDGSF
FLYSKLTVDKSRWQQGNVFSCSVMHEALHNHYTQKSLSLSPGK
(SEQ ID NO: 52).
I-394 light chain sequence:

DIVLTQSPASLAVSLGQRATISCRASESVDNFGVSFMYWFQQKPGQPPNLLIYGASNQGSGVPARFRG
SGSGTDFSLNIHPMEADDTAMYFCQQTKEVPYTFGGGTKLEIKRTVAAPSVFIFPPSDEQLKSGT
ASVVCLLNNFYPREAKVQWKVDNALQSGNSQESVTEQDSKDSTYSLSSTLTLSKADYEKHKVYACEVT
HQGLSSPVTKSFNRGEC
(SEQ ID NO: 53).

Antibody I-394 was then tested for loss of binding to CD39 mutants defined by substitutions of amino acids exposed at the molecular surface over the surface of CD39. Mutants were transfected in Hek-293T cells, as shown in the table 1, using numbering of SEQ ID NO: 2. Dose-ranges of antibodies I-394 were tested on the 20 mutants by flow cytometry. As shown in Figure 3B, I-394 showed complete loss of binding to cells expressing mutant 19 of CD39. Mutant 19 includes substitutions at residues R138, M139 and E142. The core epitope of I-394 thus includes one or more (or all of) residues R138, M139 and E142.

Unlike prior antibody BY40 which loses binding to mutant 5 and has the ability to inhibit cellular CD39 but not soluble CD39, antibody I-394 loses binding to the adjacent mutant 19, with strongly reduced binding to mutant 5 (but with some residual binding to mutant 5). Interestingly, the residues of mutant 19 are in close proximity or adjacent to those of residue 5, such that I-394 may represent a shift in epitope compared to BY40. Antibody I-394 thus presents a valuable new epitope for anti-CD39 antibodies that permits inhibition of the ATPase activity of soluble CD39 protein. It also provides a specific positive control that permits the validation and testing of screening assays for detecting further antibodies that neutralize the ATPase activity of soluble CD39 protein.

### Example 5: A non-epitope directed screen for sCD39-neutralizating mAbs

Based on the results for Example 4 indicating the antibody-mediated inhibition of soluble CD39 is possible, fusions from the different immunizations using different protocols from Example 3 were revisited in order to seek antibodies that neutralize the ATPase activity of sCD39.

Different approaches for screening for ATPase inhibition were then evaluated. In one experiment, I-394 antibody was used to spike supernatants from hybridomas of an immunization of Example 3 that were found negative for ability to inhibit the ATPase activity of soluble CD39. This addition of I-394 to supernatant did not restore the ability of negative supernatants to inhibit ATPase activity of CD39. Antibody I-394 was then purified from the negative supernatant using Protein A coated beads, and we observed the purified I-394 was again able to inhibit of ATPase activity was restored.

In view of the foregoing results, new immunization and screening protocols were developed in which growing clones from new and past immunizations were screened by flow cytometry using wild type CHO and CHO expressing huCD39 cell lines without assessment of inhibition of soluble CD39 or cellular CD39 ATPase activity, and without screening bias for epitopes. While data regarding loss of binding to mutant 5 or 19 was available for some hybridomas, such data was not used for clone selection but only retained for purposes of rescuing hybridomas for cloning in the event of negative results in the ATPase blocking assay. Hybridomas that bind CD39 were selected and cloned, and then purified using Protein A according to the following protocol:
- Add to 300 µl of hybridomas supernatant 10µl of protein A beads
- Add NaCl to be at a final concentration of 1,5M
- Rotate the tubes for 3-4h at 4°C
- Centrifuge 1 min at 1500 rpm
- Eliminate the supernatant and perform three washes with 1 ml of TBS
- Eliminate all the TBS after the third wash
- Add 50 µl of Citrate 0,1 M pH3, homogenize and incubate at RT for 5 min
- Centrifuge the beads for 1 min at 1500 rpm
- Harvest the 50 µl of elution and add rapidly 450 µl of TBS and store at 4°C

The antibodies obtained were then screened in a comparative assay for the ability to inhibit the ATPase activity of CD39 to a similar degree as I-394. Assays used for inhibition of the enzymatic activity of soluble and cellular CD39 were as described above (Methods). Surprisingly, among the exemplary antibodies produced in this way, several showed inhibition of soluble CD39 (as well as inhibition of cellular CD39). **Figure 1** shows a representative screening result, showing antibodies I-397, I-398 and I-399 compared to positive control I-394 antibody. Similarly, antibodies I-395 and I-396 from different immunization inhibited the enzymatic activity of soluble CD39 protein. **Figures 2A and 2B** shows results for antibodies I-395 and I-396 for which greater quantities of antibodies were available for additional experiments for both soluble and cellular CD39 neutralization. **Figure 2A** shows that antibodies I-395 and I-396 both inhibit cell-membrane bound CD39 in comparison to BY40 and I-394 antibodies, with both I-394 and I-395 showing greater potency and maximal inhibition of cellular CD39 compared to BY40. **Figure 2B** shows that antibodies I-395 and I-396 both inhibit soluble CD39 in comparison to BY40 and I-394 antibodies. While BY40 does not inhibit soluble CD39 at any concentration, 1-394, I-395 and I-396 all inhibit soluble CD39 with I-394 showing the greatest potency, followed by I-395 and then I-396 with lower potency.

The results obtained raise the possibility that factor(s) in hybridoma supernatants are rapidly hydrolyzing ATP in both cell culture and in the soluble CD39 assay, such that no signal for ATP is detected in screening of antibodies using conventional methods. The soluble factor may be CD39 or some other enzyme, for example produced by the fusion partner.

Antibodies were then cloned, with modification to have a human constant regions with an IgG1 Fc domain having the mutations L234A/L235E/G237A/A330S/P331S (Kabat EU numbering) which results in lack of binding to human Fcγ receptors CD16A, CD16B, CD32A, CD32B and CD64, in the same way as shown herein for I-394. The resulting antibodies can then be subjected to titrations and then more detailed activity assessment as shown in Example 7-9 (titration, inhibition of ATPase activity) to assess EC₅₀ and IC₅₀ determinations to rank antibodies according to potency.

### Example 6: Epitope mapping of sCD39 neutralizing mAbs

As shown in Example 4, I-394 showed complete loss of binding to cells expressing mutant 19 of CD39, but did not lose binding to mutant 5. In order to define the epitopes of the further anti-CD39 antibodies of Example 5, they were tested for loss of binding to the panel of CD39 mutants as described in Example 1 and Table 1. Mutants were transfected in Hek-293T cells, as shown in the table 1, using numbering of SEQ ID NO: 2. Dose-ranges of test antibodies (10 - 2.5 - 0.625 - 0.1563 - 0.0391 - 0.0098 - 0.0024 - 0.0006 µg/ml) are tested on the 20 generated mutants by flow cytometry.

Results showed that the antibodies selected in Example 5 for ability to inhibit soluble CD39 represented several different epitopes. Among the antibodies that showed inhibition of soluble extracellular CD39 in Example 5, antibody I-395 is an example of an antibody that displayed loss of binding to mutant 5 having substitutions at residues Q96, N99, E143 and R147, and also loss of binding to mutant 19 having substitutions at residues R138, M139 and E142. Mutant 19 includes substitutions at residues R138, M139 and E142. The core epitope on CD39 of I-395 thus comprises one, two, three or four of residues Q96, N99, E143 and R147 as well as one, two or three of residues R138, M139 and E142.

Antibody I-398 on the other hand, is an example of an antibody that displayed loss of binding to mutant 19 having substitutions at residues R138, M139 and E142, but does not have decreased or loss of binding to mutant 5 having substitutions at residues Q96, N99, E143 and R147.

Other antibodies that showed inhibition of soluble extracellular CD39 in Example 5 had very different epitopes and did not show loss of binding to either of mutants 5 or 19, suggesting that soluble CD39 can also be inhibited by binding to other sites on sCD39. For some antibodies, loss of binding to one of the 20 mutants of Table 1 permitted the localization of binding site on CD39, while for others the binding site remained to be determined as they did not lose binding to any of the 20 mutants. Among the antibodies showing inhibition of ATPase activity of soluble CD39 in Example 5, antibody I-396 showed loss of binding to mutant 15 having substitutions K87A, E100A and D107A, without loss of binding to any of the other 20 mutants. The core epitope on CD39 of this antibody thus comprises one or more (or all of) residues K87, E100 and D107. Antibody I-399 showed loss of binding to mutant 11 having substitutions N371K, L372K, E375A, K376G, V377A and an insertion of a valine between K376 and V377 (referred to in Table 1 as "insertion 377V"), without loss of binding to any of the other 20 mutants. The core epitope on CD39 of this antibody thus comprises one or more (or all of) residues N371, L372, E375, K376 and V377. **Figure 3A** shows the position of residues mutated in mutants 5 (M5), 15 (M15) and 19 (M19) on the surface of the CD39 protein. **Figure 3B** shows results of binding to mutants 5, 15 and 19 for different antibodies.

The results thus show that antibodies that inhibit soluble CD39 can be obtained against different epitopes. The epitopes include epitopes defined by one or more residues of mutant 19 which are located adjacent to the binding site of the BY40 or BY40-like antibodies that inhibit only cellular CD39 but not soluble CD39 (which lose binding to mutant 5), epitopes that are defined by one or more residues of mutant 19 but also partly by mutant 5, indicating possibly a smaller shift compared to BY40 or BY40-like antibodies, epitopes defined by one or more residues of mutant 19 and not by residues of mutant 5, as well as other epitopes such as those defined by one or more residues of mutant 11 or one or more residues of mutant 15, or further by other antibodies that do not have any reduced binding to any of mutants 5, 15 or 19 for which localization of epitopes remain to be determined.

### Example 7: Antibody titration on CD39 expressing cells by flow cytometry

Antibody I-394 was tested in two repeated experiments for binding to CHO cells expressing human CD39, CHO cells expressing cynomolgus (macaca fascicularis) CD39, CHO cells expressing murine CD39, and human Ramos lymphoma cells (ATCC^{™}, reference CRL-1596). Cells were incubated with various concentration of unlabeled anti-CD39 antibody from 30 µg/ml to 5×10-⁴ µg/ml, for 30 minutes at 4°C. After washes, cells were incubated with Goat anti-mouse H+L labeled secondary antibody for 30min at 4°C.

Results are shown in **Figure 4****.** Antibody I-394 bound to cells expressing human CD39 (CHO-huCD39), cells expressing cynomolgus CD39 (CHO-cyCD39) and to Ramos lymphoma cells, but not to cells expressing murine CD39 (CHO-moCD39). I-394 bound to Ramos cells with EC₅₀ values of 0.16 µg/ml and 0.19 µg/ml in the respective first and second set of experiments. Several other anti-CD39 antibodies showed comparable EC₅₀ values for binding to Ramos cells.

### Example 8: IC50 determination for inhibition of cellular ATPase activity

The inhibition by antibody I-394 of the ATPase activity of CD39 in CD39-expressing cells was evaluated using the assay used for inhibition of the enzymatic activity of cellular CD39 as described above (Methods).

Results are shown in **Figure 5****.** I-394 is highly potent at blocking CD39 enzymatic activity in tumor (Ramos) cells, with greater potency compared to all other antibodies tested. I-394 also blocks CD39 enzymatic activity in cells expressing human CD39 (CHO-huCD39), and in cells expressing cynomolgus CD39 (CHO-cyCD39). Cells expressing murine CD39 (CHO-moCD39) are shown as a negative control. The calculated IC₅₀ (inhibition of 50% of the enzymatic activity of CD39 expressed by 50,000 Ramos cells) is 0.05 µg/ml. The maximum inhibition achieved is 81.6%. Isotype control had no effect.

### Example 9: IC50 determination for inhibition of the ATPase activity of recombinant soluble CD39 protein

The inhibition by antibody I-394 of the ATPase activity of soluble CD39 protein was evaluated using the assays used for inhibition of the enzymatic activity of soluble CD39 as described above (Methods). Results are shown in **Figure 6****.** I-394 inhibits the enzymatic activity of soluble CD39 protein. Antibody BY40 in comparison did not inhibit the enzymatic activity of soluble CD39 protein. The calculated IC₅₀ is 0.003 µg/ml. The maximum inhibition achieved is 74.9%.

### Example 10: ELISA titration on CD39-L1, L2, L3, L4 isoforms

Antibody I-394 was tested for binding to recombinant human CD39 isoforms (Rec-huCD39 isoforms) having amino acid sequences shown below were coated in 96-well plate in PBS 1X at 500ng/ml or 1µg/ml at 4°C overnight. Wells were washed in TBS Tween 20, and further saturated 2H at RT in TBS Blocking buffer. Dose range concentration of primary antibody was incubated in TBS blocking buffer for 2h at RT. Wells were washed in TBS Tween 20. Secondary Antibody (GAM-HRP or GAH-HRP in TBS blocking buffer) was incubated for 1H at RT, and was revealed with TMB. Optical density was measured on Enspire^{™} at OD=450.

### Amino acid sequence of the cloned huCD39 (vascular isoform):

Human CD39-L1, also known as NTPDase2 or ENTPD2: 
1 MAGKVRSLLP PLLLAAAGLA GLLLLCVPTR DVREPPALKY GIVLDAGSSH TSMFIYKWPA
61 DKENDTGIVG QHSSCDVPGG GISSYADNPS GASQSLVGCL EQALQDVPKE RHAGTPLYLG
121 ATAGMRLLNL TNPEASTSVL MAVTHTLTQY PFDFRGARIL SGQEEGVFGW VTANYLLENF
181 IKYGWVGRWF RPRKGTLGAM DLGGASTQIT FETTSPAEDR ASEVQLHLYG QHYRVYTHSF
241 LCYGRDQVLQ RLLASALQTH GFHPCWPRGF STQVLLGDVY QSPCTMAQRP QNFNSSARVS
301 LSGSSDPHLC RDLVSGLFSF SSCPFSRCSF NGVFQPPVAG NFVAFSAFFY TVDFLRTSMG
361 LPVATLQQLE AAAVNVCNQT WAQQLLSRGY GFDERAFGGV IFQKKAADTA VGWALGYMLN
421 LTNLIPADPP GLRKGTDFSS WVVLLLLFAS ALLAALVLLL RQVHSAKLPS TI
(SEQ ID NO: 55).
Human CD39-L2, also known as NTPDase6 or ENTPD6: 
1 MKKGIRYETS RKTSYIFQQP QHGPWQTRMR KISNHGSLRV AKVAYPLGLC VGVFIYVAYI
61 KWHRATATQA FFSITRAAPG ARWGQQAHSP LGTAADGHEV FYGIMFDAGS TGTRVHVFQF
121 TRPPRETPTL THETFKALKP GLSAYADDVE KSAQGIRELL DVAKQDIPFD FWKATPLVLK
181 ATAGLRLLPG EKAQKLLQKV KEVFKASPFL VGDDCVSIMN GTDEGVSAWI TINFLTGSLK
241 TPGGSSVGML DLGGGSTQIA FLPRVEGTLQ ASPPGYLTAL RMFNRTYKLY SYSYLGLGLM
301 SARLAILGGV EGQPAKDGKE LVSPCLSPSF KGEWEHAEVT YRVSGQKAAA SLHELCAARV
361 SEVLQNRVHR TEEVKHVDFY AFSYYYDLAA GVGLIDAEKG GSLVVGDFEI AAKYVCRTLE
421 TQPQSSPFSC MDLTYVSLLL QEFGFPRSKV LKLTRKIDNV ETSWALGAIF HYIDSLNRQK
481 SPAS
(SEQ ID NO:56).
Human CD39-L3, also known as NTPDase3 or ENTPD3: 
1 MFTVLTRQPC EQAGLKALYR TPTIIALVVL LVSIVVLVSI TVIQIHKQEV LPPGLKYGIV
61 LDAGSSRTTV YVYQWPAEKE NNTGVVSQTF KCSVKGSGIS SYGNNPQDVP RAFEECMQKV
121 KGQVPSHLHG STPIHLGATA GMRLLRLQNE TAANEVLESI QSYFKSQPFD FRGAQIISGQ
181 EEGVYGWITA NYLMGNFLEK NLWHMWVHPH GVETTGALDL GGASTQISFV AGEKMDLNTS
241 DIMQVSLYGY VYTLYTHSFQ CYGRNEAEKK FLAMLLQNSP TKNHLTNPCY PRDYSISFTM
301 GHVFDSLCTV DQRPESYNPN DVITFEGTGD PSLCKEKVAS IFDFKACHDQ ETCSFDGVYQ
361 PKIKGPFVAF AGFYYTASAL NLSGSFSLDT FNSSTWNFCS QNWSQLPLLL PKFDEVYARS
421 YCFSANYIYH LFVNGYKFTE ETWPQIHFEK EVGNSSIAWS LGYMLSLTNQ IPAESPLIRL
481 PIEPPVFVGT LAFFTAAALL CLAFLAYLCS ATRRKRHSEH AFDHAVDSD
(SEQ ID NO: 57).
Human CD39-L4, also known as NTPDase5 or ENTPD5:

1 MATSWGTVFF MLVVSCVCSA VSHRNQQTWF EGIFLSSMCP INVSASTLYG IMFDAGSTGT
61 RIHVYTFVQK MPGQLPILEG EVFDSVKPGL SAFVDQPKQG AETVQGLLEV AKDSIPRSHW
121 KKTPVVLKAT AGLRLLPEHK AKALLFEVKE IFRKSPFLVP KGSVSIMDGS DEGILAWVTV
181 NFLTGQLHGH RQETVGTLDL GGASTQITFL PQFEKTLEQT PRGYLTSFEM FNSTYKLYTH
241 SYLGFGLKAA RLATLGALET EGTDGHTFRS ACLPRWLEAE WIFGGVKYQY GGNQEGEVGF

301 EPCYAEVLRV VRGKLHQPEE VQRGSFYAFS YYYDRAVDTD MIDYEKGGIL KVEDFERKAR
361 EVCDNLENFT SGSPFLCMDL SYITALLKDG FGFADSTVLQ LTKKVNNIET GWALGATFHL
421 LQSLGISH
(SEQ ID NO: 58).

I-394 bound to the CD39 but not to any of the isoforms CD39-L1, -L2, -L3 or -L4. Isotype control antibodies (IC) did not bind to any CD39 or CD39-L molecule. Results are shown in **Figure 7****.**

### Example 11: Activation of dendritic cells

While ATP has pro-inflammatory activity, CD39-mediated catabolism of ATP is believed to be able to impair dendritic cell (DC) activation, in turn altering a broader adaptive immune response against tumor antigen. In order to evaluate whether CD39 blockade using anti-CD39 antibodies could overcome CD39-mediated alteration of dendritic cell (DC) activation in the presence of ATP, we incubated monocyte-derived DC (moDC) with anti-CD39 antibodies in the presence of ATP.

Briefly, human monocytes were purified from human healthy blood and differentiated into MoDC in presence of GM-CSF and IL-4 during 6 days. Then MoDC were activated in presence of ATP (Sigma, 0.25 - 1 mM) during 24 hours and DC activation were assessed by analyzing CD80, CD83 and HLA-DR expression by flow cytometry. In some cases, MoDC were preincubated during 1 hours in presence of CD39 inhibitor: ARL6716 (Tocris, 250 µM), CD73 inhibitor: APCP (Tocris 50 µM), anti-CD39 blocking antibody I-394 or BY40 (for BY40 see WO2009/095478), or anti-CD73 blocking antibodies. LPS (Invivogen, 10 ng/ml) was used as positive control. To assess resulting effect of ATP-mediated DC activation on CD4 T cells activation, ATP-activated DC were washed and then incubated with allogenic CD4 T cells (ratio 1 MoDC / 4 T cells) for a mixed lymphocytes reaction (MLR) during 5 days. T cells activation and proliferation were analyzed through CD25 expression and Cell Trace Violet dilution by flow cytometry **(****Figure 8****).**

Results are shown in **Figures 9****,** **10** **and** **11****.** In the presence of negative control (medium), moDC activation was observed in the presence of 1 mM ATP, however ATP at 0.125 mM, 0.25 mM or 0.5mM did not permit moDC activation. Addition of chemical inhibitors of CD39 which are believed to fully block CD39 enzymatic activity by binding to the active site lead to moDC activation at each of 0.125 mM, 0.25 mM or 0.5mM. However, anti-CD39 antibodies such as BY40 or anti-CD73 antibodies were not able to favor ATP-induced activation of dendritic cell (DC), suggesting that antibodies are not able to block enzymatic activity sufficiently to avoid ATP catabolism. Surprisingly, the anti-CD39 blocking antibody I-394 (shown in Figures at concentration 10 µg/ml) which substantially fully blocks the ATPase activity of CD39 and can therefore permit accumulation of ATP, permitted moDC activation as assessed by HLA-DR or CD83 expression at each of 0.125 mM, 0.25 mM or 0.5mM **(****Figures 9** **and** **10****).** Interestingly, the MoDC activated in presence of ATP were able to induce better T cells activation and proliferation in a MLR assay. Moreover, the enhancement of ATP-mediated MoDC activation by anti-CD39 blocking antibody I-394 resulted in higher T cells proliferation and activation **(****Figure 11****).**

Assessment of the ability to CD39 inhibitors to activate DC in the presence of ATP provides a method to identify and evaluate anti-CD39 antibodies that are able to achieve a high degree of inhibition of CD39. Furthermore, the possibility of using anti-CD39 antibodies to relieve the immunosuppressive effect exerted by CD39 upon DC can provide for enhancement of the adaptive immune response toward antigens, notably on tumors cells. Furthermore, such anti-CD39 antibodies may be of particular interest when used to enhance the immunogenic effect of chemotherapeutic agents. Numerous chemotherapeutic agents that cause necrosis of tumor cells are able to induce ATP; combined use with anti-CD39 antibodies can be particularly useful to enhance the anti-tumor response in these settings.

### Example 12: Antibodies that inhibit the ATPase activity of recombinant soluble CD39 protein strongly potentiate CD73 blockade in the presence of ATP.

### T cell proliferation assay

Peripheral blood from healthy donors was obtained from EFS, and mononuclear cells were isolated on a Ficoll gradient. Lymphocytes were further enriched on a 52% Percoll gradient by collection of the cell pellets and stained with a Cell Trace dye (Thermofisher) following the TDS provided by the manufacturer. 5×10⁴ to 1×10⁵ of stained cells were distributed in 96 round-bottom plates, incubated for 1 hour at 37°C with anti-huCD73 antibodies (antibody 6E1) and/or anti-huCD39 Abs (I-394 described herein) and activated for 3 to 5 days by addition of anti-CD3/anti-CD28-coated beads (bead:cell = 1:4 ; Life Technologies). Inhibition of T cell proliferation was achieved by addition of ATP (200µM). T cell proliferation and ability of Abs to block immune suppressive effect of AMP were assessed by flow cytometry by quantifying the dye dilution in the proliferating T cell subset.

Percentage of proliferating T cells vs. anti-CD73 Ab concentration is plotted in graphs using GraphPad Prism software.

### Results

Antibodies were tested for the ability to restore CD4 or CD8 T cell proliferation in the presence of added ATP, intended to represent conditions as may be found in the tumor environment. Each of anti-CD73 and CD39 were tested in a dose range at 3 different doses of the other of the anti-CD73 or anti-CD39 antibody. Anti-CD39 antibody I-394 strongly potentiation the effect of anti-CD73 antibodies in restoring CD4 or CD8 T cell proliferation, such that even low concentrations of anti-CD73 antibodies (e.g. below 0.01 µg/ml, below 0.001 µg/ml and even below 0.001 µg/ml) strongly enhanced CD4 or CD8 T cell proliferation, when used in combination with anti-CD39 antibodies. Furthermore, when tested in a dose range alone without anti-CD73, the anti-CD39 antibody I-394 resulted in a remarkable enhancement of CD4 or CD8 T cell proliferation at concentrations of 0.1 µg/ml and 1 µg/ml. **Figure 12A** shows the dose range of anti-CD73 antibody 6E1 on CD4 T cell proliferation at 3 different doses of anti-CD39 antibody I-394, either 0.01 µg/ml, 0.1 µg/ml and 1 µg/ml. The anti-CD39 antibodies that are capable of neutralizing soluble and/or monomeric human CD39 show a strong potentiation of the effect with anti-CD73 antibodies in restoring CD4 T cell proliferation. The effect was particularly strong at concentrations where anti-CD73 antibodies were sub-optimally active, corresponding to concentrations ranges that can be observed in tumor tissues during the course of treatment with an anti-CD73 antibody. At a concentration of 0.01 µg/ml, the anti-CD39 antibodies provided an approximately 1-log increase in potency of anti-CD73 antibodies, and a concentration of 0.1 µg/ml, the anti-CD39 antibodies provided an approximately 4-log increase in potency of anti-CD73 antibodies. The anti-CD39 antibodies can therefore be useful to enhance the activity of anti-CD73 antibodies, particularly in tumor tissue, for example in tumors harboring CD73-expressing cells. Furthermore, while the anti-CD73 antibodies tested (that are capable of neutralizing soluble CD73 protein) possessed high capacity to restore CD4 T cell proliferation, other antibodies having lower potency (e.g. as assessed in an enzymatic inhibition assay, in a T cell proliferation assay, or other suitable assay) and may benefit even more from combination with the anti-sCD39 antibodies. **Figure 12B** shows the dose range of anti-CD73 antibodies on CD8 T cell proliferation. Again, anti-CD39 antibodies show a strong synergy and/or additive effect with anti-CD73 antibodies in restoring CD8 T cell proliferation. The effect was particularly strong at concentrations where anti-CD73 antibodies were sub-optimally active, corresponding to concentrations ranges that can be observed in tumor tissues during the course of treatment with an anti-CD73 antibody.

A study of CD39 and CD73 gene expression was carried out using Cancer Genome Atlas (a collaboration between the National Cancer Institute and National Human Genome Research Institute) based on multi-dimensional maps of the key genomic changes in 33 types of cancer. Levels of expression (indicated as high or low) were considered, taking account of disease stage and time. For each cancer, and each gene ( CD39/ENTPD1 and CD73/NT5E), patients were divided in 2 groups (high and low gene expression) according to the p-value of the Cox regression (each group must contain at least 10% of patients). Survival probability curves were drawn for each 2 groups. Statistical survival differences between low and high CD39 expression were observed for ovarian cancer and stomach adenocarcinoma samples, with high-expressing CD39 exhibiting lower survival. A tendency was also observed for esophageal squamous carcinoma and for lung squamous cell carcinoma samples. Results are shown in **Figure 13A-C****,** showing that low CD39 expression in human cancer (ovarian, esophageal and stomach cancer in Figures 13A, B and C, respectively) is correlated with higher survival probability while high expression CD73 is correlated with lower survival probability (ovarian, esophageal and stomach cancer).

## Claims

1. An anti-CD39 antibody that is capable of binding and inhibiting the ATPase activity of a soluble extracellular domain human CD39 (NTPDase1) protein, for use in the treatment of cancer in a human individual, the treatment comprising administering:
(a) the anti-CD39 antibody that is capable of binding and inhibiting the ATPase activity of a soluble extracellular domain human CD39 (NTPDase1) protein, wherein the antibody comprises a HCDR1 comprising an amino acid sequence DYNMH (SEQ ID NO: 8); a HCDR2 comprising an amino acid sequence YIVPLNGGSTFNQKFKG (SEQ ID NO: 9); a HCDR3 comprising an amino acid sequence GGTRFAY (SEQ ID NO: 10); a LCDR1 comprising an amino acid sequence RASESVDNFGVSFMY (SEQ ID NO: 11); a LCDR2 region comprising an amino acid sequence GASNQGS (SEQ ID NO: 12); and a LCDR3 region comprising an amino acid sequence QQTKEVPYT (SEQ ID NO: 13), and
(b) an anti-CD73 antibody that neutralizes the 5'-ectonucleotidase activity of human CD73
wherein the cancer is a leukemia, a glioma or glioblastoma, or a cancer of the bladder, breast, colon, esophagus, kidney, liver, lung, ovary, uterus, prostate, pancreas, stomach, cervix, thyroid, head and neck or skin.

2. The anti-CD39 antibody for use of claim 1, wherein the antibody that neutralizes the ATPase activity of human CD39 is administered prior to the administration of the antibody that neutralizes the 5'-ectonucleotidase activity of human CD73.

3. The anti-CD39 antibody for use of any one of claims 1 or 2, wherein the antibody that neutralizes the ATPase activity of human CD39 and the antibody that neutralizes the 5'-ectonucleotidase activity of CD73 are formulated for separate administration and are administered concurrently or sequentially.

4. The anti-CD39 antibody for use of any of the above claims, wherein the antibody that is capable of binding and inhibiting the ATPase activity of a soluble extracellular domain human CD39 (NTPDase1) protein lacks an Fc domain or comprises an Fc domain that is modified to reduce binding between the Fc domain and an Fcγ receptor.

5. The anti-CD39 antibody for use of any of the above claims, wherein the antibody that neutralizes the 5'-ectonucleotidase activity of CD73 lacks an Fc domain or comprises an Fc domain that is modified to reduce binding between the Fc domain and an Fcγ receptor.

6. The anti-CD39 antibody for use of any of the above claims, wherein the individual comprises tumor tissue and/or tumor adjacent tissue **characterized by** a high level of CD73-expressing cells.

7. The anti-CD39 antibody for use of any of the above claims, wherein the individual has a cancer that has progressed or relapsed following prior treatment with an agent that inhibits a human CD73 polypeptide.

8. The anti-CD39 antibody for use of any of the above claims, wherein the individual has a solid tumor.

9. The anti-CD39 antibody for use of any of the above claims, wherein the individual has an ovarian cancer.

10. The anti-CD39 antibody for use of any of claims 1-8, wherein the individual has a gastric cancer.

11. The anti-CD39 antibody for use of any of claims 1-8, wherein the individual has a lung cancer.

12. The anti-CD39 antibody for use of any of claims 1-8, wherein the individual has a colon cancer.

13. The anti-CD39 antibody for use of any of claims 1-8, wherein the individual has an esophageal cancer.

14. The anti-CD39 antibody for use of any of claims 1-8, wherein the individual has a breast cancer.

## Patentansprüche

1. Anti-CD39-Antikörper, der in der Lage ist, die ATPase-Aktivität eines humanen CD39-Proteins (NTPDase1) mit löslicher extrazellulärer Domäne zu binden und zu inhibieren, zur Verwendung bei der Behandlung von Krebs in einem menschlichen Individuum, wobei die Behandlung die Verabreichung umfasst:
(a) den Anti-CD39-Antikörper, der in der Lage ist, die ATPase-Aktivität eines humanen CD39-Proteins (NTPDase1) mit löslicher extrazellulärer Domäne zu binden und zu inhibieren, der Antikörper umfassend ein HCDR1, umfassend eine Aminosäuresequenz DYNMH (SEQ ID NO: 8); ein HCDR2, umfassend eine Aminosäuresequenz YIVPLNGGSTFNQKFKG (SEQ ID NO: 9); ein HCDR3, umfassend eine Aminosäuresequenz GGTRFAY (SEQ ID NO: 10); ein LCDR1, umfassend eine Aminosäuresequenz RASESVDNFGVSFMY (SEQ ID NO: 11); eine LCDR2-Region, umfassend eine Aminosäuresequenz GASNQGS (SEQ ID NO: 12); und eine LCDR3-Region, umfassend eine Aminosäuresequenz QQTKEVPYT (SEQ ID NO: 13), und
(b) einen Anti-CD73-Antikörper, der die 5'-Ectonukleotidase-Aktivität von menschlichem CD73 neutralisiert,
wobei der Krebs eine Leukämie, ein Gliom oder Glioblastom oder ein Krebs der Blase, der Brust, des Dickdarms, der Speiseröhre, der Niere, der Leber, der Lunge, des Eierstocks, der Gebärmutter, der Prostata, der Bauchspeicheldrüse, des Magens, des Gebärmutterhalses, der Schilddrüse, des Kopfes und Halses oder der Haut ist.

2. Anti-CD39-Antikörper zur Verwendung nach Anspruch 1, wobei der Antikörper, der die ATPase-Aktivität von menschlichem CD39 neutralisiert, vor der Verabreichung des Antikörpers verabreicht wird, der die 5'-Ectonukleotidase-Aktivität von menschlichem CD73 neutralisiert.

3. Anti-CD39-Antikörper zur Verwendung nach einem der Ansprüche 1 oder 2, wobei der Antikörper, der die ATPase-Aktivität von menschlichem CD39 neutralisiert, und der Antikörper, der die 5'-Ectonukleotidase-Aktivität von CD73 neutralisiert, zur getrennten Verabreichung formuliert und gleichzeitig oder nacheinander verabreicht werden.

4. Anti-CD39-Antikörper zur Verwendung nach einem der vorstehenden Ansprüche, wobei dem Antikörper, der in der Lage ist, die ATPase-Aktivität des humanen CD39-Protein (NTPDase1) mit löslicher extrazellulärer Domäne zu binden und zu inhibieren, eine Fc-Domäne fehlt oder eine Fc-Domäne umfasst, die modifiziert ist, um die Bindung zwischen der Fc-Domäne und einem Fcγ-Rezeptor zu verringern.

5. Anti-CD39-Antikörper zur Verwendung nach einem der vorstehenden Ansprüche, wobei dem Antikörper, der die 5'-Ektonukleotidaseaktivität von CD73 neutralisiert, eine Fc-Domäne fehlt oder eine Fc-Domäne umfasst, die modifiziert ist, um die Bindung zwischen der Fc-Domäne und einem Fcγ-Rezeptor zu verringern.

6. Anti-CD39-Antikörper zur Verwendung nach einem der vorstehenden Ansprüche, wobei das Individuum Tumorgewebe und/oder zum Tumor angrenzendes Gewebe enthält, das durch ein hohes Level an CD-73 exprimierenden Zellen gekennzeichnet ist.

7. Anti-CD39-Antikörper zur Verwendung nach einem der vorstehenden Ansprüche, wobei das Individuum einen Krebs hat, der nach vorheriger Behandlung mit einem Mittel, das ein humanes CD73-Polypeptid inhibiert, fortgeschritten oder rezidivirt ist.

8. Anti-CD39-Antikörper zur Verwendung nach einem der vorstehenden Ansprüche, wobei das Individuum einen soliden Tumor hat.

9. Anti-CD39-Antikörper zur Verwendung nach einem der vorstehenden Ansprüche, wobei das Individuum einen Krebs des Eierstocks hat.

10. Anti-CD39-Antikörper zur Verwendung nach einem der vorstehenden Ansprüche, wobei das Individuum. einen Krebs des Magens hat.

11. Anti-CD39-Antikörper zur Verwendung nach einem der vorstehenden Ansprüche, wobei das Individuum einen Krebs der Lunge hat.

12. Anti-CD39-Antikörper zur Verwendung nach einem der vorstehenden Ansprüche, wobei das Individuum einen Krebs des Dickdarms hat.

13. Anti-CD39-Antikörper zur Verwendung nach einem der vorstehenden Ansprüche, wobei das Individuum einen Krebs der Speiseröhre hat.

14. Anti-CD39-Antikörper zur Verwendung nach einem der vorstehenden Ansprüche, wobei das Individuum einen Krebs der Brust hat.

## Revendications

1. Anticorps anti-CD39 qui est capable de se lier à et d'inhiber l'activité d'ATPase d'une protéine humaine du domaine extracellulaire soluble CD39 (NTPDase1), pour une utilisation dans le traitement d'un cancer chez un individu humain, le traitement comprenant l'administration :
(a) de l'anticorps anti-CD39 qui est capable de se lier à et d'inhiber l'activité d'ATPase d'une protéine humaine de domaine extracellulaire soluble CD39 (NTPDase1), lequel anticorps comprend une HCDR1 comprenant la séquence d'acides aminés DYNMH (SEQ ID NO : 8) ; une HCDR2 comprenant la séquence d'acides aminés YIVPLNGGSTFNQKFKG (SEQ ID NO : 9) ; une HCDR3 comprenant la séquence d'acides aminés GGTRFAY (SEQ ID NO : 10) ; une LCDR1 comprenant la séquence d'acides aminés RASESVDNFGVSFMY (SEQ ID NO : 11) ; une région LCDR2 comprenant la séquence d'acides aminés GASNQGS (SEQ ID NO : 12) ; et une région LCDR3 comprenant la séquence d'acides aminés QQTKEVPYT (SEQ ID NO : 13) ; et
(b) d'un anticorps anti-CD73 qui neutralise l'activité de 5'-ectonucléotidase de la CD73 humaine,
dans lequel le cancer est une leucémie, un gliome ou un glioblastome, ou un cancer de la vessie, du sein, du côlon, de l'œsophage, du rein, du foie, du poumon, de l'ovaire, de l'utérus, de la prostate, du pancréas, de l'estomac, du col de l'utérus, de la thyroïde, de la tête et du cou, ou de la peau.

2. Anticorps anti-CD39 pour une utilisation selon la revendication 1, dans lequel l'anticorps qui neutralise l'activité d'ATPase de la CD39 humaine est administré avant l'administration de l'anticorps qui neutralise l'activité de 5'-ectonucléotidase de la CD73 humaine.

3. Anticorps anti-CD39 pour une utilisation selon l'une quelconque des revendications 1 et 2, dans lequel l'anticorps qui neutralise l'activité d'ATPase de la CD39 humaine et l'anticorps qui neutralise l'activité de 5'-ectonucléotidase de la CD73 sont formulés pour une administration séparée et sont administrés simultanément ou séquentiellement.

4. Anticorps anti-CD39 pour une utilisation selon l'une quelconque des revendications précédentes, dans lequel l'anticorps qui est capable de se lier à et d'inhiber l'activité d'ATPase d'une protéine humaine du domaine extracellulaire soluble CD39 (NTPDase1) est dépourvu d'un domaine Fc ou comprend un domaine Fc qui est modifié pour réduire la liaison entre le domaine Fc et un récepteur Fcy.

5. Anticorps anti-CD39 pour une utilisation selon l'une quelconque des revendications précédentes, dans lequel l'anticorps qui neutralise l'activité de la 5'-ectonucléotidase de la CD73 est dépourvu d'un domaine Fc ou comprend un domaine Fc qui est modifié pour réduire la liaison entre le domaine Fc et un récepteur Fcy.

6. Anticorps anti-CD39 pour une utilisation selon l'une quelconque des revendications précédentes, dans lequel l'individu comprend du tissu tumoral et/ou du tissu adjacent à une tumeur **caractérisé par** un niveau élevé de cellules exprimant la CD73.

7. Anticorps anti-CD39 pour une utilisation selon l'une quelconque des revendications précédentes, dans lequel l'individu a un cancer qui a progressé ou récidivé après un traitement antérieur avec un agent qui inhibe un polypeptide de la CD73 humaine.

8. Anticorps anti-CD39 pour une utilisation selon l'une quelconque des revendications précédentes, dans lequel l'individu a une tumeur solide.

9. Anticorps anti-CD39 pour une utilisation selon l'une quelconque des revendications précédentes, dans lequel l'individu a un cancer de l'ovaire.

10. Anticorps anti-CD39 pour une utilisation selon l'une quelconque des revendications 1 à 8, dans lequel l'individu a un cancer gastrique.

11. Anticorps anti-CD39 pour une utilisation selon l'une quelconque des revendications 1 à 8, dans lequel l'individu a un cancer du poumon.

12. Anticorps anti-CD39 pour une utilisation selon l'une quelconque des revendications 1 à 8, dans lequel l'individu a un cancer du côlon.

13. Anticorps anti-CD39 pour une utilisation selon l'une quelconque des revendications 1 à 8, dans lequel l'individu a un cancer de l'œsophage.

14. Anticorps anti-CD39 pour une utilisation selon l'une quelconque des revendications 1 à 8, dans lequel l'individu a un cancer du sein.
